# EUROPEAN PATENT SPECIFICATION

(11) **EP 0 956 296 B1**
(45) Date of publication and mention of the grant of the patent: **21.06.2006**
(21) Application number: 97949758.3
(22) Date of filing: 04.12.1997
(51) Int. Cl.: C07K 7/00

(54) **SOMATOSTATIN ANTAGONISTS**
SOMATOSTATIN-ANTAGONISTEN
ANTAGONISTES DE LA SOMATOSTATINE

(30) Priority: 04.12.1996 US 32358 P; 04.12.1996 US 760672; 13.05.1997 US 855204
(43) Date of publication of application: 17.11.1999
(73) Proprietor: SOCIETE DE CONSEILS DE RECHERCHES ET D'APPLICATIONS SCIENTIFIQUES (SCRAS) S.A.S, 75016 Paris (FR); The Administrators of The Tulane Educational Fund, New Orleans Louisiana 70112 (US)
(72) Inventor: MORGAN, Barry, Franklin, MA 02038 (US); MURPHY, William, Slidell, LA 70460 (US); COY, David, H., New Orleans, LA 70130 (US)
(74) Representative: Lunt, Mark George Francis
(86) International application number: PCT/US1997/022251
(87) International publication number: WO 1998/024807

(56) References cited:
- EP-A- 0 187 622
- EP-A- 0 395 417
- WO-A-89/04666
- WO-A-97/11962
- US-A- 4 853 371
- US-A- 4 904 642
- US-A- 5 633 263
- OESAPAY, GEORGE ET AL: "Lanthionine- Somatostatin Analogs: Synthesis, Characterization, Biological Activity, and Enzymic Stability Studies" J. MED. CHEM. ( 1997 ), 40(14), 2241-2251 CODEN: JMCMAR;ISSN: 0022-2623, XP002071716
- MELACINI, GIUSEPPE ET AL: "A Refined Model for the Somatostatin Pharmacophore: Conformational Analysis of Lanthionine-Sandostatin Analogs" J. MED. CHEM. ( 1997 ), 40(14), 2252-2258 CODEN: JMCMAR;ISSN: 0022-2623, XP002071717

## Description

Native somatostatin is comprised of both a 14-amino acid isoform (somatostatin-14) and a 28-amino acid isoform (somatostatin-28). Heiman, et al., Neuroendocrinology, 45:429-436 (1987). Because of the short half-life of the native somatostatin, various somatostatin, various somatostatin analogs have been developed, e.g., for the treatment of acromegaly. Raynor, et al., Molecular Pharmacol. 43:838 (1993). Various somatostatin analogs which can be effective to inhibit growth hormone release, as well as to inhibit insulin, glucagon and pancreatic exocrine secretion, are disclosed in US 4, 904, 642 and US 4, 853,371. Five distinct somatostatin receptors have been identified and characterised. Hoyer, et al., Naunyn-Schmiedeberg's Arch. Pharmacol., 350:441 (1994). Somatostatin produces a variety of effects, including modulation of hormone release, e.g., growth hormone, glucagon, insulin, amylin, and neurotransmitter release. Some of these effects have been associated with its binding to a specific somatostatin receptor. For example, the inhibition of growth hormone has been attributed to the somatostatin type-2 receptor ("SSTR-2") (Raynor, et al., Molecular Pharmacol. 43:838 (1993); Lloyd, et al., Am. J. Physiol. 268:G102 (1995)) while the inhibition of insulin has been attributed to the somatostatin type-5 receptor ("SSTR-5") (Coy, et al. 197:366371 (1993)). The following invention relates to a novel class of somatostatin analogs which are antagonists to somatostatin receptors.

### Summary of the Invention

The invention features a compound of the formula: 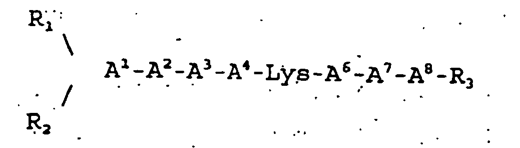 wherein
A¹ is a D- or L-isomer of an aromatic amino acid, or is deleted;
A² is a D-isomer selected from the group consisting of Cys, Pen, an aromatic amino acid, or an aliphatic amino acid;
A³ is an aromatic amino acid;
A⁴ is Trp or D-Trp;
A⁶ is Thr, Thr(Bzl), Gly, Ser, an Eaa, or an aliphatic amino acid;
A⁷ is Cys, Pen, or an aromatjkic or an aliphatic amino acid;
A⁸ is a D- or L-isomer selected from the group consisting of Thr, Ser, an aromatic amino acid, or an aliphatic amino acid;
each of R¹, and R², is, independently, H or substituted (e.g., one to four times) or unsubstituted lower alkyl, aryl, aryl lower alkyl, heterocycle, heterocycle lower alkyl, E₁SO₂ or E₁CO (where E₁ is aryl, aryl lower alkyl, heterocycle, or heterocycle lower alkyl), where said substituent is halo, lower alkyl, hydroxy, halo lower alkyl, or hydroxy lower alkyl; and
R₃ is OH, NH₂, C₁₋₁₂ alkoxy, or NH-Y-CH₂-Z, wherein Y is a C₁₋₁₂ hydrocarbon moiety and Z is H, OH, C0₂H, or CONH₂, or R₃, together with the carbonyl group of A⁸ attached thereto, are reduced to form H, lower alkyl, or hydroxy lower alkyl; provided if A² is D-Cys or D-Pen, and A⁷ is Cys or Pen, then a disulfide bond links the sidechains of A² and A⁷ , and if A¹ is D-Phe or p-NO₂-Phe; A² is D-Cys; A³ is Phe or Tyr; A⁶ is Thr or Val; and A⁷ is CYS; then A⁸ is β-Nal.

In one embodiment, A² is D-Cys, A⁷ is Cys, and A⁴ is D-Trp. In a further embodiment, A¹ is an L-aromatic amino acid.
In still a further embodiment, A¹ and A³, independently, is β-_Nal, o-X-Phe (where X is H, OH, CH₃, halo, OCH₃, NH_{2'} CN, or NO₂), p-X-Phe (where X is H, OH, CH₃, halo, OCH₃, NH_{2'} CN, or NO₂,), m-X-Phe (where X is H, OH, CH₃, halo, OCH₃, NH_{2'} CN, or NO₂), F₅, -Phe, Trp, Dip, 2-Pal, Tyr(Bzl), His, Igl, Tyr(1), Bta, Bip, Npa, or Pal; A⁶ is Thr, Ser, Tle, Thr(Bzl), Abu, Ala, Ile, Leu, Gly, Nle, β-Ala, Gaba, or Val; and A⁸ is the D- or L-isomer of Thr, Dip, F⁵-Phe, p-X-Phe (where X is H, OH, CH₃, halo, OCH₃, NH₂, CN, or NO₂), OX-Phe (where X is H, OH, CH₃, halo, OCH₃, NH₂, CN, or NO₂), m-X-Phe (where X is H, OH, CH₃, halo, OCH_{3'} NH₂, CN, or NO₂), Igl, Tyr(Bzl), or β-Nal. In yet still another embodiment, A¹ is β-Nal, Npa, Igl, Phe, p-F-Phe, Trp, p-Cl-Phe, or p-CN-Phe; A³ is Tyr, Tyr(I), or Pal; A⁶ is Val, Tle, Nle, Ile, or Leu; A⁸ is p-F-Phe, β-Nal, Tyr, Dip, pC1-Phe, Igl, or p-CN-Phe; R₁ is H, CH₃CO, 4-(2-hydroxyethyl)-lpiperazinylacetyl, or 4-(2-hydroxyethyl)-lpiperizineethanesulfonyl; R₂ is H; and R₃ is NH₂.
In another further embodiment, A¹ is a D-aromatic amino acid. In still another further embodiment, A¹ is D-β-Nal, D-o-X-Phe (where X is H, OH, CH₃. halo, OCH₃, NH_{2'} CN, or NO₂), D-p-X-Phe (where X is H, OH, CH₃, halo, OCH_{3'} NH_{2'} CN, or NO₂), D-m-X-Phe (where X is H, OH, CH₃, halo, OCH₃, NH_{2'} CN, or NO₂), F₅-Phe, D-Trp, D-Dip, D-2-Pal, D-Tyr(Bzl), D-His, D-Igl, D-Tyr(I), D-Bta, D-Bip, D-Npa, or D-Pal; A³ is β-Nal, o-X-Phe (where X is H,OH,CH₃, halo, OCH₃, NH_{2'} CN, or NO₂), p-X-Phe (where X is H, OH, CH₃, halo, OCH₃, NH₂, CN, or NO₂,), m-X-Phe (where X is H, OH, CH₃, halo, OCH₃, NH_{2'} CN, or NO₂), F₅-Phe, Trp, Dip, 2-Pal, Tyr(Bzl), His, Igl, Tyr(I), Bta, Bip, Npa, or Pal; A⁶ is Thr, Ser, Tle, Thr(Bzl), Abu, Ala, Ile, Leu, Gly, Nle, β-Ala, Gaba, or Val; and A⁸ is the D- or L-isomer of Thr, Dip, F⁵-Phe, p-X-Phe (where X is H, OH, CH₃, halo, OCH₃, NH₂, CN, or NO₂), OX-Phe (where X is H, OH, CH₃, halo, OCH_{3'} NH_{2'} CN, or NO₂), m-X-Phe (where X is H, OH, CH₃, halo, OCH₃, NH_{2'} CN, or NO₂), Tyr(Bzl), Igl, or β-Nal.

In yet still a further embodiment, A¹ is D-β-Nal, D-Npa, D-Igl, D-Phe, D-p-F-Phe, D-Trp, D-p-Cl-Phe, or D-p-CN-Phe; A³ is Tyr, Tyr (I), or Pal; A⁶ is Val, Tle, Nle, Ile, or Leu; A⁸ is p-F-Phe, β-Nal, Tyr, Dip, p-Cl-Phe, Igl, or p-CN-Phe; R¹ is H, CH₃CO, 4- (2-hydroxyethyl)-1-piperazinylacetyl, or 4-(2-hydroxyethyl)-lpiperizineethanesulfonyl; R₂ is H; and R₃ is NH₂.

In still another further embodiment, A¹ is deleted, R¹ is substituted or unsubstituted E₁CO, and R₂ is H. In still a further embodiment, R₁ is substituted or unsubstituted E₁CO (where E₁ is phenyl, β-naphthylmethyl, β-pyridinylmethyl, or 3-indolylmethyl); A³ is β-Nal, o-X-Phe (where X is H, OH, CH₃, halo, OCH₃, NH₂, CN, or NO₂), p-X-Phe (where X is H, OH, CH₃, halo, OCH₃, NH₂, CN, or NO₂), m-X-Phe (where X is H, OH, CH₃, halo, OCH₃, NH₂ CN, or NO₂), F₅-Phe, Trp, Dip, 2-Pal, Tyr(Bzl), His, Igl, Tyr (I), Bta, Bip, Npa, or Pal; A⁶ is Thr, Ser, Tle, Thr(Bzl), Abu, Ala, Ile, Leu, Gly, Nle, β-Ala, Gaba, or Val; and A⁸ is the D- or L-isomer of Thr, Dip, F₅-Phe, p-X-Phe (where X is H, OH, CH₃,, halo, OCH₃, NH_{2'} CN, or NO₂), o-X-Phe (where X is H, OH, CH₃, halo, OCH₃, NH₂, CN, or NO₂), m-X-Phe (where X is H, OH, CH₃, halo, OCH₃, NH₂, CN, or NO₂), Igl, Tyr(Bzl), or β-Nal.

In yet still a further embodiment, R¹ is E₁CO (where E₁ is 4-hydroxy-phenyl, β-naphthylmethyl, or phenyl) ; A³ is Tyr, Tyr(I), or Pal; A⁶ is Val, Tle, Nle, Ile, or Leu; A⁸ is p-F-Phe, β-Nal, Tyr, Dip, p-Cl-Phe, Igl, or p-CN-Phe; R³ is NH₂.

In yet still a further embodiment, R³, together with the carbonyl group of A⁸ attached thereto, are reduced to form H, lower alkyl, or hydroxy lower alkyl. In still another further embodiment, A¹ is the D- or L-isomer of β-Nal, o-X-Phe (where X is H, OH, CH3, halo, OCH3, NH2, CN, or N02), p-X-Phe (where X is H, OH, CH₃, halo, OCH₃, NH_{2'} CN, or NO₂), m-X-Phe (where X is H, OH, CH₃, halo, OCH₃, NH₂, CN, or NO₂) F₅-Phe, Trp, Dip, 2-Pal, Tyr(Bzl), His, Igl, Tyr(I), Bta, Bip, Npa, or Pal; A³ is β-Nal, o-X-Phe (where X is H, OH, CH₃, halo, OCH₃, NH_{2'} CN, or NO₂), p-X-Phe (where X is H, OH, CH₃, halo, OCH₃, NH_{2'} CN, or NO₂), m-X-Phe (where X is H, OH, CH₃, halo, OCH₃, NH₂, CN, or NO₂), F₅-Phe, Trp, Dip, 2-Pal, Tyr(Bzl), His, Igl, Tyr(I), Bta, Bip, Npa, or Pal; A⁶ is Thr, Ser, Tle, Thr(Bzl), Abu, Ala, Ile, Leu, Gly, Nle, β-Ala, Gaba, or Val; and A⁸ is the D- or L-isomer of Thr, Dip, F₅-Phe, p-X-Phe (where X is H, OH, CH₃, halo, OCH₃, NH₂, CN, or NO₂) o-X-Phe (where X is H, OH, CH₃, halo, OCH₃, NH_{2'} CN, or NO₂), m-X-Phe (where X is H, OH, CH₃, halo, OCH₃, NH₂, CN, or NO₂) Igl, Tyr (Bzl), or β-Nal.

In yet still another further embodiment, A¹ is the D- or Lisomer of β-Nal, Phe, p-F-Phe, Trp, p-Cl-Phe, or p-CN-Phe; A³ is Tyr, Tyr(I), or Pal; A⁶ is Val, Tle, Nle, Ile, or Leu; A³ is p-F-Phe, β-Nal, Tyr, Dip, p-Cl-Phe, Igl, or p-CN-Phe; R₁ is H, CH₃CO, 4- (2-hydroxyethyl) -1-piperazinylacetyl, or 4-(2-hydroxyethyl)-1-piperizineethanesulfonyl; R₂ is H, and R₃, together with the carboxy group of A⁸ attached thereto, are reduced to form H or CH₃0H.

In another embodiment, A² is a D-aromatic amino acid or a D-aliphatic amino acid, A⁷ is an aromatic amino acid or an aliphatic amino acid, and A⁴ is D-Trp. In a further embodiment, A¹ is an L- amino acid and A² is a D-aromatic amino acid. In still a further embodiment, A¹, A³, and A⁷ independently, is β-Nal, o-X-Phe (where X is H, OH, CH₃, halo, OCH₃, NH₂, CN, or NO₂), p-X-Phe (where X is H, OH, CH₃, halo, OCH₃, NH_{2'} CN, or NO₂), m-X-Phe (where X is H, OH, CH₃, halo, OCH₃, NH₂, CN, or NO₂), F₅-Phe, Trp, Dip, 2-Pal, Tyr(Bzl), His, Igl, Tyr(I), Bta, Bip, Npa, or Pal; A² is D-β-Nal, D-o-X-Phe (where X is H, OH, CH₃, halo, OCH₃, NH₂, CN, or NO₂), D-p-X-Phe (where X is H, OH, CH₃, halo, OCH₃, NH₂, CN, or NO₂), D-m-X-Phe (where X is H, OH, CH₃, halo, OCH₃, NH₂, CN, or NO₂), D-F₅-Phe, D-Trp, D-Dip, D-2-Pal, D-Tyr(Bzl), D-His, D-Igl, D-Tyr(I), D-Bta, D-Bip, D-Npa, or D-Pal; A⁶ Thr, Ser, Tle, Thr(Bzl), Abu, Ala, Ile, Leu, Gly, Nle, β-Ala, Gaba, or Val; and A⁸ is the D- or L-isomer of Thr, Dip, F₅-Phe, p-X-Phe (where X is H, OH, CH₃, halo, OCH₃, NH₂, CN, or NO₂), o-X-Phe (where X is H, OH, CH₃, halo, OCH₃, NH_{2'} CN, or NO₂), m-X-Phe (where X is H, OH, CH₃, halo, OCH₃, NH_{2'} CN, or NO₂), Tyr(Bzl), Igl, or β-Nal.

In yet still a further embodiment, A¹ is β-Nal or Phe, A² is D-Cpa or D-Phe; A³ is Phe or Tyr; A⁶ is Abu, Thr, or Val; A⁷ is Phe; and A⁸ is Thr; R₁ is H, CH₃CO, 4-(2-hydroxyethyl)-1-piperazinylacetyl, or 4- (2-hydroxyethyl) -1-piperizineethanesulfonyl; R₂ is H; and R₃ is NH₂.

In another further embodiment, A¹ is a D-amino acid and A² is a D-aromatic amino acid.

In still a further embodiment, A¹ and A², independently, is D-β-Nal, D-o-X-Phe (where X is H, OH, CH₃, halo, OCH₃, NH₂, CN, or NO₂), D-p-X-Phe (where X is H, OH, CH₃, halo, OCH_{3'}, NH₂, CN, or NO₂), D-m-X-Phe (where X is H, OH, CH₃, halo, OCH₃, NH₂, CN, or NO₂), D-F₅-Phe, D-Trp, D-Dip, D-2-Pal, D-Tyr(Bzl), D-His, D-Igl, D-Tyr(I), D-Bta, D-Bip, D-Npa, or D-Pal; A³ and A⁷, independently, is β-Nal, oX-Phe (where X is H, OH CH₃, halo, OCH₃, NH₂, CN, or NO₂), p-X-Phe (where X is H, OH CH₃, halo, OCH_{3'} NH₂, CN, or NO₂), m-X-Phe (where X is H, OH CH₃, halo, OCH₃, NH₂, CN, or NO₂), F₅-Phe, Trp, Dip, 2-Pal, His, Igl, Tyr(I), Bta, Bip, Npa, Tyr(Bzl), or Pal; A⁶ is Thr, Ser, Tle, Thr(Bzl), Abu, Ala, Ile, Leu, Gly, Nle, β-Ala, Gaba, or Val; and A⁸ is the D- or L-isomer of Thr, Dip, F⁵-Phe, p-X-Phe (where X is H, OH, CH₃, halo, OCH₃, NH₂, CN, or NO₂), o-X-Phe (where X is H, OH, CH₃, halo, OCH₃, NH₂, CN, or NO₂), m-X-Phe (where X is H, OH, CH₃, halo, OCH₃, NH₂, CN, or NO₂), Igl, Tyr(Bzl), or β-Nal.

In yet still a further embodiment, A¹ is D-β-Nal or D-Phe; A² is D-Cpa or D-Phe; A³ is Phe or Tyr; A⁶ is Thr or Val; A⁷ is Phe; and A⁸ is Thr; R₁ is H, CH₃CO, 4-(2-hydroxyethyl)-1-piperazinylacetyl, or 4-(2-hydroxyethyl)-1-piperizineethanesulfonyl; R₂ is H; and R₃ is NH₂.

Examples of compounds of the present invention include the following:
H₂-β-Nal-D-Cys-Tyr-D-Trp-Lys-Val-Cys-β-Nal-NH₂ (Analog No . 2);
(H) (CH₃CO)-β-Nal-D-Cys-Tyr-D-Trp-Lys-Val-Cys-β-Nal-NH₂, (Analog No. 5) ;
(H)-(4-(2-hydroxyethyl)-1-piperazinylacetyl)-β-Nal-D-Cys-Tyr-D-Trp-Lys-Val-Cys-β-Nal-NH₂;
(H)-(4-(2-hydroxyethyl)-1-piperizineethanesulfonyl)-β-Nal-D-Cys-Tyr-D-Trp-Lys-Val-Cys-β-Nal-NH₂;
H₂-β-Nal-D-Cys-Pal-D-Trp-Lys-Val-Cys-β-Nal-NH₂ (Analog No. 3)
(H) (CH₃CO)-β-Nal-D-Cys-Pal-D-Trp-Lys-Val-Cys-β-Nal-NH₂;
(H)-(4-(2-hydroxyethyl)-1-piperazinylacetyl)-β-Nal-D-Cys-Pal-D-Trp-Lys-Val-Cys-β-Nal-NH₂;
(H)-(4-(2-hydroxyethyl)-1-piperizineethanesulfonyl)-β-Nal-D-Cys-Pal-D-Trp-Lys-Val-Cys-β-Nal-NH₂;
H₂-β-Nal-D-Cys-Tyr-D-Trp-Lys-Val-Cys-Thr-NH₂;
(H) (CH₃CO)-β-Nal-D-Cys-Tyr-D-Trp-Lys-Val-Cys-Thr-NH₂;
(H)(4-(2-hydroxyethyl)-1-piperazinylacetyl)-β-Nal-D-Cys-Tyr-D-Trp-Lys-Val-Cys-Thr-NH₂;
(H)(4-(2-hydroxyethyl)-1-piperizineethanesulfonyl)-β-Nal-D-Cys-Tyr-D-Trp-Lys-Val-Cys-Thr-NH₂;
H₂-β-Nal-D-Cys-Pal-D-Trp-Lys-Val-Cys-Thr-NH₂;
(H) (CH₃CO) -β-Nal-D-Cys-Pal-D-Trp-Lys-Val-Cys-Thr-NH₂;
(H) (4-(2-hydroxyethyl)-1-piperazinylacetyl)-β-Nal-D-Cys-Pal-D-Trp-Lys-Val-Cys-Thr-NH₂;
(H) (4-(2-hydroxyethyl)-1-piperizineethanesulfonyl)-β-Nal-D-Cys-Pal-D-Trp-Lys-Val-Cys-Thr-NH₂;
H₂-Phe-D-Cys-Tyr-D-Trp-Lys-Val-Cys-β-Nal-NH₂;
(H) (CH₃CO) Phe-D-Cys-Tyr-D-Trp-Lys-Val-Cys-β-Nal-NH₂;
(H) (4-(2-hydroxyethyl) -1-piperazinylacetyl) -Phe-D-Cys-Tyr-D-Trp-Lys-Val-Cys-β-Nal-NH₂;
(H)(4-(2-hydroxyethyl)-1-piperizineethanesulfonyl)-Phe-D-Cys-Tyr-D-Trp-Lys-Val-Cys-β-Nal-NH₂;
H₂-Phe-D-Cys-Pal-D-Trp-Lys-Val-Cys-β-Nal-NH₂ (Analog No. 4);
(H) (CH₃CO)Phe-D-Cys-Pal-D-Trp-Lys-val-Cys-β-Nal-NH₂;
(H) (4-(2-hydroxyethyl)-1-piperazinylacetyl)-Phe-D-Cys-Pal-D-Trp-Lys-Val-Cys-/3-Nal-NH₂;
(H) (4-(2-hydroxyethyl)-1-piperizineethanesulfonyl)-Phe-D-Cys-Pal-D-Trp-Lys-Val-Cys-β-Nal-NH₂;
H₂-Phe-D-Cys-Pal-D-Trp-Lys-Val-Cys-Thr-NH₂;
(H) (CH₃CO) -Phe-D-Cys-Pal-D-Trp-Lys-Val-Cys-Thr-NH₂
(H) (4-(2-hydroxyethyl)-1-piperazinylacetyl)-Phe-D-Cys-Pal-D-Trp-Lys-Val-Cys-Thr-NH₂;
(H) (4-(2-hydroxyethyl)-1-piperizineethanesulfonyl)-Phe-D-Cys-Pal-D-Trp-Lys-Val-Cys-Thr-NH₂;
H₂-β-Nal-D-Cys-Tyr-D-Trp-Lys-Thr-Cys-β-Nal-NH₂;
(H) (CH₃CO)-β-Nal-D-Cys-Pal-D-Trp-Lys-Thr-Cys-β-Nal-NH₂;
(H) (4-(2-hydroxyethyl)-1-piperazinylacetyl)-β-Nal-D-Cys-Tyr-D-Trp-Lys-Thr-Cys-β-Nal-NH₂;
(H) (4-(2-hydroxyethyl)-1-piperizineethanesulfonyl)-β-Nal-D-Cys-Tyr-D-Trp-Lys-Thr-Cys-β-Nal-NH₂;
H₂-β-Nal-D-Cys-Pal-D-Trp-Lys-Thr-Cys-β-Nal-NH₂;
(H) (CH₃CO)-β-Nal-D-Cys-Pal-D-Trp-Lys-Thr-Cys-β-Nal-NH₂;
(H) (4-(2-hydroxyethyl)-1-piperazinylacetyl)-β-Nal-D-Cys-Pal-D-Trp-Lys-Thr-Cys-β-Nal-NH₂;
(H) (4-(2-hydroxyethyl)-1-piperizineethanesulfonyl)-β-Nal-D-Cys-Pal-D-Trp-Lys-Thr-Cys-β-Nal-NH₂;
H₂-β-Nal-D-Cys-Tyr-D-Trp-Lys-Thr-Cys-Thr-NH₂;
H(CH₃CO)-β-Nal-D-Cys-Tyr-D-Trp-Lys-Thr-Cys-Thr-NH₂;
(H) (4- (2-hydroxyethyl) -1-piperazinylacetyl) -β-Nal-D-Cys-Tyr-D-Trp-Lys-Thr-Cys-Thr-NH₂;
(H) (4- (2-hydroxyethyl)-1-piperizineethanesulfonyl)-β-Nal-D-Cys-Tyr-D-Trp-Lys-Thr-Cys-Thr-NH₂;
H₂-β-Nal-D-Cys-Pal-D-Trp-Lys-Thr-Cys-Thr-NH₂
(H) (CH₃CO)-β-Nal-D-Cys-Pal-D-Trp-Lys-Thr-Cys-Thr-NH₂;
(H) (4- (2-hydroxyethyl) -1-piperazinylacetyl) -β-Nal-D-Cys-Pal-D-Trp-Lys-Thr-Cys-Thr-NH₂;
(H) (4-(2-hydroxyethyl)-1-piperizineethanesulfonyl)-β-Nal-D-Cys-Pal-D-Trp-Lys-Thr-Cys-Thr-NH₂;
H₂-Phe-D-Cys-Tyr-D-Trp-Lys-Thr-Cys-β-Nal-NH₂;
(H) (CH₃CO)Phe-D-Cys-Tyr-D-Trp-Lys-Thr-Cys-β-Nal-NH₂;
(H)(4-(2-hydroxyethyl)-1-piperazinylacetyl)Phe-D-Cys-Tyr-D-Trp-Lys-Thr-Cys-β-Nal-NH₂;
(H) (4- (2-hydroxyethyl)-1-piperizineethanesulfonyl) Phe-D-Cys-Tyr-D-Trp-Lys-Thr-Cys-β-Nal-NH₂;
H₂-Phe-D-Cys-Pal-D-Trp-Lys-Thr-Cys-β-Nal-NH₂;
(H) (CH₃CO)Phe-D-Cys-Pal-D-Trp-Lys-Thr-Cys-β-Nal-NH₂
(H)(4-(2-hydroxyethyl)-1-piperazinylacetyl)Phe-D-Cys-Pal-D-Trp-Lys-Thr-Cys-β-Nal-NH₂;
(H) (4-(2-hydroxyethyl) -1-piperizineethanesulfonyl) Phe- D-Cys-Pal-D-Trp-Lys-Thr-Cys-β-Nal-NH₂;
H₂-Phe-D-Cys-Tyr-D-Trp-Lys-Thr-Cys-Thr-NH₂;
(H) (CH₃CO)Phe-D-Cys-Tyr-D-Trp-Lys-Thr-Cys-Thr-NH₂
(H) (4-(2-hydroxyethyl)-1-piperazinylacetyl)Phe-D-Cys-Tyr-D-Trp-Lys-Thr-Cys-Thr-NH₂;
(H)(4-(2-hydroxyethyl)-:L-piperizineethanesulfonyl) Phe-D-Cys-Tyr-D-Trp-Lys-Thr-Cys-Thr-NH₂;
H₂-Phe-D-Cys- Pal -D-Trp-Lys-Thr-Cys -Thr-NH₂ (Analog No. 6);
(H) (CH₃CO)-Phe-D-Cys-Pal-D-Trp-Lys-Thr-Cys-Thr-NH₂;
(H) (4-(2-hydroxyethyl)-1-piperazinylacetyl)Phe-D-Cys-Pal-D-Trp-Lys-Thr-Cys-Thr-NH₂;
(H) (4- (2-hydroxyethyl)-1-piperizineethanesulfonyl) Phe-D-Cys-Pal-D-Trp-Lys-Thr-Cys-Thr-NH₂;
H₂-β-Nal-D-Cys-Tyr-D-Trp-Lys-Abu-Cys-β-Nal-NH₂;
H₂-Phe-D-Cys-Tyr-D-Trp-Lys-Abu-Cys-β-Nal-NH₂;
H₂-β-Nal-D-Cys-Pal-D-Trp-Lys-Abu-Cys-β-Nal-NH₂;
H₂-Phe-D-Cys-Pal-D-Trp-Lys-Abu-Cys-β-Nal-NH₂;
H₂-β-Nal-D-Cys-Tyr-D-Trp-Lys-Abu-Cys-Thr-NH₂;
H₂-Phe-D-Pen-Tyr-D-Trp-Lys-Val-Pen-Ø-Nal-NH₂; or
H₂-Phe-D-pen-Pal-D-Trp-Lys-Thr-Pen-Thr-NH₂;
H₂-Dip-D-Cys-Pal-D-Trp-Lys-Val-Cys-Dip-NH₂ (Analog No. 10);
H₂-F₅-Phe-D-Cys-His-D-Trp-Lys-Val -Cys-F₅-Phe-NH₂ (Analog No. 11);
H₂-Dip-D-Cys-Pal-D-Trp-Lys-Val-Cys-β-Nal-NH₂ (Analog No. 13);
H₂-m-F-Phe-D-Cys-Pal-D-Trp-Lys-Val-Cys-m-F-Phe-NH₂ (Analog No. 14);
H₂-O-F-Phe-D-Cys-Pal-D-Trp-Lys-Val-Cys-o-F-Phe-NH₂ (Analog No. 15) ;
H₂-p-F-Phe-D-Cys-Pal-D-Trp-Lys-Val-Cys-p-F-Phe-NH₂ (Analog No. 12);
H₂-F₅-Phe-D-Cys-Pal-D-Trp-Lys-Val-Cys-F₅-Phe-NH₂ (Analog No. 16);
H₂-F₅-Phe-D-Cys -2 -Pal -D-Trp-Lys-Val -Cys -F₃-Phe-NH₂ (Analog No. 17);
H₂-β-Nal-D-Cys-His-D-Trp-Lys-Val-Cys-β-Nal-NH₂ (Analog No. 19);
H₂-Dip-D-Cys-His-D-Trp-Lys-Val-Cys-β-Nal-NH₂ (Analog No. 20);
H₂-Dip-D-Cys-His-D-Trp-Lys-Val-Cys-Dip-NH₂ (Analog No. 21);
H₂-β-Nal-D-Cys-His-D-Trp-Lys-Val-Cys-β-Nal-NH₂ (Analog No. 22);
H₂-Trp-D-Cys-Tyr-D-Trp-Lys-Val-Cys-D-β-Nal-NH₂ (Analog No. 24);
H₂-β-Nal-D-Cys-Tyr-D-Trp-Lys-Val-Cys-D-β-Nal-NH₂ (Analog No. 25);
H₂-β-Nal-D-Cys-Pal-D-Trp-Lys-Val-Cys-D-p-F-Phe-NH₂ (Analog No. 28);
H₂-β-Nal-D-Cys-Pal-D-Trp-Lys-Tle-Cys-β-Nal-NH₂ (Analog No. 29);
H₂-p-F-Phe-D-Cys-Pal-D-Trp-Lys-Val-Cys-β-Nal-NH₂ (Analog No. 30);
H₂-β-Nal-D-Cys-Pal-D-Trp-Lys-Nle-Cys-β-Nal-NH₂, (Analog No. 31) ;
H2-β-Nal-D-Cys-Pal-D-Trp-Lys-Ile-Cys-β-Nal-NH₂, (Analog No. 32); .
H₂-β-Nal-D-Cys-Pal-D-Trp-Lys-Gly-Cys-β-Nal-NH₂ (Analog No. 33);
H₂-β-Nal-D-Cys-Pal-D-Trp-Lys-Ala-Cys-β-Nal-NH₂ (Analog No. 34);
H₂-β-Nal-D-Cys-Pal-D-Trp-Lys-Leu-Cys-β-Nal-NH₂ (Analog No. 35);
H₂-Bip-D-Cys-Tyr-D-Trp-Lys-Ile-Cys-Bip-NH₂ (Analog No. 36);
H₂-p-F-Phe-D-Cys-His-D-Trp-Lys-Val-Cys-p-F-Phe-NH₂ (Analog No. 38);
H₂-Npa-D-cys-Pal-D-Trp-Lys-Val-Cys-Tyr-NH₂ (Analog No. 39);
H₂-m-F-Phe-D-Cys-His-D-Trp-Lys-Val-Cys-m-F-Phe-NH₂ (Analog No. 40);
H₂-o-F-Phe-D-Cys-His-D-Trp-Lys-Val-Cys-o-F-Phe-NH₂ (Analog No. 41);
H₂-β-Nal-D-Cys-Pal-D-Trp-Lys-Val-Cys-Dip-NH₂ (Analog No. 42);
H₂-Cpa-D-Cys-Pal-D-Trp-Lys-Val-Cys-Cpa-NH₂ (Analog No. 43);
H₂-Igl-D-Cys-Pal-D-Trp-Lys-Val-Cys-Igl-NH₂ (Analog No. 44);
H₂-P-Nal-D-Cys-Pal-D-Trp-Lys-Val-Cys-D-Dip-NH₂ (Analog No. 45) ;
H₂-β-Nal-D-Cys-3-I-Tyr-D-Trp-Lys-Val-Cys-β-Nal-NH₂ (Analog No. 46);
H₂-p-CN-Phe-D-Cys-Pal-D-Trp-Lys-Val-Cys-p-CN-Phe-NH₂ (Analog No. 47);
H₂-β-Nal-D-Cys-Tyr-D-Trp-Lys-Val-Cys-D-Dip-NH₂ (Analog No. 48);
H₂-β-Nal-D-Cys-Bta-D-Trp-Lys-Val-Cys-p-Nal-NH-, (Analog No. 49) ;
H₂-p-F-Phe-D-Cys-Pal-D-Trp-Lys-Tle-Cys-β-Nal-NH₂ (Analog No. 50);
H₂-Bpa-D-Cys-Pal-D-Trp-Lys-Val-Cys-Bpa-NH₂ (Analog No. 52);
H₂-Iph-D-Cys-Pal-D-Trp-Lys-Val-Cys-Iph-NH₂ (Analog No. 53);
H₂-Trp-D-Cys-Pal-D-Trp-Lys-Tle-Cys-β-Nal-NH₂ (Analog No. 54);
H₂-p-Cl-Phe-D-Cys-Pal-D-Trp-Lys-Val-Cys-β-Nal-NH₂ (Analog No. 55);
H₂-p-Cl-Phe-D-Cys-Pal-D-Trp-Lys-Tle-Cys-β-Nal-NH₂ (Analog No. 56);
H₂-p-Cl-Phe-D-Cys-Pal-D-Trp-Lys-Tle-Cys-p-Cl-Phe-NH₂ (Analog No. 57) ;
H₂-p-Cl-Phe-D-Cys-Pal-D-Trp-Lys-Cha-Cys-p-Cl-Phe-NH₂;
H₂-p-Cl-Phe-D-Cys-Tyr(I)-D-Trp-Lys-Val-Cys-p-CI-Phe-NH₂;
H₂-p-Cl-Phe-D-Cys-Tyr(I)-D-Trp-Lys-Val-Cys-β-Nal-NH₂;
H₂-P-Cl-Phe-D-Cys-Tyr(I)-D-Trp-Lys-Tle-Cys-β-Nal-NH₂;
H₂-p-F-Phe-D-Cys-Tyr(I)-D-Trp-Lys-Val-Cys-β-Nal-NH₂;
H₂-p-F-Phe-D-Cys-Tyr(I)-D-Trp-Lys-Tle-Cys-β-Nal-NH₂;
H₂-β-Nal-D-Cys-Tyr-D-Trp-Lys-Abu-Cys-β-Nal-NH₂;
(H) (CH₃CO) -β-Nal-D-Cys-Tyr-D-Trp-Lys-Abu-Cys-β-Nal-NH₂;
H₂-p-NO0₂-Phe-D-Cys-Tyr-D-Trp-Lys-Abu-Cys-β-Nal-NH₂;
(H) (CH₃CO) -p-NO₂-Phe-D-Cys-Tyr-D-Trp-Lys-Abu-Cys-β-Nal-NH₂;
H₂-p-NO₂-Phe-D-Cys-Tyr(Bzl) -D-Trp-Lys-Thr(Bzl) *-*Cys-β-Nal- NH₂;
(H) (4- (2-hydroxyethyl)-1-piperazinylacetyl) -p-NO₂-Phe-D-Cys-Tyr(Bzl)-D-Trp-Lys-Thr(Bzl)-Cys-β-Nal-NH₂;
(H)(4-(2-hydroxyethyl)-1-piperazinylacetyl)-p-NO₂-Phe-D-Cys-Tyr-D-Trp-Lys-Thr-Cys-Tyr-NH₂;
H₂-p-NO₂-Phe-D-Cys-Tyr-D-Trp-Lys-Val-Cys-β-Nal-NH₂;
(H) (4- (2-hydroxyethyl) -1-piperazinylacetyl) -p-NO₂-Phe-D-Cys-Tyr-D-Trp-Lys-Val-Cys-β-Nal-NH₂;
(H) (4-(2-hydroxyethyl)-1-píperazinylacetyl)-β-Nal-Phe-D-Cys-Tyr-D-Trp-Lys-Val-Cys-β-Nal-NH₂;
H₂,-β-Nal-D-Cys-Tyr(Bzl)-D-Trp-Lys-Thr(Bzl)-Cys-β-Nal-NH₂;
(H) (4-2-hydroxyethyl)-1-piperazinylacetyl)-β-Nal-D-Cys-Tyr(Bzl)-D-Trp-Lys-Thr(Bzl)-Cys-Tyr(Bzl)-NH₂;
H₂-D-Phe-D-Pen-Tyr-D-Trp-Lys-Val-Cys-Thr-NH₂;
H₂-D-β-Nal-D-Cys-Tyr-D-Trp-Lys-Val-Cys-Thr-NH₂;
H₂-D-β-Nal-D-Cys-Tyr-D-Trp-Lys-Val-Cys-β-Nal-NH₂ (Analog No. 9);
H₂-D-β-Nal-D-Cys-Tyr-D-Trp-Lys-Thr-Cys-β-Nal-NH₂;
H₂-D-Phe-D-Cys-Pal-D-Trp-Lys-Thr-Cys-Thr-NH₂;
H₂-D-Phe-D-Cys-Tyr-D-Trp-Lys-Abu-Cys-Thr-NH₂;
H₂-D-β-Nal-D-Cys-Tyr-D-Trp-Lys-Abu-Cys-Thr-NH₂;
H₂-D-β-Nal-D-Cys-Tyr-D-Trp-Lys-Val-Cys-D-β-Nal-NH₂ (Analog No. 26);
H₂-D-p-F-Phe-D-Cys-Pal-D-Trp-Lys-Val-Cys-D-p-F-Phe-NH₂ (Analog No. 27);
H₂-D-Bip-D-Cys-Tyr-D-Trp-Lys-Val-Cys-β-Nal-NH₂ (Analog No. 37);
H₂-D-Dip-D-Cys-Pal-D-Trp-Lys-Val-Cys-β-Nal-NH₂ (Analog No. 18);
H₂-D-p-F-Phe-D-Cys-Pal-D-Trp-Lys-Tle-Cys-β-Nal-NH₂ (Analog No. 51);
H₂-D-p-Cl-Phe-D-Cys-Pal-D-Trp-Lys-Tle-Cys-p-Cl-Phe-NH₂ (Analog No. 7) ;
p-NO₂-D-Phe-D-Cys-Pal-D-Trp-Lys-Thr(Bzl)-Cys-Tyr(Bzl)-NH₂;
p-NO₂-D-Phe-D-Cys-Tyr (Bzl) -D-Trp-Lys-Val-Cys-Tyr (Bzl)-NH₂;
(H) (4-(2-hydroxyethyl)-1-piperazinylacetyl) -p-NO₂-D-Phe-D-Cys-Pal-D-Trp-Lys-Thr(Bzl)-Cys-Tyr(Bzl)-NH₂;
(H) (4- (2-hydroxyethyl) -1-piperazinylacetyl) -p-NO₂-D-Phe-D-Cys-Tyr(Bzl)-D-Trp-Lys-Val-Cys-Tyr(Bzl)-NH₂;
(H) (3-phenylpropionyl)-D-Cys-Tyr-D-Trp-Lys-Val-Cys-β-Nal- NH₂;
(H) (3-phenylpropionyl)-D-Cys-Pal-D-Trp-Lys-Val-Cys-β-Nal-NH₂ ;
(H) (3-phenylpropionyl)-D-Cys-Tyr-D-Trp-Lys-Thr-Cys-β-Nal-NH₂;
(H) (3-phenylpropionyl)-D-Cys-Pal-D-Trp-Lys-Thr-Cys-β-Nal-NH₂;
(H) (3-phenylpropionyl)-D-Cys-Tyr-D-Trp-Lys-Val-Cys-Thr-NH₂;
(H)(3-phenylpropionyl)-D-Cys-Pal-D-Trp-Lys-Val-Cys-Thr-NH₂;
(H)(3-phenylpropionyl)-D-Cys-Tyr-D-Trp-Lys-Thr-Cys-Thr-NH₂ ;
(H)(3-phenylpropionyl)-D-Cys-Pal-D-Trp-Lys-Thr-Cys-Thr-NH₂ ;
(H)(3-[2-naphthyl]propionyl)-D-Cys-Tyr-D-Trp-Lys-Val-Cys-β-Nal-NH₂;
(H) (3-[2-naphthyl]propionyl)-D-Cys-Pal-D-Trp-Lys-Val-Cys-β-Nal-NH₂;
(H) (3- [2-naphthyl]propionyl)-D-Cys-Tyr-D-Trp-Lys-Thr-Cys -β-Nal-NH₂;
(H) (3- [2-naphthyl] propionyl) -D-Cys-Pal-D-Trp-Lys-Thr-Cys-β-Nal-NH₂ ;
(H) (3- [2-naphthyl] propionyl) -D-Cys-Tyr-D-Trp-Lys-Val-Cys-Thr-NHZ ;
(H)(3- [2-naphthyl] propionyl) -D-Cys-Pal-D-Trp-Lys-Val-Cys-Thr-NH₂;
(H) (3- [2-naphthyl]propionyl)-D-Cys-Tyr-D-Trp-Lys-Thr-Cys -Thr-NH₂;
(H) (3- [2-naphthyl]propionyl)-D-Cys-Pal-D-Trp-Lys-Thr-Cys -Thr-NH₂;
(H)(3-[p-hydroxyphenyl])-D-Cys-Tyr-D-Trp-Lys-Val-Cys-β-Nal-NH₂ ;
(H) (3-naphthyl]propionyl)-D-Cys-Tyr-D-Trp-Lys-Abu-Cys-β-Nal-NH₂ ;
(H) (3-naphthyl]propionyl)-D-Cys-Tyr-D-Trp-Lys-Abu-Cys-Thr-NH₂ ;
(H)(3-phenyly-lpropionyl)-D-Cys-Tyr-D-Trp-Lys-Abu-Cys-β-Nal-NH₂
(H)(3-phenylylpropionyl)-D-Cys-Tyr-D-Trp-Lys-Abu-Cys-Thr-NH₂ ;
H₂-β-Nal-D-Cys -Tyr-D-Trp-Lys-Val-Cys-2R,3R- (2-hydroxymethyl)-3-hydroxy)propylamide;
(H) (CH₃CO)-β-Nal-D-Cys-Tyr-D-Trp-Lys-Val-Cys-2R,3R- (2-hydroxymethyl)-3-hydroxy)propylamide;
(H) (4-(2-hydroxyethyl)-1-piperazinylacetyl)-β-Nal-D-Cys-Tyr-D-Trp-Lys-Val-Cys-2R,3R-(2-hydroxymethyl)-3-hydroxy)propylamide;
(H) (4-(2-hydroxyethyl)-1-piperizineethanesulfonyl)-β-Nal-D-Cys-Tyr-D-Trp-Lys-Val-Cys-2R,3R-(2-hydroxymethyl)-3-hydroxy)propylamide;
H₂-(*β*-Nal-D-Cys-Pal-D-Trp-Lys-Val-Cys-2R,3R-(2-hydroxymethyl) -3-hydroxy)propylamide;
(H) (CH₃CO)-β-Nal-D-Cys-Pal-D-Trp-Lys-Val-Cys-2R,3R- (2-hydroxymethyl)-3-hydroxy)propylamide;
(H) (4-(2-hydroxyethyl)-1-piperazinylacetyl)-β-Nal-D-Cys-Pal-D-Trp-Lys-Val-Cys-2R,3R-(2-hydroxymethyl)-3-hydroxy)propylamide;
(H) (4-(2-hydroxyethyl)-1-piperizineethanesulfonyl)-β-Nal-D-Cys-Pal-D-Trp-Lys-Val-Cys-2R,3R-(2-hydroxymethyl)-3-hydroxy)propylamide;
H₂-β-Nal-D-Cys-Tyr-D-Trp-Lys-Thr-Cys-2R,3R-(2-hydroxymethyl)-3-hydroxy)propylamide;
(H) (CH₃CO)-β-Nal-D-Cys-Tyr-D-Trp-Lys-Thr-Cys-2R, 3R- (2-hydroxymethyl)-3-hydroxy)propylamide;
(H) (4-(2-hydroxyethyl)-1-piperazinylacetyl)-β-Nal-D-Cys-Tyr-D-Trp-Lys-Thr-Cys-2R,3R-(2-hydroxymethyl)-3-hydroxy)propylamide;
(H)(4-(2-hydroxyethyl)-1-piperizineethanesulfonyl)-β-Nal-D-Cys-Tyr-D-Trp-Lys-Thr-Cys-2R,3R-(2-hydroxymethyl)-3-hydroxy)propylamide;
H₂-β-Nal-D-Cys-Pal-D-Trp-Lys-Thr-Cys-2R,3R-(2-hydroxymethyl)-3-hydroxy)propylamide;
(H) (CH₃CO)-β-Nal-D-Cys-Pal-D-Trp-Lys-Thr-Cys-2R, 3R-(2-hydroxymethyl)-3-hydroxy)propylamide;
(H) (4-(2-hydroxyethyl)-1-piperazinylacetyl)-β-Nal-D-Cys-Pal-D-Trp-Lys-Thr-Cys-2R,3R-(2-hydroxymethyl)-3-hydroxy)propylamide;
(H) (4-(2-hydroxyethyl)-1-piperizineethanesulfonyl)-β-Nal-D-Cys-Pal-D-Trp-Lys-Thr-Cys-2R,3R-(2-hydroxymethyl)-3-hydroxy)propylamide;
H₂-Phe-D-Cys-Tyr-D-Trp-Lys-Val-Cys-2R,3R-(2-hydroxymethyl)-3-hydroxy)propylamide;
(H) (CH₃CO) Phe-D-Cys-Tyr-D-Trp-Lys-Val-Cys-2R, 3R- (2-hydroxymethyl)-3-hydroxy)propylamide;
(H)(4-(2-hydroxyethyl)-1-piperazinylacetyl)Phe-D-Cys-Tyr-D-Trp-Lys-Val-Cys-2R,3R-(2-hydroxymethyl)-3-hydroxy)propylamide;
(H)(4-(2-hydroxyethyl)-1-piperizineethanesulfonyl) Phe-D-Cys-Tyr-D-Trp-Lys-Val-Cys-2R,3R-(2-hydroxymethyl)-3-hydroxy)propylamide;
H₂-Phe-D-Cys-Pal-D-Trp-Lys-Val-Cys-2R,3R-(2-hydroxymethyl)-3-hydroxy)propylamide;
H(CH₃CO) Phe-D-Cys-Pal-D-Trp-Lys-Val-Cys-2R,3R- (2-hydroxymethyl)-3-hydroxy)propylamide;
(H) (4-(2-hydroxyethyl)-1-piperazinylacetyl)Phe-D-Cys-Pal-D-Trp-Lys-Val-Cys-2R,3R-(2-hydroxymethyl)-3-hydroxy)propylamide;
(H) (4-(2-hydroxyethyl)-1-piperizineethanesulfonyl) Phe-D-Cys-Pal-D-Trp-Lys-Val-Cys-2R,3R-(2-hydroxymethyl)-3-hydroxy)propylamide;
H₂-Phe-D-Cys-Tyr-D-Trp-Lys-Thr-Cys-2R,3R-(2-hydroxymethyl)-3-hydroxy)propylamide;
(H) (CH₃CO) Phe-D-Cys-Tyr-D-Trp-Lys-Thr-Cys-2R, 3R- (2-hydroxymethyl)-3-hydroxy)propylamide;
(H)(4-(2-hydroxyethyl)-1-piperazinylacetyl)Phe-D-Cys-Tyr-D-Tr-p-Lys-Thr-Cys-2R,3R-(2-hydroxymethyl)-3-hydroxy)propylamide;
(H) (4-(2-hydroxyethyl)-1-piperizineethanesulfonyl) Phe-D-Cys-Tyr-D-Trp-Lys-Thr-Cys-2R,3R-(2-hydroxymethyl)-3-hydroxy)propylamide;
H₂-Phe-D-Cys-Pal-D-Trp-Lys-Thr-Cys-2R, 3R-(2-hydroxymethyl)-3-hydroxy)propylamide;
(H) (CH₃CO) Phe-D-Cys-Pal-D-Trp-Lys-Thr-Cys-2R, 3R- (2-hydroxymethyl)-3-hydroxy)propylamide;
(H) (4-(2-hydroxyethyl)-1-piperazinylacetyl)Phe-D-Cys-Pal-D-Trp-Lys-Thr-Cys-2R,3R-(2-hydroxymethyl)-3-hydroxy)propylamide;
(H)(4-(2-hydroxyethyl)-1-piperizineethanesulfonyl) Phe-D-Cys-Pal-D-Trp-Lys-Thr-Cys-2R,3R-(2-hydroxymethyl)-3-hydroxy)propylamide;
H₂-β-Nal-D-Cys-Tyr-D-Trp-Lys-Val-Cys-2R-(2-naphthyl)ethylamide;
(H) (CH₃CO) -β-Nal -D- Cys -Tyr-D-Trp-Lys -Val - Cys - 2R- (2-naphthyl)ethylamide;
(H) (4- (2-hydroxyethyl) -1-piperazinylacetyl) -β-Nal-D-Cys-Tyr-D-Trp-Lys-Val-Cys-2R-(2-naphthyl)ethylamide;
(H) (4- (2-hydroxyethyl) -1-piperizineethanesulfonyl)-β-Nal-D-Cys-Tyr-D-Trp-Lys-Val-Cys-2R-(2-naphthyl)ethylamide;
H₂-β-Nal-D-Cys-Pal-D-Trp-Lys-Val-Cys-2R- (2-naphthyl)ethylamide;
(H) (CH₃CO)-β-Nal-D-Cys-Pal-D-Trp-Lys-Val-Cys-2R- (2-naphthyl)ethylamide;
(H) (4- (2-hydroxyethyl) -1-piperazinylacetyl) -β-Nal-D-Cys-Pal-D-Trp-Lys-Val-Cys-2R-(2-naphthyl)ethylamide;
(H) (4- (2-hydroxyethyl)-1-piperizineethanesulfonyl) -β-Nal-D-Cys-Pal-D-Trp-Lys-Val-Cys-2R-(2-naphthyl)ethylamide;
H₂-β-Nal-D-Cys-Tyr-D-Trp-Lys-Thr-Cys-2R-(2-naphthyl) ethylamide;
(H) (CH₃CO)-β-Nal-D-Cys-Tyr-D-Trp-Lys-Thr-Cys-2R-(2-naphthyl)ethylamide;
(H) (4-(2-hydroxyethyl)-1-piperazinylacetyl)-β-Nal-D-cys-Tyr-D-Trp-Lys-Thr-Cys-2R-(2-naphthyl)ethylamide;
(H) (4-(2-hydroxyethyl)-1-piperizineethanesulfonyl)-β-Nal-D-Cys-Tyr-D-Trp-Lys-Thr-Cys-2R-(2-naphthyl)ethylamide;
H₂-β-Nal-D-Cys-Pal-D-Trp-Lys-Thr-Cys-2R-(2-naphthyl)ethylamide;
(H) (CH₃CO)-β-Nal-D-Cys-Pal-D-Trp-Lys-Thr-Cys-2R-(2-naphthyl)ethylamide;
(H) (4-(2-hydroxyethyl)-1-piperazinylacetyl)-β-Nal-D-Cys-Pal-D-Trp-Lys-Thr-Cys-2R-(2-naphthyl)ethylamide;
(H)(4-(2-hydroxyethyl)-1-piperizineethanesulfonyl)-β-Nal-D-Cys-Pal-D-Trp-Lys-Thr-Cys-2R-(2-naphthyl)ethylamide;
H₂-Phe-D-Cys-Tyr-D-Trp-Lys-Val-Cys-2R-(2-naphthyl)ethylamide;
(H) (CH₃CO)Phe-D-Cys-Tyr-D-Trp-Lys-Val-Cys-2R- (2-naphthyl)ethylamide;
(H)(4-(2-hydroxyethyl)-1-piperazinylacetyl)Phe-D-Cys-Tyr-D-Trp-Lys-Val-Cys-2R-(2-naphthyl)ethylamide;
(H)(4-(2-hydroxyethyl)-1-piperizineethanesulfonyl) Phe-D-Cys-Tyr-D-Trp-Lys-Val-Cys-2R-(2-naphthyl)ethylamide;
H₂-Phe-D-Cys -Pal-D-Trp-Lys-Val-Cys-2R-(2-naphthyl) ethylamide;
(H) (CH₃CO)Phe-Cys-Pal-D-Trp-Lys-Val-Cys-2R- (2-naphthyl)ethylamide;
(H) (4- (2-hydroxyethyl) -1-piperazinylacetyl) Phe-D-Cys-Pal-D-Trp-Lys-Val-Cys-2R-(2-naphthyl)ethylamide;
(H)(4-(2-hydroxyethyl)-1-piperizineethanesulfonyl) Phe-D-Cys-Pal-D-Trp-Lys-Val-Cys-2R-(2-naphthyl)ethylamide;
H₂-Phe-D-Cys-Tyr-D-Trp-Lys-Thr-Cys-2R- (2-naphthyl) ethylamide;
(H) (CH₃CO)Phe-D-Cys-Tyr-D-Trp-Lys-Thr-Cys-2R- (2-naphthyl)ethylamide;
(H) (4-(2-hydroxyethyl)-1-piperazinylacetyl) Phe-D-Cys-Tyr-D-Trp-Lys-Thr-Cys-2R-(2-naphthyl)ethylamide;
(H)(4-(2-hydroxyethyl)-1-piperizineethanesulfonyl) Phe-D-Cys-Tyr-D-Trp-Lys-Thr-Cys-2R-(2-naphthyl)ethylamide;
H₂-Phe-D-Cys-Pal-D-Trp-Lys-Thr-Cys-2R-(2-naphthyl)ethylamide;
(H) (CH₃CO) Phe-D-Cys-Pal-D-Trp-Lys-Thr-Cys-2R-(2-naphthyl)ethylamide;
(H)(4-(2-hydroxyethyl)-1-piperazinylacetyl)Phe-D-Cys-Pal-D-Trp-Lys-Thr-Cys-2R-(2-naphthyl)ethylamide;
(H) (4-(2-hydroxyethyl)-1-piperizineethanesulfonyl) Phe-D-Cys-Pal-D-Trp-Lys-Thr-Cys-2R-(2-naphthyl)ethylamide;
H₂-β-Nal-D-Cys-Tyr-D-Trp-Lys-Abu-Cys-2R- (2-naphthyl)ethylamide;
H₂-Phe-D-Cys-Tyr-D-Trp-Lys-Abu-Cys-2R-(2-naphthyl)ethylamide;
H₂-β-Nal-D-Cys-Tyr-D-Trp-Lys-Abu-Cys-2R,3R-(2-hydroxymethyl)-3-hydroxy)propylamide;
H₂-Phe-D-Cys-Tyr-D-Trp-Lys-Abu-Cys-2R,3R-(2-hydroxymethyl)-3-hydroxy)propylamide;
H₂-Phe-D-Phe-Tyr-D-Trp-Lys-Thr-Phe-Thr-NH₂;
H₂-Phe-D-Phe-Tyr-D-Trp-Lys-Val-Phe-Thr-NH₂;
H₂-Phe-D-Cpa-Tyr-D-Trp-Lys-Val-Phe-Thr-NH₂;
H₂-β-Nal-D-Cpa-Tyr-D-Trp-Lys-Val-Phe-Thr-NH₂ (Analog No.
(H) (CH₃CO)-β-Nal-D-Cpa-Tyr-D-Trp-Lys-Val-Phe-Thr-NH₂;
(H)(4-(2-hydroxyethyl)-1-piperazinylacetyl)-β-Nal-D-Cpa-Tyr-D-Trp-Lys-Val-Phe-Thr-NH₂;
(H) (4-(2-hydroxyethyl)-1-piperizineethanesulfonyl)-β-Nal-D-Cpa-Tyr-D-Trp-Lys-Val-Phe-Thr-NH₂;
H₂-β-Nal-D-Cpa-Pal-D-Trp-Lys-Val-Phe-Thr-NH₂;
(H) (CH₃CO)-β-Nal-D-Cpa-Pal-D-Trp-Lys-Val-Phe-Thr-NH₂;
(H)(4-(2-hydroxyethyl)-1-piperazinylacetyl)-β-Nal-D-Cpa-Pal-D-Trp-Lys-Val-Phe-Thr-NH₂;
(H) (4-(2-hydroxyethyl)-1-piperizineethanesulfonyl)-β-Nal-D-Cpa-Pal-D-Trp-Lys-Val-Phe-Thr-NH₂;
H₂,-β-Nal-D-Cpa-Tyr-D-Trp-Lys-Thr-Phe-Thr-NH₂;
(H) (CH₃CO)-β-Nal-D-Cpa-Tyr-D-Trp-Lys-Thr-Phe-Thr-NH₂;
(H) (4-(2-hydroxyethyl)-1-piperazinylacetyl)-β-Nal-D-Cpa-Tyr-D-Trp-Lys-Thr-Phe-Thr-NH;
(H) (4-(2-hydroxyethyl)-1-piperizineethanesulfonyl)-β-Nal-D-Cpa-Tyr-D-Trp-Lys-Thr-Phe-Thr-NH₂;
H₂-β-Nal-D-Cpa-Pal-D-Trp-Lys-Thr-Phe-Thr-NH₂
(H) (CH₃CO) -β-Nal-D-Cpa-Pal-D-Trp-Lys-Thr- Phe-Thr-NH₂;
(H)(₄-(2-hydroxyethyl)-1-piperazinylacetyl)-β-Nal-D-Cpa-Pal-D-Trp-Lys-Thr-Phe-Thr-NH₂;
(H) (4-(2-hydroxyethyl)-1-piperizineethanesulfonyl)-β-Nal-D-Cpa-Pal-D-Trp-Lys-Thr-Phe-Thr-NH₂;
H₂-P-Nal-D-Cpa-Tyr-D-Trp-Lys-Val-Phe-β-Nal-NH₂
(H) (CH₃CO)-β-Nal-D-Cpa-Tyr-D-Trp-Lys-Val-Phe-β-Nal-NH₂
(H)(4-(2-hydroxyethyl)-1-piperazinylacetyl)-β-Nal-D-Cpa-Tyr-D-Trp-Lys-Val-Phe-β-Nal-NH₂; or
(H) (4-(2-hydroxyethyl)-1-piperizineethanesulfonyl)-β-Nal-D-Cpa-Tyr-D-Trp-Lys-Val-Phe-β-Nal-NH₂;
H₂-β-Nal-D-Cpa-Tyr-D-Trp-Lys-Val-Phe-β-Nal-NH₂ (Analog No. 23);
H₂-β-Nal-D-Cpa-Tyr-D-Trp-Lys-Val-Phe-Thr-NH₂;
H₂-D-β-Nal-D-Cpa-Phe-D-Trp-Lys-Val-Phe-Thr-NH₂;
H₂-D-β-Nal-D-Phe-Tyr-D-Trp-Lys-Thr-Phe-Thr-NH₂;
H₂-D-Phe-D-Phe-Tyr-D-Trp-Lys-Val-Phe-Thr-NH₂;
H₂-D-β-Nal-D-Cpa-Tyr-D-Trp-Lys-Val-Phe-Thr-NH₂
(Analog No. 8); or
H₂-D-β-Nal-D-Cpa-Tyr-D-Trp-Lys-Val-Phe-β-Nal-NH₂; or
a pharmaceutically acceptable salt thereof.

With the exception of the N-terminal amino acid, all abbreviations (e.g., Ala or A₂) of amino acids in this disclosure stand for the structure of -NH-CH(R)-CO-, wherein R is a side chain of an amino acid (e.g., CH₃ for Ala). For the N-terminal amino acid, the abbreviation stands for the structure of =N-CH(R)-CO-, wherein R is a side chain of an amino acid. Pen, β-Ala, Gaba, Nle, Nva, Pal, F₅-Phe, 2,4-dichloro-Phe, Cpa, β-Nal, β-1-Nal, Abu, Dip, 2-Pal, Bip, Npa, Igl, Bta, Tle, Bpa, Iph, Cha, Thr(Bzl), -Tyr (Bzl) and Aib are respective abbreviations of the following α-amino acids: penicillamine, 3-aminopropionic acid, 4-aminobutyric acid, norleucine, norvaline, β- [3-pyridyl] -alanine, β-[2,3,4,5,6-pentafluorophenyl]-alanine,β-[2,4-dichlorophenyl]-alanine, β[4-chlorophenyll-alanine, β-[2-napthyll-alanine, β-[1-naphthyl]-alanine; 2-aminobutyric acid, 3, 3'-diphenylalanine, β-[2-pyridyl]-alanine, 4, 4'-biphenylalanine, p-NO₂phenylalanine, 2-indanylglycine, 3-benzothienylalanine, α-[t-butyl]-glycine, 4-bromophenylalanine, 4-iodo-phenylalanine, β-(cyclohexyl)-alanine, O-benzyl-threonine, O-benzyl-tyrosine, and 2-aminoisobutyric acid. Tyr(I) refers to an iodinated tyrosine residue (e.g., 3-1-Tyr, 5-I-Tyr, 3,5-I-Tyr) wherein the iodine may be a radioactive isotope, e.g., I₁₂₅, I₁₂₇, or I₁₃₁. An aliphic amino acid is an α-amino acid having one or two side chains which, independently, are hydrocarbons, e.g., a straight or branched chain of 1-6 carbons. Examples of aliphatic amino acids include Ala, Aib, Val, Leu, Tle, Ile, Nle, Nva, or Abu. An aromatic amino acid is an α-amino acid the side chain of which has a neutral (e.g., not acidic or basic) aromatic substituent, e.g., a substituted or unsubstituted phenyl, naphthyl, or aromatic heterocycle group (e.g., pyridyl or indolyl). Examples of aromatic amino acids include Phe, p-X-Phe (where X is a halo (e.g., F, Cl, Br, or I), OH, OCH₃, CH₃, or NO₂), o-X-Phe (where X is a halo, OH, OCH₃, CH₃, or NO₂), m-X-Phe (where X is a halo, OH, OCH₃, CH₃, or NO₂), His, Pal, Trp, β-Nal, 2,4-dichloro-Phe, Tyr(I), β-[3,4,5-trifluorophenyl]-alanine, Bta, β-[3-cyanophenyl]alanine, β-[4-cyanophenyl]-alanine, β-[3,4-difluorophenyl]-alanine, β-[3,5-difluorophenyl]-alanine, β-[2-fluorophenyl]alanine, β-[4-thiazolyl]-alanine, Bip, Dip, Npa, Igl, Bpa, Iph, homophenylalanine, 2-Pal, β-[4-pyridyl]-alanine, β-[4-thiazolyl]-alanine, β-[2-thiazolyl]-alanine, para-(CF₃)-phenylalanine, and F₅, -Phe. What is meant by an "Eaa" is an amino acid of the formula -NH- [CH(R)ₙ,-CO- (where n is 2-6 and R is H, lower alkyl, or hydroxy lower alkyl). Examples of an Eaa include β-Ala and Gaba.

As used herein, "lower alkyl", is intended to include both branched and straight-chain saturated aliphatic hydrocarbon groups having 1-6 carbon atoms. Examples of lower alkyl groups include methyl, ethyl, propyl, isopropyl, butyl, t-butyl, isobutyl, sec-butyl, and the like.

As used herein, "aryl", is intended to include any stable monocyclic, bicyclic, or tricyclic carbon ring(s) of up to 7 members in each ring, wherein at least one ring is aromatic. Examples of aryl groups include phenyl, naphthyl, anthracenyl, biphenyl, tetrahydronaphthyl, indanyl, phenanthrenyl, and the like.

The term "heterocyclyl", as used herein, represents a stable 5- to 7-membered monocyclic or stable 8- to 11-membered bicyclic or stable 11-15 membered tricyclic heterocyclic ring which is either saturated or unsaturated, and which consists of carbon atoms and from one to four heteroatoms selected from the group consisting of N, O, and S, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include, but are not limited to, azepinyl, benzimidazolyl, benzisoxazolyl, benzofurazanyl, benzopyranyl, benzothiopyranyl, benzofuryl, benzothiazolyl, benzothienyl, benzoxazolyl, chromanyl, cinnolinyl, dihydrobenzofuryl, dihydrobenzothienyl, dihydrobenzothiopyranyl, dihydrobenzothio-pyranyl sulfone, furyl, imidazolidinyl, imidazolinyl, imidazolyl, indolinyl, indolyl, isochromanyl, isoindolinyl, isoquinolinyl, isothiazolidinyl, isothiazolyl, isothiazolidinyl, morpholinyl, naphthyridinyl, oxadiazolyl, 2-oxoazepinyl, 2-oxopiperazinyl, 2-oxopiperidinyl, 2-oxopyrrolidinyl, piperidyl, piperazinyl, pyridyl, pyridyl N-oxide, quinoxalinyl, tetrahydrofuryl, tetrahydroisoquinolinyl, tetrahydroisoquinolinyl, tetrahydro-quinolinyl, thiamorpholinyl, thiamorpholinyl sulfoxide, thiazolyl, thiazolinyl, thienofuryl, thienothienyl, thienyl, and the like.

The term "substituted" is meant to include the recited chemical group (e.g., lower alkyl, heterocycle, aryl, cycloalkyl, etc.) substituted with one to four of the recited substituents (e.g., halo, hydroxy, lower alkyl, etc.). The substituent may be attached to any atom in the chemical group.

The structure of 4-(2-hydroxyethyl)]-1-piperazinylacetyl and 4-(2-hydroxyethyl)]-1-piperizineethanesulfonyl are, respectively, as follows:

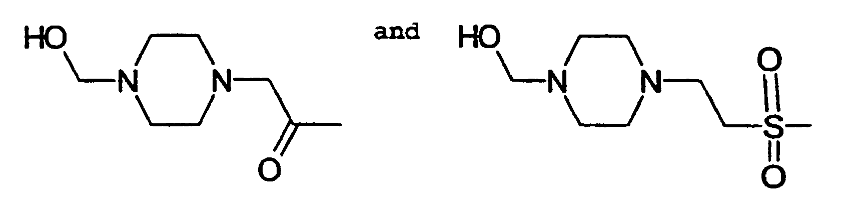

The compounds of this invention can be provided in the form of pharmaceutically acceptable salts. Acceptable salts include, but are not limited to, acid addition salts of inorganic acids such as hydrochloride, sulfate, phosphate, diphosphate, hydrobromide, and nitrate or organic acids such as acetate, maleate, fumarate, tartrate, succinate, citrate, lactate, methanesulfonate, p-toluenesulfonate, pamoate, salicylate, oxalate, and stearate. Also within the scope of the present invention, where applicable, are salts formed from bases such as sodium or potassium hydroxide. For further examples of pharmaceutically acceptable salts see,
"Pharmaceutical Salts," J. Pharm. Sci. 66:1 (1977).

Where the amino acid residue is optically active, it is the L-isomer that is intended unless otherwise specified. In the formulae set forth herein, the disulfide bond between the thiol group on the side chain of residue A₂ (e.g., Cys, Pen, D-Cys, or D-Pen) and the thiol group on the side chain of residue A₇ (e.g., Cys or Pen) is not shown.

The peptides of the invention can be used to promote the release of growth hormone or insulin in a subject (e.g., a mammal such as a human patient). Thus, the peptides are useful in the treatment of physiological conditions in which the promotion of the release of growth hormone or insulin is of benefit. The peptides of the invention can also be used in enhancing wound healing or promoting angiogenesis. Also, peptides of the invention having a Tyr(1) residue can be used to image cells containing somatostatin receptors. Such peptides of the invention can be used either *in vivo* to detect cells having somatostatin receptors (e.g., cancer cells) or in vitro as a radioligand in a somatostatin receptor binding assay. The peptide of the invention can also be used as vectors to target cells with radioactive isotopes.

A therapeutically effective amount of a peptide of this invention and a pharmaceutically acceptable carrier substance (e.g., magnesium carbonate, lactose, or a phospholipid with which the therapeutic compound can form a micelle) together form a therapeutic composition (e.g., a pill, tablet, capsule, or liquid) for administration (e.g., orally, intravenously, transdermally, pulmonarily, vaginally, subcutaneously, nasally, iontophoretically, or by intratracheally) to a subject in need of the peptide. The pill, tablet, or capsule can be coated with a substance capable of protecting the composition from the gastric acid or intestinal enzymes in the subject's stomach for a period of time sufficient to allow the composition to pass undigested into the subject's small intestine. The therapeutic composition can'also be in the form of a biodegradable or nonbiodegradable sustained release formulation for subcutaneous or intramuscular administration. See, e.g., U.S. Patents 3,773,919 and 4,767,628 and PCT Application No. WO 94/00148. Continuous administration can also be obtained using an implantable or external pump (e.g., INFUSAID TM pump) to administer the therapeutic composition.

The dose of a peptide of the present invention for treating the above-mentioned diseases or disorders varies depending upon the manner of administration, the age and the body weight of the subject, and the condition of the subject to be treated, and ultimately will be decided by the attending physician or veterinarian. Such an amount of the peptide as determined by the attending physician or veterinarian is referred to herein as a "therapeutically effective amount".

Also contemplated within the scope of this invention is a peptide covered by the above generic formula for both use in treating diseases or disorders associated with the need to promote the release of growth hormone or insulin, and use in detecting somatostatin receptors, e.g., radioimaging.

Other features and advantages of the present invention will be apparent from the detailed description and from the claims.

### Detailed Description of the Invention

It is believed that one skilled in the art can, based on the description herein, utilize the present invention to its fullest extent. The following specific embodiments are, therefore, to be construed as merely illustrative, and not limitative of the remainder of the disclosure in any way whatsoever.

Unless defined otherwise, all technical and scientific terms used herein have the same meaning as commonly understood by one of ordinary skill in the art to which this invention belongs. Also, all publications, patent applications, patents, and other references mentioned herein are incorporated by reference.

### Synthesis

The synthesis of short peptides is well examined in the peptide art. See e.g., Stewart, et al., Solid Phase Peptide Synthesis (Pierce Chemical Co., 2d ed., 1984). The following describes the synthesis of D-β-Nal-Cys-Pal-D-Trp-Lys-Val-Cys-β-Nal-NH₂ and D-β-Nal-Cpa-Tyr-D-Trp-Lys-Val-Phe-Thr-NH₂. Other peptides of the invention can be prepared in an analogous manner by a person of ordinary skill in the art.

### (a) Synthesis of H₂-β-Nal-D-Cys-Pal-D-Trp-Lys-Val-Cys-β-Nal-NH₂.

### 1) Boc-β-naphthylalanine-S-methylbenzyl-D-cysteine-3-pyridyl-2-alanine-D-tryptophan-N^{e-}benzyloxycarbonyl-lysine-valine-S-methylbenzyl-cysteine-β-naphthylalanine-benzhydrylamine resin.

Benzhhydrylamine-polystyrene resin (Advanced ChemTech, Inc., Louisville, KY) (1.2 g; 0.5 mmole) in the chloride ion form was placed in the reaction vessel of an Advanced ChemTech™ peptide synthesizer programmed to perform the following reaction cycle: (a) methylene chloride; (b) 33% trifluoroacetic acid in methylene chloride (2 times for 1 and 25 min each); (c) methylene chloride; (d) ethanol; (e) methylene chloride; and (f) 10% triethylamine in chloroform.

The neutralized resin was stirred with Boc-0-β-naphthylalanine and diisopropylcarbodiimide (1.5 mmole each) in methylene chloride for 1 hr, and the resulting amino acid resin was then cycled through steps (a) to (f) in the above wash program. The following amino acids (1.5 mmole) were then coupled successively by the same procedure: Boc-S-methylbenzyl-Cys, Boc-Val, Boc-N^{e-}benzyloxycarbonyl-lysine, Boc-D-Trp, Boc-Pal, and Boc-S-methylbenzyl-D-Cys and Boc-β-Nal. After washing and drying, the completed resin weighed 2.0 g.

### 2) β-naphthylalanine-c[D-cysteine-3-pyridyl-2-alanine-D-tryptophan:lysine-valine-cysteine]-/3-naphthylalanine-NH₂

The completed resin described in (1) (1.0 g, 0.25 mmole) was mixed with anisole (5 ml), dithiothreitol (100 mg), and anhydrous hydrogen fluoride (35 ml) at 0°C and stirred for 45 min. Excess hydrogen fluoride was evaporated rapidly under a stream of dry nitrogen, and the free peptide is precipitated and washed with ether. The crude peptide was then dissolved in 500 ml of 90% acetic acid to which was added a concentrated solution of I₂/MeOH until a permanent brown color is observed. Excess I₂ is removed by addition of ascorbic acid and the solution evaporated to a small volume which was applied to a column (2.5 x 90 cm) of Sephadex™ G-25, which was eluted with 50% AcOH. Fractions containing a major component by ultraviolet (UV) absorption and thin layer chromatography were then pooled, evaporated to a small volume, and applied to a column (1.5 x 70 cm) of Vydac™ octadecylsilane silica (10 - 15 *µ*m). This was eluted with a linear gradient of acetonitrile in 0.1% trifluoroacetic acid in water. Fractions were examined by thin layer chromatography (TLC) and analytical high performance liquid chromatography (HPLC) and pooled to give maximum purity. Repeated lyophilization of the solution from water gave the desired product as a white, fluffy powder. The product was found to be homogeneous by HPLC and TLC. Amino acid analysis of an acid hydrolysate and matrix-assisted laser desorption (MALD) mass spectroscopy confirmed the composition of the octapeptide.

### (b) Synthesis of H₂-D-β-Nal-Cpa-Tyr-D-Trp-Lys-Val-Phe-Thr-NH₂

### 1) Boc-β-D-naphthylalanine-D-4-chlorophenylalanine-0-dichlorobenzyl-tyrosine-D-tryptophan-N^{e-}benzyloxycarbonyl-lysine-valine-S-phenylalanine-O-benzyl-threonine-benzhydrylamine resin

Benzhydrylamine-polystyrene resin (Advanced ChemTech™, Inc.) (1.2 g, 0.5 mmole) in the chloride ion form was placed in the reaction vessel of an Advanced ChemTech peptide synthesizer programmed to perform the following reaction cycle: (a) methylene chloride; (b) 33% trifluoroacetic acid in methylene chloride (2 times for 1 and 25 min each); (c) methylene chloride; (d) ethanol; (e) methylene chloride; and (f) 10% triethylamine in chloroform.

The neutralized resin was stirred with Boc-0-benzylthreonine and diisopropylcarbodiimide (1.5 mmole each) in methylene chloride for 1 hr and the resulting amino acid resin was cycled through steps (a) to (f) in the above wash program. The following amino acids (1.5 mmole) were then coupled successively by the same procedure: Boc-phenylalanine, Boc-Val, Boc-N^{e}-benzyloxycarbonyl-lysine, Boc-D-Trp, Boc-0-dichlorobenzyl-Tyr, and Boc-D-4-chlorophenylalanine, and Boc-β-D-Nal. After washing and drying, the completed resin weighed 2.1 g.

### 2) β-D-naphthylalanine-D-4-chlorophenylalanine-tyrosine-D-tryptophan-lysine-valine-phenylalaninethreonine-NH₂

The peptide resin from (1) was subjected to HF cleavage as described above. Column purification as described yielded the desired compound as a white, fluffy powder (170 mg) which is found to be homogeneous by HPLC and TLC. Amino acid analysis of an acid hydrolysate and MALD mass spectroscopy confirms the composition of the peptide.

Peptides containing C-terminal substituted amides can be made by solid phase methods by displacing the appropriate peptide off the solid phase with the corresponding amine. Alternatively, these analogs may be synthesized by solutionphase peptide synthesis methods in which the growing peptide chain is maintained in solution in an organic solvent during synthesis and assembled by iterative coupling/deprotection cycles. Final removal of the side chain protecting groups yields the desired peptide after appropriate purification. Peptides containing N-terminal substitutions (e.g., where R₁ is E, CO, or E₁S0₂ (where E₁ is heterocycle lower alkyl) substituted with hydroxy lower alkyl and R₂ is H such as 4-(2- hydroxyethyl)-1-piperazinylacetyl or 4-(2-hydroxyethyl)-1-piperdineethanesulfonyl) can be synthesized as described in PCT Application No. WO 95/04752.

### Bioassay on the In Vitro Release of Growth Hormone

### (a) Rat Pituitary Cell Dispersion

Pituitaries from adult Charles River CD male rats (Wilmington, MA) housed under controlled conditions were dispersed and cultured using aseptic technique by modification of previously described methods (Hoefer, M.T., et al., Mol. Cell. Endocrinol. 35:229 (1984); Ben-Jonathan, N., et al., Methods Enzymol. 103:249 (1983); and Heiman, M.L., et al., Endocrinology 116:410 (1985)). Pituitaries were removed from sacrificed rats, sectioned, and then placed into a siliconized, liquid scintillation vial containing 2 ml 0.2% trypsin (Worthington Biochemicals, Freehold, NJ) in sterilefiltered Krebs-Ringer bicarbonate buffer supplemented with 1% bovine serum albumin, 14 mM glucose, modified Eagle medium (MEM) vitamin solution, and MEM amino acids (Gibco Laboratories, Grand Island, NY) (KRBGA). All glassware was siliconized as described by Sayers, et al., Endocrinology 88:1063 (1971). The fragments were incubated in a water bath for 35 min at 37°C with agitation. The vial contents then were poured into a scintillation vial containing 2 ml 0.1% DNase (Sigma Chemical Co., St. Louis, MO) in KRBGA and incubated for 2 min at 37°C with agitation. After incubation, the tissue was decanted into a 15 ml centrifuge tube and allowed to settle. Medium was discarded, and pituitary sections were washed 3 times with 1 ml fresh KRBGA. The cells were then dispersed in 2 ml 0.05% LBI (lima bean trypsin inhibitor, Worthington Biochemicals) by gently drawing the fragments into and expelling them out of a siliconized, fire polished Pasteur pipette. Dispersed cells were then filtered through a 630 µm diameter Nylon mesh (Tetko, Elmsford, NY) into a fresh 15 ml centrifuge tube. An additional 2 ml of 0.05% LBI solution was used to rinse the first tube and was transferred to the second tube with filtering.

### (b) Cell culture

The dispersed cells were then further diluted with approximately 15 ml sterile-filtered Dulbeccol's modified Eagle medium (GIBCO), which was supplemented with 2.5% fetal calf serum (GIBCO), 3% horse serum (GIBCO), 10% fresh rat serum (stored on ice for no longer than 1 hr) from the pituitary donors, 1% MEM non-essential amino acids (GIBCO), and gentamycin (10 ng/ml; Sigma) and nystatin (10,000 U/ml; GIBCO). The cells were poured into a 50 ml round-bottomed glass extraction flask with a large diameter opening and then randomly plated at a density of approximately 200,000 cells per well (Co-star cluster 24; Rochester Scientific Co., Rochester, NY). The plated cells were maintained in the above Dulbeccols medium in a humidified atmosphere of 95% air and 5% CO₂ at 37°C for 4-5 days.

### (c) Experimental incubation and IC₅₀ determination

In preparation for a hormone challenge, the cells were washed 3 times with medium 199 (GIBCO) to remove old medium and floating cells. Each treatment well contained a total volume of 1 ml medium 199 containing 1% BSA (fraction V; Sigma) with treatments as described below. Each antagonist candidate was tested using a single 24-well cell culture plate. Each treatment was performed in triplicate. Each plate contained 8 treatment groups: one 1 nM growth hormone releasing factor (GRF) (1-29)NH₂-stimulated control group; one 1 nM somatostatin-inhibited control group in the presence of 1 nM GRF (1 -29) NH₂; and 6 doses of a given antagonist in the presence of both 1 nM SRIF and 1 nM GRF per plate. After 3 hrs at 37°C in a air/carbon dioxide atmosphere (95/5%), the medium was removed and stored at -20°C until radioimmunoassayed for growth hormone content. IC₅₀' s of each antagonist versus 1 nm @ SRIF were calculated using a computer program (*SigmaPlot*, Jandel Scientific, San Rafael, CA) with the maximum response constrained to the value of the 1 nm GRF(1-29)NH₂-stimulated control. These IC₅₀'s are presented in Table I.

**TABLE I**

| ANALOG NO. | IC₅₀ (µM) | ANALOG NO. | IC₅₀ (µM) |
|---|---|---|---|
| 1 | 3.03 | 30 | 0.0065 |
| 2 | 0.04 | 31 | 0.0038 |
| 3 | 0.01 | 32 | 0.012 |
| 4 | 0.03 | 33 | 1.50 |
| 5 | 0.06 | 34 | 0.42 |
| 6 | 0.9 | 35 | 0.052 |
| 7 | 0.071 | 36 | 1.03 |
| 8 | 3.96 | 37 | 0.78 |
| 9 | 1.36 | 38 | 0.11 |
| 10 | 0.62 | 39 | 0.034 |
| 11 | 0.72 | 40 | 0.11 |
| 12 | 0.056 | 41 | 0.21 |
| 13 | 0.11 | 42 | 0.044 |
| 14 | 0.11 | 43 | 0.00082 |
| 15 | 0.14 | 44 | 0.021 |
| 16 | 0.82 | 45 | 0.13 |
| 17 | 1 | 46 | 0.02 |
| 18 | 0.38 | 47 | 0.053 |
| 19 | 0.11 | 48 | 0.050 |
| 20 | 0.12 | 49 | 0.23 |
| 21 | 0.97 | 50 | 0.0011 |
| 22 | 0.066 | 51 | 0.012 |
| 23 | 0.91 | 52 | 0.0026 |
| 24 | 0.068 | 53 | 0.0029 |
| 25 | 0.28 | 54 | 0.029 |
| 26 | 0.38 | 55 | 0.0026 |
| 27 | 0.041 | 56 | 0.0018 |
| 28 | 0.10 | 57 | 0.0059 |
| 29 | 0.0084 | | |

### Other

It is to be understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A compound of the formula: 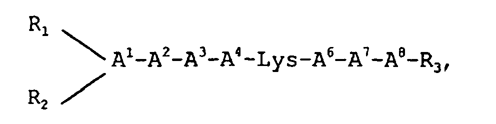 wherein
A¹ is a D- or L-isomer of an aromatic amino acid, or is deleted;
A² is a D-isomer selected from the group consisting of of Cys, Pen, an aromatic amino acid, or an aliphatic amino acid;
A³ is an aromatic amino acid;
A⁴ is Trp or D-Trp;
A⁶ is Thr, Thr (Bz1), Gly, Ser, an Eaa, or an aliphatic amino acid;
A⁷ is Cys, Pen, or an aromatic or an aliphatic amino acid;
A⁸ is a D- or L-isomer selected from the group consisting of Thr, Ser, an aromatic amino acid, or an aliphatic amino acid;
each of R₁ and R₂, is, independently, H or substituted or unsubstituted lower alkyl, aryl, aryl lower alkyl, heterocycle, heterocycle lower alkyl, E₁SO₂ or E₁CO (where E₁, is aryl, aryl lower alkyl, heterocycle, or heterocycle lower alkyl), where said substituent is halo, lower alkyl, hydroxy, halo lower alkyl, or hydroxy lower alkyl; and
R₃ is OH, NH₂, C₁₋₁₂ alkoxy, or NH-Y-CH₂-Z, wherein Y is a C₁₋₁₂ hydrocarbon moiety and Z is H, OH, CO₂H, or CONH₂, or R₃, together with the carbonyl group of A⁸ attached thereto, are reduced to form H, lower alkyl, or hydroxy lower alkyl; provided if A² is D-Cys or D-Pen, and A⁷ is Cys or Pen, then a disulfide bond links the sidechains of A² and A⁷, and if A¹ is D-Phe or p-NO₂-Phe; A² is D-Cys; A³ is Phe or Tyr; A⁶ is Thr or Val; and A⁷ is Cys; then A⁶ is β-Nal.

2. A compound of claim 1, wherein A² is D-Cys, A⁷ is Cys, and A⁴ is D-Trp.

3. A compound of claim 2, wherein A¹ is an L-aromatic amino acid.

4. A compound of claim 3, wherein A¹ and A³, independently, is β-Nal, o-X-Phe (where X is H, OH, CH₃, halo, OCH₃, NH₂, CN, or NO₂), p-X-phe (where X is H, OH, CH₃, halo, OCH₃, NH₂, CN, or NO₂), m-X-Phe (where X is H, OH, CH₃, halo, OCH₃, NH₂, CN, or NO₂), F₅-phe, Trp, Dip, 2-pal, Tyr(Bzl), His, Igl, Tyr(I), Bta, Bip, Npa, or Pal; A⁶ is Thr, Ser, Tle, Thr(Bzl), Abu, Ala, Ile, Leu, Gly, Nle, β-Ala, Gaba, or Val; and A⁹ is the D- or L-isomer of Thr, Dip, F₅-Phe, p-X-Phe (where X is H, OH, CH₃, halo, OCH₃, NH_{2'} CN, or NO₂), o-X-Phe (where X is H, OH, CH₃, halo, OCH₃, NH₂, CN, or NO₂), m-X-Phe (where X is H, OH, CH₃, halo, OCH₃, NH₂, CN, or NO₂), Igl, Tyr(Bzl), or β-Nal.

5. A compound of claim 4, wherein A¹ is β-Nal, Npa, Igl, Phe, p-F-Phe, Trp, p-Cl-Phe, or p-CN-Phe; A³ is Tyr, Tyr (I), or Pal; A⁶ is Val, Tle, Nle, Ile, or Leu; A⁸ is p-F-Phe, β-Nal, Tyr, Dip, p-Cl-Phe, Igl, or p-CN-Phe; R₁ is H, CH₃CO, 4- (2-hydroxyethyl) -1-piperazinylacetyl, or 4-(2hydroxyethyl)-1-piperizineethanesulfonyl; R₂ is H; and R₃ is NH₂.

6. A compound of claim 4 of the formula:
H₂-β-Nal-D-Cys-Tyr-D-Trp-Lys-Val-Cys-β-Nal-NH₂;
(H) (CH₃CO)-β-Nal-D-Cys-Tyr-D-Trp-Lys-Val-Cys-β-Nal-NH₂ (V);
(H)-(4-(2-hydroxyethyl)-1-piperazinylacetyl)-β-Nal-D-Cys-Tyr-D-Trp-Lys-val-Cys-β-Nal-NH₂;
(H)-(4-(2-hydroxyethyl)-1-piperizineethanesulfonyl)-βNal-D-Cys-Tyr-D-Trp-Lys-Val-Cys-β-Nal-NH₂;
H₂-β-Nal-D-cys-Pal- D-Trp-Lys-Val-Cys-β-Nal-NH₂;
(H) (CH₃CO)-β-Nal-D-Cys-Pal-D-Trp-Lys-Val-Cys-β-Nal-NH₂;
(H)-(4-(2-hydroxyethyl)-1-piperazinylacetyl)-β-Nal-D-Cys-Pal-D-Trp-Lys-Val-Cys-β-Nal-NH₂;
(H)-(4-(2-hydroxyethyl)-1-piperizineethanesulfonyl)-βNal-D-Cys-Pal-D-Trp-Lys-Val-Cys-β-Nal-NH₂;
H₂-β-Nal-D-Cys-Tyr-D-Trp-Lys-Val-Cys-Thr-NH₂;
(H) (CH₃CO) -β-Nal-D-Cys-Tyr-D-Trp-Lys-Val-Cys-Thr-NH₂;
(H) (4-(2-hydroxyethyl)-1-piperazinylacetyl)-β-Nal-D-Cys-Tyr-D-Trp-Lys-Val-Cys-Thr-NH₂;
(H) (4-(2-hydroxyethyl)-1-piperizineethanesulfonyl)-β-Nal-D-Cys-Tyr-D-Trp-Lys-Val-Cys-Thr-NH₂;
H₂-β-Nal-D-cys-Pal-D-Trp-Lys-Val-Cys-Thr-NH₂;
(H) (CH₃CO) -β-Nal-D-Cys-Pal-D-Trp-Lys-Val-Cys-Thr-NH₂;
(H) (4-(2-hydroxyethyl)-1-piperazinylacetyl)-β-Nal-D-Cys-Pal-D-Trp-Lys-Val-Cys -Thr-NH₂ ;
(H) (4-(2-hydroxyethyl)-1-piperizineethanesulfonyl)-β-Nal-D-CYs-Pal-D-Trp-Lys-Val-Cys-Thr-NH₂;
H₂- Phe-D-Cys-Tyr-D-Trp-Lys-val-Cys-β-Nal-NH₂;
(H) (CH₃CO) Phe-D-Cys-Tyr-D-Trp-Lys-Val-Cys-β-Nal-NH₂;
(H) (4-(2-hyrdroxyethyl)-1-piperazinylacetyl)-Phe-D-Cys-Tyr-D-Trp-Lys-Val-Cys-β-Nal-NH₂;
(H) (4-(2-hydroxyethyl)-1-piperizineethanesulfonyl)-Phe-D-Cys-Tyr-D-Trp-Lys-Val-Cys-β-Nal-NH₂;
H₂-Phe-D-Cys-Pal-D-Trp-Lys-Val-Cys-β-Nal-NH₂;
(H) (CH₃CO)Phe-D-Cys-Pal-D-Trp-Lys-Val-Cys-β-Nal-NH₂;
(H)(4-(2-hydroxyethyl)-1-piperazinylacetyl)-Phe-D-Cys-Pal-D-Trp-Lys-Val-Cys-β-Nal-NH₂;
(H)(4-(2-hydroxyethyl)-1-piperizineethanesulfonyl)-Phe-D-Cys-Pal-D-Trp-Lys-Val-Cys-β-Nal-NH₂;
H₂-Phe-D-Cys-Pal-D-Trp-Lys-Val-Cys-Thr-NH₂;
(H) (CH₃CO)-Phe-D-Cys-Pal-D-Trp-Lys-Val-Cys-Thr-NH₂;
(H) (4-(2-hydroxyethyl)-1-piperazinylacetyl)-Phe-D-Cys-Pal-D-Trp-Lys-Val-Cys-Thr-NH₂;
(H)(4-(2-hydroxyethyl)-1-piperizineethanesulfonyl)-Phe-D-Cys-Pal-D-Trp-Lys-Val-Cys-Thr-NH₂;
H₂-β-Nal-D-Cys-Tyr-D-Trp-Lys-Thr-Cys-β-Nal-NH₂;
(H) (CH₃CO)-β-Nal-D-Cys-Pal-D-Trp-Lys-Thr-Cys-β-Nal-NH₂;
(H)(4-(2-hydroxyethyl)-1-piperazinylacetyl)-β-Nal-D-Cys-Tyr-D-Trp-Lys-Thr-Cys-β-Nal-NH₂;
(H)(4-(2-hydroxyethyl)-1-piperizineethanesulfonyl)-β-Nal-D-Cys-Tyr-D-Trp-Lys-Thr-Cys-p-Nal-NH₂;
H₂-β-Nal-D-Cys-Pal-D-Trp-Lys-Thr-Cys-β-Nal-NH₂;
(H) (CH3CO)-β-Nal-D-Cys-Pal-D-Trp-Lys-Thr-Cys-β-Nal-NH₂; (H) (4-(2-hydroxyethyl)-1-piperazinylacetyl)-β-Nal-D-Cys-Pal-D-Trp-Lys-Thr-Cys-β-Nal-NH₂;
(H) (4-(2-hydroxyethyl)-1-piperizineethanesulfonyl)-β-Nal-D-Cys-Pal-D-Trp-Lys-Thr-Cys-β-Nal-NH₂;
H₂-β-Nal-D-Cys-Tyr-D-Trp-Lys-Thr-Cys-Thr-NH₂;
H (CH₃CO)-β-Nal-D-Cys -Tyr-D-Trp-Lys-Thr-Cys-Thr-NH₂;
(H) (4-(2-hydroxyethyl)-1-piperazinylacetyl)-β-Nal-D-Cys-Tyr-D-Trp-Lys-Thr-Cys-Thr-NH₂;
(H)(4-(2-hydroxyethyl)-1-piperizineethanesulfonyl)-β-Nal-D-Cys-Tyr-D-Trp-Lys-Thr-Cys-Thr-NH₂;
H₂-β-Nal-D-Cys-Pal-D-Trp-Lys-Thr-Cys-Thr-NH₂;
(H) (CH₃CO)-β-Nal-D-Cys-Pal-D-Trp-Lys-Thr-Cys-Thr-NH₂;
(H) (4-(2-hydroxyethyl)-1-piperazinylacetyl)-β-Nal-D-Cys-Pal-D-Trp-Lys-Thr-Cys-Thr-NH₂;
(H) (4-(2-hydroxyethyl)-1-piperizineethanesulfonyl)-β-Nal-D-Cys-Pal-D-Trp-Lys-Thr-Cys-Thr-NH₂;
H₂-Phe-D-Cys-Tyr-D-Trp-Lys-Thr-Cys-β-Nal-NH₂;
(H) (CH₃CO) Phe-D-Cys -Tyr- D-Trp-Lys-Thr-Cys-β-Nal-NH₂;
(H) (4-(2-hydroxyethyl)-1-piperazinylacetyl)Phe-D-Cys-Tyr-D-Trp-Lys-Thr-Cys-β-Nal-NH₂;
(H) (4-(2-hydroxyethyl)-1-piperizineethanesulfonyl) Phe-D-Cys-Tyr-D-Trp-Lys-Thr-Cys-β-Nal-NH₂;
H₂-Phe-D-Cys-pal-D-Trp-Lys-Thr-Cys-β-Nal-NH₂;
(H) (CH₃CO)Phe-D-Cys-Pal-D-Trp-Lys-Thr-Cys-β-Nal- NH₂;
(H) (4-(2-hydroxyethyl)-1-piperazinylacetyl)Phe-D-Cys-Pal-D-Trp-Lys-Thr-Cys-β-Nal-NH₂;
(H) (4-(2-hydroxyethyl)-1-piperizineethanesulfonyl) Phe-D-Cys-Pal-D-Trp-Lys-Thr-Cys-p-Nal-NH₂;
H₂-Phe-D-Cys-Tyr-D-Trp-Lys-Thr-Cys-Thr-NH₂;
(H) (CH₃CO)Phe-D-Cys-Tyr-D-Trp-Lys-Thr-Cys-Thr-NH₂;
(H) (4-(2-hydroxyethyl)-1-piperazinylacetyl)Phe-D-Cys-Tyr-D-Trp-Lys-Thr-Cys-Thr-NH₂;
(H) (4-(2-hydroxyethyl)-1-piperizineethanesulfonyl) Phe-D-Cys-Tyr-D-Trp-Lys-Thr-Cys-Thr-NH₂;
H₂-Phe-D-Cys-Pal-D-Trp-Lys-Thr-Cys-Thr-NH₂;
(H) (CH₃CO)-Phe-D-Cys-Pal-D-Trp-Lys-Thr-Cys-Thr-NH₂;
(H) (4-(2-hydroxyethyl)-1-piperazinylacetyl)Phe-D-Cys-Pal-D-Trp-Lys-Thr-Cys-Thr-NH₂;
(H) (4-(2-hydroxyethyl)-1-piperizineethanesulfonyl) Phe-D-Cys-Pal-D-Trp-Lys-Thr-Cys-Thr-NH₂;
H₂-β-Nal-D-Cys-Tyr-D-Trp-Lys-Abu-Cys-β-Nal-NH₂;
H₂-Phe-D-Cys-Tyr-D-Trp-Lys-Abu-Cys-β-Nal-NH₂;
H₂-β-Nal-D-cys-Pal-D-Trp-Lys-Abu-Cys-β-Nal-NH₂;
H₂-Phe-D-Cys-Pal-D-Trp-Lys-Abu-Cys-O-Nal-NH₂;
H₂-β-Nal-D-Cys-Tyr-D-Trp-Lys-Abu-Cys-Thr-NH₂;
H₂-Phe-D-Pen-Tyr-D-Trp-Lys-Val-Pen-β-Nal-NH₂; or
H₂,-Phe-D-Pen-Pal-D-Trp-Lys-Thr-Pen-Thr-NH₂;
H₂-Dip-D-Cys-Pal-D-Trp-Lys-Val-Cys-Dip-NH₂;
H₂-F₅-Phe-D-Cys-His-D-Trp-Lys-Val-Cys-F₅-Phe-NH₂;
H₂-DiP-D-Cys-Pal-D-Trp-Lys-Val-Cys-β-Nal-NH₂;
H₂-m-F-Phe-D-Cys-Pal-D-Trp-Lys-Val-Cys-m-F-Phe-NH₂;
H₂-o-F-Phe-D-Cys-Pal-D-Trp-Lys-Val-Cys-o-F-Phe-NH₂;
H₂-p-F-Phe-D-Cys-Pal-D-Trp-Lys-Val-Cys-p-F-Phe-NH₂;
H₂-F₅-Phe-D-Cys-Pal-D-Trp-Lys-Val-Cys-F₅-Phe-NH₂;
H₂-F₅-Phe-D-Cys-2 -Pal -D-Trp-Lys-Val-Cys-F₅-Phe-NH₂;
H₂-β-Nal-D-Cys-His-D-Trp-Lys-Val-Cys-D-Dip-NH₂;
H₂-Dip-D-Cys-Hi s-D-Trp-Lys-Val-Cys-β-Nal-NH₂;
H₂-Dip-D-Cys-His-D-Trp-Lys-Val-Cys-Dip-NH₂;
H₂-P-Nal-D-Cys-His-D-Trp-Lys-Val-Cys-β-Nal-NH₂;
H₂-Trp-D-Cys-Tyr-D-Trp-Lys-Val-Cys-D-β-Nal-NH₂;
H₂-β-Nal-D-Cys-Tyr-D-Trp-Lys-Val-Cys-D-β-Nal-NH₂;
H₂-β-Nal-D-Cys-Pal-D-Trp-Lys-Val-Cys-D-p-F-Phe-NH₂;
H₂-β-Nal-D-Cys-Pal-D-Trp-Lys-Tle-Cys-β-Nal-NH₂;
H₂-p-F-Phe-D-Cys-Pal-D-Trp-Lys-Val-Cys-β-Nal-NH₂;
H₂-β-Nal-D-Cys-Pal-D-Trp-Lys-Nle-Cys-β-Nal-NH₂;
H₂-β-Nal-D-Cys-Pal-D-Trp-Lys-Ile-Cys-β-Nal-NH₂;
H₂-β-Nal-D-Cys-Pal-D-Trp-Lys-Gly-Cys-β-Nal-NH₂;
H₂-β-Nal-D-Cys-Pal-D-Trp-Lys-Ala-Cys-β-Nal-NH₂;
H₂-β-Nal-D-Cys-Pal-D-Trp-Lys-Leu-Cys-β-Nal-NH₂;
H₂-Bip-D-Cys-Tyr-D-Trp-Lys-Ile-Cys-Bip-NH₂;
H₂-p-F-Phe-D-Cys-His-D-Trp Lys-Val-Cys-p-F-Phe-NH₂;
H₂-Npa-D-Cys-Pal-D-Trp-Lys-Val-Cys-Tyr-NH₂;
H₂-m-F-Phe-D-Cys -His-D-Trp-Lys-Val-Cys-m-F-Phe-NH₂;
H₂-o-F-Phe-D-Cys-His-D-Trp-Lys-Val-Cys-o-F-Phe-NH₂;
H₂-β-Nal-D-Cys-Pal-D-Trp-Lys-Val-Cys-Dip-NH₂;
H₂-Cpa-D-Cys-Pal-D-Trp-Lys-Val-Cys-Cpa-NH₂;
H₂-Igl-D-Cys-Pal-D-Trp-Lys-Val-Cys-Igl-NH₂;
H₂-β-Nal-D-Cys-Pal-D-Trp-Lys-Val-Cys-D-Dip-NH₂;
H₂-β-Nal-D-Cys-3-I-Tyr-D-Trp-Lys-Val-Cys-β-Nal-NH₂;
H₂-p-CN-Phe-D-Cys-Pal-D-Trp-Lys-Val-Cys-p-CN-Phe-NH₂;
H₂-β-Nal-D-Cys-Tyr-D-Trp-Lys-Val-Cys-D-Dip-NH₂;
H₂-β-Nal-D-Cys-Bta-D-Trp-Lys-Val-Cys-β-Nal-NH₂;
H₂-p-F-Phe-D-Cys-Pal-D-Trp-Lys-Tle-Cys-β-Nal-NH₂;
H₂-Bpa-D-Cys-Pal-D-Trp-Lys-Val-Cys-Bpa-NH₂;
H₂-Iph-D-Cys-Pal-D-Trp-Lys-Val-Cys-Iph-NH₂:
H₂-Trp-D-Cys-Pal-D-Trp-Lys-Tle-Cys-β-Nal-NH₂;
H₂-p-Cl-Phe-D-Cys-Pal-D-Trp-Lys-Val-Cys-β-Nal-NH₂
H₂-p-Cl-Phe-D-Cys-Pal-D-Trp-Lys-Tle-Cys-β-Nal-NH₂;
H₂-p-Cl-Phe-D-Cys-Pal-D-Trp-Lys-Tle-Cys-p-Cl-Phe-NH₂;
H₂-p-Cl-Phe-D-Cys-Pal-D-Trp-Lys-Cha-Cys-p-Cl-Phe-NH₂;
H₂-p-Cl-Phe-D-Cys-Tyr(I)-D-Trp-Lys-Val-Cys-p-Cl-Phe-NH₂; H₂-p-Cl-Phe-D-Cys-Tyr(I)-D-Trp-Lys-Val-Cys-β-Nal-NH₂;H₂-p-Cl-Phe-D-Cys-Tyr(I)-D-Trp-Lys-Tle-Cys-β-Nal-NH₂;
H₂-p-F-Phe-D-Cys-Tyr(I)-D-Trp-Lys-Val-Cys-β-Nal-NH₂;
H₂-p-F-Phe-D-Cys-Tyr(I)-D-Trp-Lys-Tle-Cys-β-Nal-NH₂;
H₂-β-Nal-D-Cys-Tyr-D-Trp-Lys-Abu-Cys-β-Nal-NH₂;
(H) (CH₃CO)-β-Nal-D-Cys-Tyr-D-Trp-Lys,Abu-Cys-β-Nal-NH₂; H₂-p-NO₂-Phe-D-Cys-Tyr-D-Yrp-Lys-Abu-cys-β-Nal-NH₂;
(H) (CH₃CO)-β-Nal-D-Cys-Tyr-D-Trp-Lys-Abu-Cys-β-Nal-NH₂; H₂-p-NO2-Phe-D-Cys-Tyr(Bzl)-D-Trp-Lys-Thr(Bzl)-Cys-Nal-NH₂ :
(H) (4-(2-hydroxyethyl)-1-piperazinylacetyl)-p-NO₂-phe-D-Cys-Tyr(Bzl)-D-Trp-Lys-Thr(Bzl)-Cys-β-Nal-NH₂;
(H) (4-(2-hydroxyethyl)-1-piperazinylacetyl)-p-NO₂-Phe-D-Cys-Tyr-D-Trp-Lys-Thr-Cys-Tyr-NH₂;
H₂-_{P}-NO₂-Phe-D-Cys-Tyr-D-Trp-Lys-Val-Cys-p-NaI-NH₂;
(H) (4-(2-hydroxyethyl)-1-piperazinylacetyl)-p-NO₂-Phe-D-Cys-Tyr-D-Trp-Lys-Val-Cys-p-Nal-NH₂;
(H) (4-(2-hydroxyethyl)-1-piperazinylacetyl)-β-Nal-Phe-D-Gys-Tyr-D-Trp-Lys-Val-Cys-β-Nal-NH₂;
H₂-β-Nal-D-Cys-Tyr(Bzl)-D-Trp-Lys-Thx(Bzl)-Cys-β-Nal-NH₂; or
(H) (4-(2-hydroxyethyl)-1-piperazinylacetyl)-β-Nal-D-Cys-Tyr(Bzl)-D-Trp-Lys-Thr(Bzl)-Cys-Tyr(Bzl)-NH₂; or a pharmaceutically acceptable salt thereof.

7. A compound of claim 2, wherein A¹ is a D-aromatic amino acid.

8. A compound of claim 7, wherein A¹ is D-β-Nal, Do-X-Phe (where X is H, OH, CH₃, halo, OCH₃, NH₂, CN, or NO₂), D-p-X-Phe (where X is H, OH CH₃, halo, OCH₃, NH₂, CN, or NO₂), Dm-X-Phe (where X is H, OH, CH₃, halo, OCH₃, NH₂, CN, or NO₂), D-F₅-Phe, D-Trp, D-Dip, D-2-Pal, D-Tyr(Bzl), D-His, D-Igl, DTyr (I), D-Bta, D-Bip, D-Npa, or D-Pal; A³ is β-Nal, o-X-Phe (where X is H, OH, CH₃, halo, OCH₃, NH₂, CN, or NO₂), p-X-Phe (where X is H, OH, CH₃, halo, OCH₃, NH₂, CN, or NO₂), m-X-Phe (where X is H, OH, CH₃, halo, OCH₃, NH₂, CN, or NO₂), F₅-Phe, Trp, Dip, 2-Pal, Tyr (Bzl), His, Igl, Tyr(I), Bta, Bip, Npa, or Pal; A⁶ is Thr, Ser, Tle, Thr(Bzl), Abu, Ala, Ile, Leu, Gly, Nle, β-Ala, Gaba, or Val; and A⁸ is the D- or L-isomer of Thr, Dip, F₅-Phe, p-x-Phe (where X is H, OH, CH₃, halo, OCH₃, NH₂, CN, or NO₂), oX-Phe (where X is H, OH, CH₃, halo, OCH₃, NH₂, CN, or NO₂), m-X-Phe (where X is H, OH, CH₃, halo, OCH₃, NH₂, CN, or NO₂), Igl, Tyr(Bzl), or β-Nal.

9. A compound of claim 8, wherein A¹ is D-β-Nal, D-Npa, D-Igl, D-Phe, D-p-F-Phe, D-Trp, D-p-Cl-Phe, or D-pCN-Phe; A³ is Tyr, Tyr (I), or Pal; A⁶ is Val, Tle, Nle, Ile, or Leu; A⁸ is p-F-Phe, β-Nal, Tyr, Dip, p-Cl-Phe, Igl, or p-CNPhe; R₁ is H, CH₃CO 4-(2-hydroxyethyl)-1-piperazinylacetyl, or 4-(2-hydroxyethyl)-1-piperizineethanesulfonyl; R₂ is H; and R₃ is NH₂.

10. A compound of claim 7, of the formula:
H₂-D-Phe-D-Pen-Tyr-D-Trp-Lys-Val-Cys-Thr-NH₂;
H₂-D-β-Nal-D-Cys-Tyr-D-Trp-Lys-Val-Cys-Thr-NH₂;
H₂-D-β-Nal-D-Cys-Tyr-D-Trp-Lys-Val-Cys-β-Nal-NH₂;
H₂-D-β-Nal-D-Cys-Tyr-D-Trp-Lys-Thr-Cys-p-Nal-NH₂;
H₂-D-Phe-D-Cys-Pal-D-Trp-Lys-Thr-Cys-Thr-NH₂;
H₂-D-Phe-D-Cys-Tyr-D-Trp-Lys-Abu-Cys-Thr-NH₂;
H₂-D-β-Nal-D-Cys-Tyr-D-Trp-Lys-Abu-Cys-Thr-NH₂;
H₂-D-β-Nal-D-Cys-Tyr-D-Trp-Lys-Val-Cys-D-β-Nal-NH₂;
H₂-D-p-F-Phe-D-Cys-Pal-D-Trp-Lys-Val-Cys-D-p-F-Phe-NH₂;
H₂-D-Bip-D-Cys-Tyr-D-Trp-Lys-Val-Cys-β-Nal-NH₂;
H₂-D-Dip-D-Cys-Pal-D-Trp-Lys-Val-Cys-β-Nal-NH₂;
H₂-D-p-F-Phe-D-Cys-Pal-D-Trp-Lys-Tle-Cys-β-Nal-NH₂;
H₂-D-p-Cl-Phe-D-Cys-Pal-D-Trp-Lys-Tle-Cys-p-Cl-Phe-NH₂;
p-NO₂-D-Phe-D-Cys-Pal-D-Trp-Lys-Thr(Bzl)-Cys-Tyr(Bzl)-NH₂;
p-NO₂-D-Phe-D-Cys-Tyr(Bzl)-D-Trp-Lys-Val-Cys-Tyr(Bzl)-NH₂;
(H) (4-(2-hydroxyethyl)-1-piperazinylacetyl)-p-NO₂-D-Phe-D-Cys-Pal-D-Trp-Lys-Thr(Bzl)-Cys-Tyr(Bzl)-NH₂; or
(H)(4-(2-hydroxyethyl)-1-piperazinylacetyl)-p-NO₂-D-Phe-D-Cys-Tyr(Bz1)-D-Trp-Lys-val-Cys-Tyr(Bzl)-NH₂; or a pharmaceutically acceptable salt thereof.

11. A compound of claim 2, wherein A¹ is deleted, R² is substituted or unsubstituted E₁CO, and R₂ is H.

12. A compound of claim 11, wherein R₁ is substituted or unsubstituted E₁CO (where E₁ is phenyl, β-naphthylmethyl, β-pyridinylmethyl, or 3-indolylmethyl); A³ is β-Nal, o-X-Phe (where X is H, OH, CH₃, halo, OCH₃, NH₂, CN, or NO₂), p-X-Phe (where X is H, OH, CH₃. halo, OCH₉ NH₂, CN, or NO₂), m-X-Phe (where X is H, OH, CH₃, halo, OCH₃, NH₂, CN, or NO₂), F₅-Phe, Trp, Dip, 2-Pal, Tyr(Bzl), His, Igl, Tyr (I), Bta, Bip, Npa, or Pal; A⁶ is Thr, Ser, Tle, Thr(Bzl), Abu, Ala, Ile, Leu, Gly, Nle, β-Ala, Gaba, or Val; and A⁸ is the D- or L-isomer of Thr, Dip, F₅-Phe, p-X-Phe (where X is H, OH, CH₃, halo, OCH₃, NH₂, CN, or NO₂), oX-Phe (where X is H, OH, CH₃, halo, OCH₃, NH₂, CN, or NO₂), m-X-Phe (where X is H, OH, CH₃, halo, OCH₃, NH_{2'} CN, or NO₂), Igl, Tyr(Bzl), or β-Nal.

13. A compound of claim 12, wherein R₁ is E₁CO (where E₁ is 4-hydroxy-phenyl, β-naphthylmethyl, or phenyl); A³ is Tyr, Tyr (I), or Pal; A⁶ is Val, Tle, Nle, Ile, or Leu; A₈ is p-F-Phe, β-Nal, Tyr, Dip, p-Cl-Phe, Igl, or p-CN-Phe; R₃ is NH₂; or
of the formula
(H) (3-phenylpropionyl)-D-Cys-Tyx-D-Trp-Lys-val-Cys-β-Nal-NH₂;
(H) (3-phenylpropionyl)-D-Cys-Pal-D-Trp-Lys-Val-Cys-β-Nal-NH₂;
(H) (3-phenylpropionyl)-D-Cys-Tyr-D-Trp-Lys-Thr-Cys-β-Nal-NH₂;
(H) (3-phenylpropionyl)-D-Cys-Pal-D-Trp-Lys-Thr-Cys-β-Nal-NH₂;
(H) (3-phenylpropionyl)-D-cys-Tyr-D-Trp-Lys-Val-cys-Thr-NH₂;
(H) (3-phenylpropionyl)-D-Cys-Pal-D-Trp-Lys-Val-Cys-Thr-NH₂;
(H) (3-phenylpropionyl)-D-Cys-Tyr-D-Trp-Lys-Thr-Cys-Thr-NH₂ ;
(H) (3-phenylpropionyl)-D-Cys-Pal-D-Trp-Lys-Thr-Cys-Thr-NH₂;
(H) (3-[2-naphthyl]propionyl)-D-Cys-Tyr-D-Trp-Lys-Val-Cys-β-Nal-NH₂;
(H) (3-[2-naphthyl]propionyl)-D-Cys-Pal-D-Trp-Lys-Val-Cys-β-Nal-NH₂;
(H) (3-[2-naphthyl]propionyl)-D-Cys-Tyr-D-Trp-Lys-Thr-Cys-β-Nal-NH₂;
(H) (3-[2-naphthyl]propionyl)-D-Cys-Pal-D-Trp-Lys-Thr-Cys-β-Nal-NH₂;
(H) (3-[2-naphthyl]propionyl)-D-Cys-Tyr-D-Trp-Lys-Val-Cys-Thr-NH₂ ;
(H) (3-[2-naphthyl]propionyl)-D-Cys-Pal-D-Trp-Lys-Val-Cys-Thr-NH₂;
(H) (3-[2-naphthyl]propionyl)-D-Cys-Tyr-D-Trp-Lys-Thr-Cys-Thr-NH₂;
(H) (3-[2-naphthyl]propionyl)-D-Cys-Pal-D-Trp-Lys-Thr-Cys-Thr-NH₂;
(H) (3-[p-hydroxyphenyl])-D-Cys-Tyr-D-Trp-Lys-Val-Cys-β-Nal-NH₂;
(H) (3-naphthyl]propionyl)-D-Cys-Tyr-D-Trp-Lys-Abu-Cys-β-Nal-NH₂;
(H) (3-naphthyl]propionyl)-D-Cys-Tyr-D-Trp-Lys-Abu-Cys-Thr-NH₂;
(H) (3-phenylylpropionyl)-D-Cys-Tyr-D-Trp-Lys-Abu-Cys-β-Nal-NH₂; or
(H) (3-phenylylpropionyl)-D-Cys-Tyr-D-Trp-Lys-Abu-Cys-Thr-NH₂; or
a pharmaceutically acceptable salt thereof.

14. A compound of claim 2, wherein R₃, together with the carbonyl group of A⁸ attached thereto, are reduced to form H, lower alkyl, or hydroxy lower alkyl.

15. A compound of claim 14, wherein A¹ is the D- or L-isomer of β-Nal, o-X-Phe (where X is H, OH, CH₃, halo, OCH₃, NH₂, CN, or NO₂), -p-X-Phe (where X is H, OH, CH₃, halo, OCH₃, NH₂, CN, or NO₂), m-X-Phe (where X is H, OH, CH₃, halo, OCH₃, NH_{2'} CN, or NO₂), F₅-Phe, Trp, Dip, 2-Pal, Tyr (Bzl), His, Igl, Tyr (I), Bta, Bip, Npa, or Pal; A³ is β-Nal, o-X-Phe (where X is H, OH, CH₃, halo, OCH₃, NH_{2'} CN, or NO₂), p-X-Phe (Where X is H, OH, CH₃, halo, OCH₃, NH₂, CN, or NO₂), m-X-Phe (where X is H, OH, CH₃, halo, OCH₃, NH₂, CN, or NO₂), F₅-Phe, Trp, Dip, 2-Pal, Tys (Bzl), His, Igl, Tyr (I), Bta, Bip, Npa, or Pal; A⁶ is Thr, Ser, Tle, Thr (Bzl), Abu, Ala, Ile, Leu, Gly, Nle, β-Ala, Gaba, or Val; and A⁸ is the D- or L-isomer of Thr, Dip, F₅-Phe, p-X-Phe (where X is H, OH, CH₃, halo, OCH₃, NH₂, CN, or NO₂), o-X-Phe (where X is H, OH, CH₃, halo, OCH₃, NH₂, CN, or NO₂), m-X-Phe (where X is H, OH, CH₃, halo, OCH₃, NH₂, CN, or NO₂), Igl, Tyr(Bzl), or β-Nal.

16. A compound of claim 15, wherein A¹ is the D- or L-isomer of β-Nal, Phe, p-F-Phe, Trp, p-Cl-Phe, or p-CN-Phe; A³ is Tyr, Tyr (I), or Pal; A⁶ is Val, Tle, Nle, Ile, or Leu; A⁸ is p-F-Phe, β-Nal, Tyr, Dip, p-Cl-Phe, Igl, or p-CN-Phe; R₁ is H, CH₃CO, 4-(2-hydroxyethyl)-1-piperaxinylacetyl, or 4-(2-hydroxyethyl)-1-piperizineethanesulfonyl: R₂ is H, and R₃, together with the carboxy group of A⁸ attached thereto, are reduced to form H or CH₃OH.

17. A compound of claim 9, 13 or 16, wherein A³ is Pal.

18. A compound of claim 15, of the formula:
H₂-β-Nal-D-Cys-Tyr-D-Trp-Lys-Val-Cys-2R,3R-(2-hydroxymethyl)-3-hydroxy)propylamide;
(H) (CH₃CO)-β-Nal-D-Cys-Tyr-D-Trp-Lys-Val-Cys-2R, 3R-(2-hydroxymethyl)-3-hydroxy)propylamide;
(H) (4-(2-hydroxyethyl)-1-piperazinylacetyl)-β-Nal-D-Cys-Tyr-D-Trp-Lys-Val-Cys-2R, 3R-(2-hydroxymethyl)-3-hydroxy)propylamide;
(H) (4-(2-hydroxyethyl)-1-piperizineethanesulfonyl)-β-Nal-D-Cys-Tyr-D-Trp-Lys-Val-Cys-2R,3R-(2-hydroxymethyl)-3-hydroxy)propylamide;
H₂,-β-Nal-D-Cys-Pal-D-Trp-Lys-Val-Cys-2R,3R-(2-hydroxymethyl)-3-hydroxy)propylamide;
(H) (CH₃CO) -β-Nal-D-Cys-Pal-D-Trp-Lys-Val-Cys-2R,3R-(2-hydroxymethyl)-3-hydroxy)propylamide;
(H) (4-(2-hydroxyethyl)-1-piperazinylacetyl)-β-Nal-D-Cys-Pal-D-Trp-Lys-Val-Cys-2R,3R-(2-hydroxymethyl)-3-hydroxy)propylamide;
(H) (4-(2-hydroxyethyl)-1-piperizineethanesulfonyl)-β-Nal-D-Cys-Pal-D-Trp-Lys-Val-Cys-2R,3R-(2-hydroxymethyl)-3-hydroxy)propylamide;
H₂-β-Nal-D-Cys-Tyr-D-Trp-Lys-Thr-Cys-2R,3R-(2-hydroxymethyl)-3-hydroxy)propylamide;
(H) (CH₃CO)-β-Nal-D-Cys-Tyr-D-Trp-Lys-Thr-Cys-2R,3R-(2-hydroxymethyl)-3-hydroxy)propylamide;
(H) (4-(2-hydroxyethyl)-1-piperazinylacetyl)-β-Nal-D-Cys-Tyr-D-Trp-Lys-Thr-Cys-2R,3R-(2-hydroxymethyl)-3-hydroxy)propylamide;
(H) (4-(2-hydroxyethyl)-1-piperizineethanesulfonyl)-β-Nal-D-Cys-Tyr-D-Trp-Lys-Thr-Cys-2R,3R-(2-hydroxymethyl)-3-hydroxy)propylamide;
H₂-β-Nal-D-Cys-Pal-D-Trp-Lys-Thr-Cys-2R,3R-(2-hydroxymethyl)-3-hydroxy)propylamide;
(H) (CH₃CO)-β-Nal-D-Cys-Pal-D-Trp-Lys-Thr-Cys-2R,3R-(2-hydroxymethyl)-3-hydroxy)propylamide;
(H) (4-(2-hydroxyethyl)-1-piperazinylacetyl)-β-Nal-D-Cys-Pal-D-Trp-Lys-Thr-Cys-2R,3R-(2-hydroxymethyl)-3-hydroxy)propylamide;
(H) (4-(2-hydroxyethyl)-1-piperizineethanesulfonyl)-β-Nal-D-Cys-Pal-D-Trp-Lys-Thr-Cys-2R,3R-(2-hydroxymethyl)-3-hydroxy)propylamide;
H₂-Phe-D-Cys-Tyr-D-Trp-Lys-Val-Cys-2R,3R-(2-hydroxymethyl)-3-hydroxy)propylamide;
(H) (CH₃CO) Phe-D-Cys-Tyr-D-Trp-Lys-Val-Cys-2R, 3R- (2-hydroxymethyl)-3-hydroxy)propylamide;
(H) (4-(2-hydroxyethyl)-1-piperazinylacetyl)Phe-D-Cys-Tyr-D-Trp-Lys-Val-Cys-2R,3R-(2-hydroxymethyl)-3-hydroxy)propylamide;
(H) (4-(2-hydroxyethyl)-1-piperizineethanesulfonyl) Phe-D-Cys-Tyr-D-Trp-Lys-Val-Cys-2R,3R-(2-hydroxymethyl)-3-hydroxy)propylamide;
H₂-Phe-D-Cys-Pal-D-Trp-Lys-Val-Cys-2R,3R-(2-hydroxymethyl)-3-hydroxy)propylamide;
H(CH₃CO)Phe-D-Cys-Pal-D-Trp-Lys-Val-Cys-2R,3R-(2-hydroxymethyl)-3-hydroxy)propylamide;
(H) (4-(2-hydroxyethyl)-1-piperazinylacetyl)Phe-D-Cys-Pal-D-Trp-Lys-Val-Cys-2R,3R-(2-hydroxymethyl)-3-hydroxy)propylamide;
(H) (4-(2-hydroxyethyl)-1-piperizineethanesulfonyl) Phe-D-Cys-Pal-D-Trp-Lys-Val-Cys-2R,3R-(2-hydroxymethyl)-3-hydroxy)propylamide;
H₂-Phe-D-Cys-Tyr-D-Trp-Lys-Thr-Cys-2R,3R-(2-hydroxymethyl)-3-hydroxy)propylamide;
(H) (CH₃CO) Phe-D-Cys-Tyr-D-Trp-Lys-Thr-Cys-2R, 3R- (2-hydroxymethyl)-3-hydroxy)propylamide;
(H) (4-(2-hydroxyethyl)-1-piperazinylacetyl)Phe-D-Cys-Tyr-D-Trp-Lys-Thr-Cys-2R,3R-(2-hydroxymethyl)-3-hydroxy)propylamide;
(H) (4-(2-hydroxyethyl)-1-piperizineethanesulfonyl) Phe-D-Cys-Tyr-D-Trp-Lys-Thr-Cys-2R,3R-(2-hydroxymethyl)-3-hydroxy)propylamide;
H₂-Phe-D-Cys-Pal-D-Trp-Lys-Thr-Cys-2R,3R-(2-hydroxymethyl)-3-hydroxy)propylamide;
(H) (CH₃CO) Phe -D-Cys -Pal- D-Trp-Lys-Thr-Cys-2R, 3R- (2-hydroxymethyl)-3-hydroxy)propylamide;
(H) (4-(2-hydroxyethyl)-1-piperazinylacetyl)Phe-D-Cys-Pal-D-Trp-Lys-Thr-Cys-2R,3R-(2-hydroxymethyl)-3-hydroxy)propylamide;
(H) (4-(2-hydroxyethyl)-1-piperizineethanesulfonyl) Phe-D-Cys-Pal-D-Trp-Lys-Thr-Cys-2R,3R-(2-hydroxyrnethyl)-3-hydroxy)propylamide;
H₂-β-Nal-D-Cys-Tyr-D-Trp-Lys-Val-Cys-2R-(2-naphthyl)ethylamide;
(H) (CH₃CO)-β-Nal-D-Cys-Tyr-D-Trp-Lys-Val-Cys-2R-(2-naphthyl)ethylamide;
(H) (4-(2-hydroxyethyl)-1-piperazinylacetyl)-β-Nal-D-Cys-Tyr-D-Trp-Lys-Val-Cys-2R-(2-naphthyl)ethylamide;
(H) (4-(2-hydroxyethyl)-1-piperizineethanesulfonyl)-β-Nal-D-Cys-Tyr-D-Trp-Lys-Val-Cys-2R-(2-naphthyl)ethylamide;
H₂-β-Nal-D-Cys-Pal-D-Trp-Lys-Val-Cys-2R-(2-naphthyl)ethylamide;
(H) (CH₃CO)-β-Nal-D-Cys-Pal-D-Trp-Lys-Val-Cys-2R-(2-naphthyl)ethylamide;
(H) (4-(2-hydroxyethyl)-1-piperazinylacetyl)-β-Nal-D-Cys-Pal-D-Trp-Lys-Val-Cys-2R-(2-naphthyl)ethylamide;
(H) (4-(2-hydroxyethyl)-1-piperizineethanesulfonyl)-β-Nal-D-Cys-Pal-D-Trp-Lys-Val-Cys-2R-(2-naphthyl)ethylamide;
H₂-β-Nal-D-Cys-Tyr-D-Trp-Lys-Thr-Cys-2R-(2-naphthyl) ethylamide;
(H) (CH₃CO)-β-Nal-D-Cys-Tyr-D-Trp-Lys-Thr-Cys-2R- (2-naphthyl)ethylamide;
(H) (4-(2-hydroxyethyl)-1-piperazinylacetyl)-β-Nal-D-Cys-Tyr-D-Trp-Lys-Thr-Cys-2R-(2-naphthyl)ethylamide:
(H) (4-(2-hydroxyethyl)-1-piperizineethanesulfonyl)-β-Nal-D-Cys-Tyr-D-Trp-Lys-Thr-Cys-2R-(2-naphthyl)ethylamide;
H₂-β-Nal-D-Cys-Pal-D-Trp-Lys-Thr-Cys-2R-(2-naphthyl)ethylamide;
(H) (CH₃CO)-β-Nal-D-Cys-Pal-D-Trp-Lys-Thr-Cys-2R- (2-naphthyl)ethylamide;
(H) (4-(2-hydroxyethyl)-1-piperazinylacetyl)-β-Nal-D-Cys-Pal-D-Trp-Lys-Thr-Cys-2R-(2-naphthyl)ethylamide;
(H) (4-(2-hydroxyethyl)-1-piperizineethanesulfonyl)-β-Nal-D-Cys-Pal-D-Trp-Lys-Thr-Cys-2R-(2-naphthyl)ethylamide;
H₂-Phe-D-Cys-Tyr-D-Trp-Lys-Val-Cys-2R-(2-naphthyl)ethylamide;
(H) (CH₃CO) Phe-D-Cys-Tyr-D-Trp-Lys-Val-Cys-2R- (2-naphthyl)ethylamide;
(H) (4-(2-hydroxyethyl)-1-piperazinylacetyl)Phe-D-Cys-Tyr-D-Trp-Lys-Val-Cys-2R-(2-naphthyl)ethylamide;
(H) (4-(2-hydroxyethyl)-1-piperizineethanesulfonyl) Phe-D-Cys-Tyr-D-Trp-Lys-Val-Cys-2R-(2-naphthyl)ethylamide;
H₂-Phe-D-Cys-Pal-D-Trp-Lys-Val-Cys-2R-(2-naphthyl)ethylamide;
(H) (CH₃CO) Phe-Cys-Pal-D-Trp-Lys-Val-Cys-2R- (2-naphthyl)ethylamide;
(H) (4-(2-hydroxyethyl)-1-piperazinylacetyl)Phe-D-Cys-Pal-D-Trp-Lys-Val-Cys-2R-(2-naphthyl)ethylamide;
(H) (4-(2-hydroxyethyl)-1-piperizineethanesulfonyl) Phe-D-Cys-Pal-D-Trp-Lys-Val-Cys-2R-(2-naphthyl)ethylamide;
H₂-Phe-D-Cys-Tyr-D-Trp-Lys-Thr-Cys-2R-(2-naphthyl)ethylamide;
(H) (CH₃CO) Phe-D-Cys-Tyr-D-Trp-Lys-Thr-Cys-2R- (2-naphthyl)ethylamide;
(H) (4-(2-hydroxyethyl)-1-piperazinylacetyl)Phe-D-Cys-Tyr-D-Trp-Lys-Thr-Cys-2R-(2-naphthyl)ethylamide;
(H) (4-(2-hydroxyethyl)-1-piperizineethanesulfonyl)
Phe-D-Cys-Tyr-D-Trp-Lys-Thr-Cys-2R-(2-naphthyl)ethylamide;
H₂-Phe-D-Cys-Pal-D-Trp-Lys-Thr-Cys-2R-(2-naphthyl)ethylamide;
(H) (CH₃CO) Phe-Cys-Pal-D-Trp-Lys-Thr-Cys-2R- (2-naphthyl)ethylamide;
(H) (4-(2-hydroxyethyl)-1-piperazinylacetyl)Phe-D-Cys-Pal-D-Trp-Lys-Thr-Cys-2R-(2-naphthyl)ethylamide;
(H) (4-(2-hydroxyethyl)-1-piperizineethanesulfonyl) Phe-D-Cys-Pal-D-Trp-Lys-Thr-Cys-2R-(2-naphthyl)ethylamide;
H₂-β-Nal-D-Cys-Tyr-D-Trp-Lys-Abu-Cys-2R-(2-naphthyl)ethylamide;
H₂-Phe-D-Cys-Tyr-D-Trp-Lys-Abu-Cys-2R-(2-naphthyl)ethylamide;
H₂-β-Nal-D-Cys-Tyr-D-Trp-Lys-Abu-Cys-2R,3R-(2-hydroxymethyl)-3-hydroxy)propylamide; or
H₂-Phe-D-Cys-Tyr-D-Trp-Lys-Abu-Cys-2R,3R-(2-hydroxymethyl)-3-hydroxy)propylamide; or a pharmaceutically acceptable salt thereof.

19. A compound of claim 1, wherein A₂ is a D-aromatic amino acid or a D-aliphatic amino acid, A₇ is an aromatic amino acid or an aliphatic amino acid, and A₄ is D-trp.

20. A compound of claim 19, wherein A₁ is an L-amino acid and A₂ is a D-aromatic amino acid.

21. A compound of claim 20, wherein A₁, A₃, and A₇ independently, is β-Nal, o-X-Phe (where X is H, OH, CH₃, halo, OCH₃, NH₂, CN or NO₂), p-X-Phe (where X is H, OH, CH₃, halo, OCH₃, NH₂, CN or NO₂), m-X-Phe (where X is H, OH, CH₃, halo, OCH₃, NH₂, CN, or NO₂), F₅-Phe, Trp, Dip, 2-Pal, Tyr (Bzl), His, Igl, Tyr (I), Bta, Bip, Npa, or Pal; A² is D-β-Nal, D-o-X-Phe (where X is H, OH, CH₃, halo, OCH₃, NH₂, CN, or NO₂), D-p-X-Phe (where X is H, OH, CH₃, halo, OCH₃, NH₂, CN, or NO₂), D-m-X-Phe (where X is H, OH, CH₃, halo, OCH₃, NH₂, CN, or NO₂), D-F₅-Phe, D-Trp, D-Dip, D-2-Pal, D-Tyr (Bzl), D-His, D-Igl, D-Tyr(I), DBta, D-Bip, D-Npa, or D-Pal; A⁶ is Thr, Ser, Tle, Thr(Bzl), Abu, Ala, Ile, Leu, Gly, Nle, β-Ala, Gaba, or Val; and A⁸ is the D- or L-isomer of Thr, Dip, F₅-Phe, p-X-Phe (where X is H, OH, CH₃, halo, OCH₃, NH₂, CN, or NO₂), o-X-Phe (where X is H, OH, CH₃, halo, OCH₃, NH₂, CN, or NO₂), m-X-Phe (where X is H, OH, CH₃, halo, OCH₃, NH₂, CN, or NO₂), Igl, Tyr (Bzl), or β-Nal.

22. A compound of claim 21, wherein A¹ is β-Nal or Phe, A² is D-Cpa or D-Phe; A³ is Phe or Tyr; A⁶ is Abu, Thr, or Val; A⁷ is Phe; and A⁸ is Thr; R₁ is H, CH₃CO, 4- (2-hydroxyethyl) -1-piperazinylacetyl, or 4-(2-hydroxyethyl)-1-piperizineethanesulfonyl; R₂ is H; and R₃ is NH₂; or
of the formula:
H₂-Phe-D-Phe-Tyr-D-Trp-Lys-Thr-Phe-Thr-NH₂;
H₂-Phe-D-Phe-Tyr-D-Trp-Lys-Val-Phe-Thr-NH₂;
H₂-Phe-D-Cpa-Tyr-D-Trp-Lys-Val-Phe-Thr-NH₂;
H₂-β-Nal-D-Cpa-Tyr-D-Trp-Lys-Val-phe-Thr-NH₂;
(H) (CH₃CO)-β- Nal-D-Cpa-Tyr-D-Trp-Lys-Val-Phe-Thr-NH₂;
(H) (4-(2-hydroxyethyl)-1-piperazinylacetyl)-β-Nal-D-Cpa-Tyr-D-Trp-Lys-Val-Phe-Thr-NH₂;
(H) (4-(2-hydroxyethyl)-1-piperizineethanesulfonyl)-β-Nal-D-Cpa-Tyr-D-Trp-LyS-Val-Phe-Thr-NH₂;
H₂-β-Nal-D-Cpa -Pal- D-Trp-Lys-Val-Phe-Thr-NH₂;
(H) (CH₃CO)-β-Nal-D-Cpa-Pal-D-Trp-Lys-Val-Phe-Thr-NH₂;
(H) (4-(2-hydroxyethyl)-1-piperazinylacetyl)-β-Nal-D-Cpa-Pal-D-Trp-Lys-Val-Phe-Thr-NH₂;
(H) (4-(2-hydroxyethyl)-1-piperizineethanesulfonyl)-β-Nal-D-Cpa-Pal-D-Trp-Lys-Val-Phe-Thr-NH₂;
H₂-β-Nal-D-Cpa-Tyr-D-Trp-Lys-Thr-Phe-Thr-NH₂;
(H) (CH₃CO)-β-Nal-D-Cpa-Tyr-D-Trp-Lys-Thr-Phe-Thr-NH₂;
(H) (4-(2-hydroxyethyl)-1-piperazinylacetyl)-β-Nal-D-Cpa-Tyr-D-Trp-Lys-Thr-Phe-Thr-NH₂;
(H) (4-(2-hydroxyethyl)-1-piperizineethanesulfonyl)-β-Nal-D-Cpa-Tyr-D-Trp-Lys-Thr-Phe-Thr-NH₂;
H₂-β-Nal-D-Cpa-Pal-D-Trp-Lys-Thr-Phe-Thr-NH₂;
(H) (CH₃CO)-β-Nal-D-Cpa-Pal-D-Trp-Lys-Thr-Phe-Thr-NH₂;
(H) (4-(2-hydroxyethyl)-1-piperazinylacetyl)-β-Nal-D-Cpa-Pal-D-Trp-Lys-Thr-Phe-Thr-NH₂;
(H) (4-(2-hydroxyethyl)-1-piperizineethanesulfonyl)-β-Nal-D-Cpa-Pal-D-Trp-Lys-Thr-Phe-Thr-NH₂;
H₂-β-Nal-D-Cpa-Tyr-D-Trp-Lys-Val-Phe-β-Nal-NH2;
(H) (CH₃CO)-β-Nal-D-Cpa-Tyr-D-Trp-Lys-Val-Phe-β-Nal-NH₂; (H) (4-(2-hydroxyethyl)-1-piperazinylacetyl)-β-Nal-D-Cpa-Tyr-D-Trp-Lys-Val-Phe-β-Nal-NH₂; or
(H) (4-(2-hydroxyethyl)-1-piperizineethanesulfonyl)-β-Nal-D-Cpa-Tyr-D-Trp-Lys-Val-Phe-β-Nal-NH₂;
H₂-β-Nal-D-Cpa-Tyr-D-Trp-Lys-Val-Phe-β-Nal-NH₂-; or
H₂-β-Nal-D-Cpa-Tyr-D-Trp-Lys-Val-Phe-Thr-NH₂; or a pharmaceutically acceptable salt thereof.

23. A compound of claim 19, wherein A¹ is a D-amino acid and A² is a D-aromatic amino acid.

24. A compound of claim 23, wherein A¹ and A², independently, is D-β-Nal, D-o-X-Phe (where X is H, OH, CH₃, halo, OCH₃, NH₂, CN, or NO₂), D-p-X-Phe (where X is H, OH, CH₃, halo, OCH₃, NH₂, CN, or NO₂), D-m-X-Phe (where X is H, OH, CH₃, halo, OCH₃, NH₂, CN, or NO₂), D-F₅-Phe, D-Trp, D-Dip, D-2-Pal, D-Tyr (Bzl), D-His, D-Igl, D-Tyr(I), D-Bta, D-Bip, D-Npa, or DPal; A³ and A⁷, independently, is β-Nal, o-X-Phe (where X is H, OH, CH₃, halo, OCH₃, NH₂, CN, or NO₂), p-X-Phe (where X is H, OH, CH₃, halo, OCH₃, NH₂, CN, or NO₂), m-X-Phe (where X is H, OH, CH₃, halo, OCH₃, NH₂, CN, or NO₂), F₅-Phe, Trp, Dip, 2-Pal, His, Igl, Tyr(I), Bta, Bip, Npa, Tyr(Bzl), or Pal; A⁶ is Thr, Ser, Tle, Thr(Bzl), Abu, Ala, Ile, Leu, Gly, Nle, β-Ala, Gaba, or Val; and A⁸ is the D- or L-isomer of Thr, Dip, F₅-Phe, p-XPhe (where X is H, OH,CH₃, halo, OCH₃, NH₂, CN, or NO₂), o-X-Phe (where X is H, OH,CH₃, halo, OCH₃, NH₂, CN, or NO₂), m-X-Phe (where X is H, OH,CH₃, halo, OCH₃, NH₂, CN, or NO₂), Igl, Tyr(Bzl), or β-Nal.

25. A compound of claim 24, wherein A¹ is D-β-Nal or D-Phe; A² is D-Cpa or D-Phe; A³ is Phe or Tyr; A⁶ is Thr or Val; A⁷ is Phe; and A⁸ is Thr; R₁ is H, CH₃CO, 4-(2-hydroxyethyl)-1-piperazinylacetyl, or 4-(2-hydroxyethyl)-1-piperizineethanesulfonyl; R₂ is H; and R₃ is NH₂; or
of the formula:
H₂-D-β-Nal-D-Cpa-Phe-D-Trp-Lys-Val-Phe-Thr-NH₂;
H₂-D-β-Nal-D-Phe-Tyr-D-Trp-Lys-Thr-Phe-Thr-NH₂;
H₂-D-Phe-D-Phe-Tyr-D-Trp-Lys-Val-Phe-Thr-NH₂;
H₂-D-β-Nal-D-Cpa-Tyr-D-Trp-Zys-Val-Phe-Thr-NH₂; or
H₂-D-β-Nal-D-cpa-Tyr-D-Trp-Lys-Val-Phe-β-Nal-NH₂; or
a pharmaceutically acceptable salt thereof.

## Patentansprüche

1. Verbindung mit der Formel: 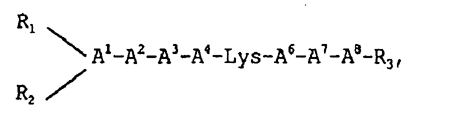 worin
A¹ ein D- oder L-Isomer einer aromatischen Aminosäure ist oder fehlt;
A² ein D-Isomer ausgewählt aus der Gruppe bestehend aus Cys, Pen, einer aromatischen Aminosäure oder einer aliphatischen Aminosäure ist;
A³ eine aromatische Aminosäure ist;
A⁴ Trp oder D-Trp ist;
A⁶ Thr, Thr(Bzl), Gly, Ser, ein Eaa oder eine aliphatische Aminosäure ist;
A⁷ Cys, Pen oder eine aromatische oder aliphatische Aminosäure ist;
A⁸ ein D- oder L-Isomer ausgewählt aus der Gruppe bestehend aus Thr, Ser, einer aromatischen Aminosäure oder einer aliphatischen Aminosäure ist;
jedes von R₁ und R₂ unabhängig H oder substituiertes oder unsubstituiertes niederes Alkyl, Aryl, Aryl-niederes Alkyl, Heterocyclus, Heterocyclus-niederes Alkyl, E₁SO₂ oder E₁CO ist (wobei E₁ Aryl, Aryl-niederes Alkyl, Heterocyclus oder Heterocyclus-niederes Alkyl ist), wobei der Substituent Halogen, niederes Alkyl, Hydroxy, Halogen-niederes Alkyl oder Hydroxy-niederes Alkyl ist, und
R₃ OH, NH₂, C₁₋₁₂-Alkoxy oder NH-Y-CH₂-Z ist, wobei Y eine C₁₋₁₂-Kohlenwasserstoffeinheit ist und Z H, OH, CO₂H oder CONH₂ ist, oder R₃ zusammen mit der daran gebundenen Carbonylgruppe von A⁸ unter Bildung von H, niederem Alkyl oder Hydroxy-niederem Alkyl reduziert worden ist; mit der Maßgabe, dass, wenn A² D-Cys oder D-Pen ist und A⁷ Cys oder Pen ist, dann eine Disulfidbrücke die Seitenketten von A² und A⁷ verbindet, und wenn A¹ D-Phe oder p-NO₂-Phe ist; A² D-Cys ist; A³ Phe oder Tyr ist; A⁶ Thr oder Val ist und A⁷ Cys ist; dann A⁸ β-Nal ist.

2. Verbindung nach Anspruch 1, bei der A² D-Cys ist, A⁷ Cys ist und A⁴ D-Trp ist.

3. Verbindung nach Anspruch 2, bei der A₁ eine aromatische L-Aminosäure ist.

4. Verbindung nach Anspruch 3, bei der A¹ und A³ unabhängig β-Nal, o-X-Phe (wobei X H, OH, CH₃, Halogen, OCH₃, NH₂, CN, oder NO₂ ist), p-X-Phe (wobei X H, OH CH₃, Halogen, OCH₃, NH₂, CN oder NO₂ ist), m-X-Phe (wobei X H, OH CH₃, Halogen, OCH₃, NH₂, CN oder NO₂ ist), F₅-phe, Trp, Dip, 2-Pal, Tyr(Bzl), His, Igl, Tyr(I), Bta, Bip, Npa oder Pal sind; A⁶ Thr, Ser, Tle, Thr(Bzl), Abu, Ala, Ile, Leu, Gly, Nle, β-Ala, Gaba oder Val ist; und A⁸ das D- oder L-Isomer von Thr, Dip, F₅-Phe, p-X-Phe (wobei X H, OH, CH₃, Halogen, OCH₃, NH₂, CN oder NO₂ ist), o-X-Phe (wobei X H, OH, CH₃, Halogen, OCH₃, NH₂, CN oder NO₂ ist), m-X-Phe (wobei X H, OH, CH₃, Halogen, OCH₃, NH₂, CN oder NO₂ ist), Igl, Tyr(Bzl) oder β-Nal ist.

5. Verbindung nach Anspruch 4, bei der A¹ β-Nal, Npa, Igl, Phe, p-F-Phe, Trp, p-Cl-Phe oder p-CN-Phe ist; A³ Tyr, Tyr(I) oder Pal ist; A⁶ Val, Tle, Nle, Ile oder Leu ist; A⁸ p-F-Phe, β-Nal, Tyr, Dip, p-Cl-Phe, Igl oder p-CN-Phe ist; R₁ H, CH₃CO, 4-(2-Hydroxyethyl)-1-piperazinylacetyl, oder 4-(2-Hydroxyethyl)-1-piperizinethansulfonyl ist; R₂ H ist und R₃ NH₂ ist.

6. Verbindung nach Anspruch 4 mit der Formel: H₂-β-Nal-D-Cys-Tyr-D-Trp-Lys-Val-Cys-β-Nal-NH₂;
(H)(CH₃CO)-β-Nal-D-Cys-Tyr-D-Trp-Lys-Val-Cys-β-Nal-NH₂ (V) ;
(H)-(4-(2-Hydroxyethyl)-1-piperazinylacetyl)-β-Nal-D-Cys-Tyr-D-Trp-Lys-Val-Cys-β-Nal-NH₂;
(H)-(4-(2-Hydroxyethyl)-1-piperizinethansulfonyl)-β-Nal-D-Cys-Tyr-D-Trp-Lys-Val-Cys-β-Nal-NH₂;
H₂-β-Nal-D-Cys-Pal-D-Trp-Lys-Val-Cys-β-Nal-NH₂;
(H)(CH₃CO)-β-Nal-D-Cys-Pal-D-Trp-Lys-Val-Cys-β-Nal-NH₂;
(H)-(4-(2-Hydroxyethyl)-1-piperazinylacetyl)-β-Nal-D-Cys-Pal-D-Trp-Lys-Val-Cys-β-Nal-NH₂;
(H)-(4-(2-Hydroxyethyl)-1-piperizinethansulfonyl)-β-Nal-D-Cys-Pal-D-Trp-Lys-Val-Cys-β-Nal-NH₂;
H₂-β-Nal-D-Cys-Tyr-D-Trp-Lys-Val-Cys-Thr-NH₂;
(H) (CH₃CO)-β-Nal-D-Cys-Tyr-D-Trp-Lys-Val-Cys-Thr-NH₂;
(H) (4-(2-Hydroxyethyl)-1-piperazinylacetyl)-β-Nal-D-Cys-Tyr-D-Trp-Lys-Val-Cys-Thr-NH₂;
(H) (4-(2-Hydroxyethyl)-1-piperizinethansulfonyl)-β-Nal-D-Cys-Tyr-D-Trp-Lys-Val-Cys-Thr-NH₂;
H₂-β-Nal-D-Cys-Pal-D-Trp-Lys-Val-Cys-Thr-NH₂;
(H) (CH₃CO)-β-Nal-D-Cys-Pal-D-Trp-Lys-Val-Cys-Thr-NH₂;
(H) (4-(2-Hydroxyethyl)-1-piperazinylacetyl)-β-Nal-D-Cys-Pal-D-Trp-Lys-Val-Cys-Thr-NH₂;
(H) (4- (2-Hydroxyethyl)-1-piperizinethansulfonyl)-O-Nal-D-Cys-Pal-D-Trp-Lys-Val-Cys-Thr-NH₂;
H₂-Phe-D-Cys-Tyr-D-Trp-Lys-Val-Cys-β-Nal-NH₂;
(H) (CH₃CO) Phe-D-Cys-Tyr-D-Trp-Lys-Val-Cys-β-Nal-NH₂;
(H) (4-(2-Hydroxyethyl)-1-piperazinylacetyl)-Phe-D-Cys-Tyr-D-Trp-Lys-Val-Cys-β-Nal-NH₂;
(H) (4- (2-Hydroxyethyl) -1-piperizinethansulfonyl) -) Phe-D-Cys-Tyr-D-Trp-Lys-Val-Cys-β-Nal-NH₂;
H₂-Phe-D-Cys-Pal-D-Trp-Lys-Val-Cys-β-Nal-NH₂;
(H) (CH₃CO)Phe-D-Cys-Pal-D-Trp-Lys-Val-Cys-β-Nal-NH₂;
(H)(4-(2-Hydroxyethyl)-1-piperazinylacetyl)-Phe-D-Cys-Pal-D-Trp-Lys-Val-Cys-β-Nal-NH₂;
(H)(4-(2-Hydroxyethyl)-1-piperizinethansulfonyl)-Phe-D-Cys-Pal-D-Trp-Lys-Val-Cys-β-Nal-NH₂;
H₂-Phe-D-Cys-Pal-D-Trp-Lys-Val-Cys-Thr-NH₂;
(H) (CH₃CO)-Phe-D-Cys-Pal-D-Trp-Lys-Val-Cys-Thr-NH₂;
(H)(4-(2-Hydroxyethyl)-1-piperazinylacetyl)-Phe-D-Cys-Pal-D-Trp-Lys-Val-Cys-Thr-NH₂;
(H)(4-(2-Hydroxyethyl)-1-piperizinethansulfonyl)-Phe-D-Cys-Pal-D-Trp-Lys-Val-Cys-Thr-NH₂;
H₂-β-Nal-D-Cys-Tyr-D-Trp-Lys-Thr-Cys-β-Nal-NH₂;
(H)(CH₃CO)-β-Nal-D-Cys-Pal-D-Trp-Lys-Thr-Cys-β-Nal-NH₂;
(H)(4-(2-Hydroxyethyl)-1-piperazinylacetyl)-β-Nal-D-Cys-Tyr-D-Trp-Lys-Thr-Cys-β-Nal-NH₂;
(H)(4-(2-Hydroxyethyl)-1-piperizinethansulfonyl)-β-Nal-D-Cys-Tyr-D-Trp-Lys-Thr-Cys-β-Nal-NH₂;
H₂-β-Nal-D-Cys-Pal-D-Trp-Lys-Thr-Cys-β-Nal-NH₂;
(H)(CH₃CO)-β-Nal-D-Cys-Pal-D-Trp-Lys-Thr-Cys-β-Nal-NH₂;
(H)(4-(2-Hydroxyethyl)-1-piperazinylacetyl)-β-Nal-D-Cys-Pal-D-Trp-Lys-Thr-Cys-β-Nal-NH₂;
(H)(4-(2-Hydroxyethyl)-1-piperizinethansulfonyl)-β-Nal-D-Cys-Pal-D-Trp-Lys-Thr-Cys-β-Nal-NH₂;
H₂-β-Nal-D-Cys-Tyr-D-Trp-Lys-Thr-Cys-Thr-NH₂;
H(CH₃CO)-β-Nal-D-Cys-Tyr-D-Trp-Lys-Thr-Cys-Thr-NH₂;
(H)(4-(2-Hydroxyethyl)-1-piperazinylacetyl)-β-Nal-D-Cys-Tyr-D-Trp-Lys-Thr-Cys-Thr-NH₂;
(H)(4-(2-Hydroxyethyl)-1-piperizinethansulfonyl)-β-Nal-D-Cys-Tyr-D-Trp-Lys-Thr-Cys-Thr-NH₂;
H₂-β-Nal-D-Cys-Pal-D-Trp-Lys-Thr-Cys-Thr-NH₂;
(H)(CH₃CO)-β-Nal-D-Cys-Pal-D-Trp-Lys-Thr-Cys-Thr-NH₂;
(H)(4-(2-Hydroxyethyl)-1-piperazinylacetyl)-β-Nal-D-Cys-Pal-D-Trp-Lys-Thr-Cys-Thr-NH₂;
(H)(4-(2-Hydroxyethyl)-1-piperizinethansulfonyl)-β-Nal-D-Cys-Pal-D-Trp-Lys-Thr-Cys-Thr-NH₂;
H₂-Phe-D-Cys-Tyr-D-Trp-Lys-Thr-Cys-β-Nal-NH₂;
(H)(CH₃CO)Phe-D-Cys-Tyr-D-Trp-Lys-Thr-Cys-β-Nal-NH₂;
(H)(4-(2-Hydroxyethyl)-1-piperazinylacetyl)Phe-D-Cys-Tyr-D-Trp-Lys-Thr-Cys-β-Nal-NH₂;
(H)(4-(2-Hydroxyethyl)-1-piperizinethansulfonyl)Phe-D-Cys-Tyr-D-Trp-Lys-Thr-Cys-β-Nal-NH₂;
H₂-Phe-D-Cys-Pal-D-Trp-Lys-Thr-Cys-β-Nal-NH₂;
(H) (CH₃CO) Phe-D-Cys-Pal-D-Trp-Lys-Thr-Cys-β-Nal-NH₂;
(H) (4-(2-Hydroxyethyl)-1-piperazinylacetyl)Phe-D-Cys-Pal-D-Trp-Lys-Thr-Cys-β-Nal-NH₂;
(H)(4-(2-Hydroxyethyl)-1-piperizinethansulfonyl)Phe-D-Cys-Pal-D-Trp-Lys-Thr-Cys-β-Nal-NH₂;
H₂-Phe-D-Cys-Tyr-D-Trp-Lys-Thr-Cys-Thr-NH₂;
(H)(CH₃CO)Phe-D-Cys-Tyr-D-Trp-Lys-Thr-Cys-Thr-NH₂;
(H)(4-(2-Hydroxyethyl)-1-piperazinylacetyl)Phe-D-Cys-Tyr-D-Trp-Lys-Thr-Cys-Thr-NH₂;
(H)(4-(2-Hydroxyethyl)-1-piperizinethansulfonyl)Phe-D-Cys-Tyr-D-Trp-Lys-Thr-Cys-Thr-NH₂;
H₂-Phe-D-Cys-Pal-D-Trp-Lys-Thr-Cys-Thr-NH₂;
(H) (CH₃CO)-Phe-D-Cys-Pal-D-Trp-Lys-Thr-Cys-Thr-NH₂;
(H) (4-(2-Hydroxyethyl)-1-piperazinylacetyl)Phe-D-Cys-Pal-D-Trp-Lys-Thr-Cys-Thr-NH₂;
(H) (4-(2-Hydroxyethyl)-1-piperizinethansulfonyl)Phe-D-Cys-Pal-D-Trp-Lys-Thr-Cys-Thr-NH₂;
H₂-β-Nal-D-Cys-Tyr-D-Trp-Lys-Abu-Cys-β-Nal-NH₂;
H₂-Phe-D-Cys-Tyr-D-Trp-Lys-Abu-Cys-β-Nal-NH₂;
H₂-β-Nal-D-Cys-Pal-D-Trp-Lys-Abu-Cys-β-Nal-NH₂;
H₂-Phe-D-Cys-Pal-D-Trp-Lys-Abu-Cys-β-Nal-NH₂;
H₂-β-Nal-D-Cys-Tyr-D-Trp-Lys-Abu-Cys-Thr-NH₂;
H₂-Phe-D-Pen-Tyr-D-Trp-Lys-Val-Pen-β-Nal-NH₂ oder
H₂-Phe-D-Pen-Pal-D-Trp-Lys-Thr-Pen-Thr-NH₂;
H₂-Dip-D-Cys-Pal-D-Trp-Lys-Val-Cys-Dip-NH₂;
H₂-F₅-Phe-D-Cys-His-D-Trp-Lys-Val-Cys-F₅-Phe-NH₂;
H₂-Dip-D-Cys-Pal-D-Trp-Lys-Val-Cys-β-Nal-NH₂;
H₂-m-F-Phe-D-Cys-Pal-D-Trp-Lys-Val-Cys-m-F-Phe-NH₂;
H₂-o-F-Phe-D-Cys-Pal-D-Trp-Lys-Val-Cys-o-F-Phe-NH₂;
H₂-p-F-Phe-D-Cys-Pal-D-Trp-Lys-Val-Cys-p-F-Phe-NH₂;
H₂-F₅-Phe-D-Cys-Pal-D-Trp-Lys-Val-Cys-F₅-Phe-NH₂;
H₂-F₅-Phe-D-Cys-2-Pal-D-Trp-Lys-Val-Cys-F₅-Phe-NH₂;
H₂-β-Nal-D-Cys-His-D-Trp-Lys-Val-Cys-D-Dip-NH₂;
H₂-Dip-D-Cys-His-D-Trp-Lys-Val-Cys-β-Nal-NH₂;
H₂-Dip-D-Cys-His-D-Trp-Lys-Val-Cys-Dip-NH₂;
H₂-β-Nal-D-Cys-His-D-Trp-Lys-Val-Cys-β-Nal-NH₂;
H₂-Trp-D-Cys-Tyr-D-Trp-Lys-Val-Cys-D-β-Nal-NH₂;
H₂-β-Nal-D-Cys-Tyr-D-Trp-Lys-Val-Cys-D-β-Nal-NH₂;
H_{Z}-β-Nal-D-Cys-Pal-D-Trp-Lys-Val-Cys-D-p-F-Phe-NH₂;
H₂-β-Nal-D-Cys-Pal-D-Trp-Lys-Tle-Cys-β-Nal-NH₂;
H₂-p-F-Phe-D-Cys-Pal-D-Trp-Lys-Val-Cys-β-Nal-NH₂;
H₂-β-Nal-D-Cys-Pal-D-Trp-Lys-Nle-Cys-β-Nal-NH₂;
H₂-β-Nal-D-Cys-Pal-D-Trp-Lys-Ile-Cys-β-Nal-NH₂;
H2-β-Nal-D-Cys-Pal-D-Trp-Lys-Gly-Cys-β-Nal-NH₂ ;
H₂-β-Nal-D-Cys-Pal-D-Trp-Lys-Ala-Cys-β-Nal-NH₂;
H₂-β-Nal-D-Cys-Pal-D-Trp-Lys-Leu-Cys-β-Nal-NH₂;
H₂-Bip-D-Cys-Tyr-D-Trp-Lys-Ile-Cys-Bip-NH₂;
H₂-p-F-Phe-D-Cys-His-D-Trp-Lys-Val-Cys-p-F-Phe-NH₂;
H₂-Npa-D-Cys-Pal-D-Trp-Lys-Val-Cys-Tyr-NH₂;
H₂-m-F-Phe-D-Cys-His-D-Trp-Lys-Val-Cys-m-F-Phe-NH₂;
H₂-o-F-Phe-D-Cys-His-D-Trp-Lys-Val-Cys-o-F-Phe-NH₂;
H₂-β-Nal-D-Cys-Pal-D-Trp-Lys-Val-Cys-Dip-NH₂;
H₂-Cpa-D-Cys-Pal-D-Trp-Lys-Val-Cys-Cpa-NH₂;
H₂-Igl-D-Cys-Pal-D-Trp-Lys-Val-Cys-Igl-NH₂;
H₂-β-Nal-D-Cys-Pal-D-Trp-Lys-Val-Cys-D-Dip-NH₂;
H₂-β-Nal-D-Cys-3-I-Tyr-D-Trp-Lys-Val-Cys-β-Nal-NH₂;
H₂-p-CN-Phe-D-Cys-Pal-D-Trp-Lys-Val-Cys-p-CN-Phe-NH₂;
H₂-β-Nal-D-Cys-Tyr-D-Trp-Lys-Val-Cys-D-Dip-NH₂;
H₂-β-Nal-D-Cys-Bta-D-Trp-Lys-Val-Cys-β-Nal-NH₂;
H₂-p-F-Phe-D-Cys-Pal-D-Trp-Lys-Tle-Cys-β-Nal-NH₂;
H₂-Bpa-D-Cys-Pal-D-Trp-Lys-Val-Cys-Bpa-NH₂;
H₂-Iph-D-Cys-Pal-D-Trp-Lys-Val-Cys-Iph-NH₂;
H₂-Trp-D-Cys-Pal-D-Trp-Lys-Tle-Cys-β-Nal-NH₂;
H₂-p-Cl-Phe-D-Cys-Pal-D-Trp-Lys-Val-Cys-β-Nal-NH₂;
H₂-p-Cl-Phe-D-Cys-Pal-D-Trp-Lys-Tle-Cys-β-Nal-NH₂;
H₂-p-Cl-Phe-D-Cys-Pal-D-Trp-Lys-Tle-Cys-p-Cl-Phe-NH₂;
H₂-p-Cl-Phe-D-Cys-Pal-D-Trp-Lys-Cha-Cys-p-Cl-Phe-NH₂;
H₂-p-Cl-Phe-D-Cys-Tr(I)-D-Trp-Lys-Val-Cys-p-Cl-Phe-NH₂;
H₂-p-Cl-Phe-D-Cys-Tyr(I)-D-Trp-Lys-Val-Cys-β-Nal-NH₂;
H₂-p-Cl-Phe-D-Cys-Tyr(I)-D-Trp-Lys-Tle-Cys-β-Nal-NH₂;
H₂-p-F-Phe-D-Cys-Tyr(I)-D-Trp-Lys-Val-Cys-β-Nal-NH₂;
H₂-p-F-Phe-D-Cys-Tyr(I)-D-Trp-Lys-Tle-Cys-β-Nal-NH₂;
H₂-β-Nal-D-Cys-Tyr-D-Trp-Lys-Abu-Cys-β-Nal-NH₂;
(H) (CH₃CO)-β-Nal-D-Cys-Tyr-D-Trp-Lys-Abu-Cys-β-Nal-NH₂;
H₂-p-NO₂-Phe-D-Cys-Tyr-D-Trp-Lys-Abu-Cys-β-Nal-NH₂;
(H) (CH₃CO)-β-Nal-D-Cys-Tyr-D-Trp-Lys-Abu-Cys-β-Nal-NH₂;
H₂-p-NO₂-Phe-D-Cys-Tyr(Bzl)-D-Trp-Lys-Thr(Bzl)-Cys-Nal-NH₂;
(H) (4-(2-Hydroxyethyl)-1-piperazinylacetyl)-p-NO₂-Phe-D-Cys-Tyr(Bzl)-D-Trp-Lys-Thr(Bzl)-Cys-β-Nal-NH₂;
(H) (4-(2-Hydroxyethyl)-1-piperazinylacetyl)-p-NO₂-Phe-D-Cys-Tyr-D-Trp-Lys-Thr-Cys-Tyr-NH₂;
H₂-β-NO₂-Phe-D-Cys-Tyr-D-Trp-Lys-Val-Cys-β-Nal-NH₂;
(H)(4-(2-Hydroxyethyl)-1-piperazinylacetyl)-β-NO₂-Phe-D-Cys-Tyr-D-Trp-Lys-Val-Cys-β-Nal-NH₂;
(H)(4-(2-Hydroxyethyl)-1-piperazinylacetyl)-β-Nal-Phe-D-Cys-Tyr-D-Trp-Lys-Val-Cys-β-Nal-NH₂;
H₂-β-Nal-D-Cys-Tyr(Bzl)-D-Trp-Lys-Thr(Bzl)-Cys-β-Nal-NH₂; oder
(H)(4-(2-Hydroxyethyl)-1-piperazinylacetyl)-β-Nal-D-Cys-Tyr(Bzl)-D-Trp-Lys-Thr(Bzl)-Cys-Tyr(Bzl)-NH₂ oder ein pharmazeutisch annehmbares Salz davon.

7. Verbindung nach Anspruch 2, bei der A¹ eine aromatische D-Aminosäure ist.

8. Verbindung nach Anspruch 7, bei der A¹ D-β-Nal, D-o-X-Phe (wobei X H, OH, CH₃, Halogen, OCH₃, NH₂, CN oder NO₂ ist), D-p-X-Phe (wobei X H, OH CH₃, Halogen, OCH₃, NH₂, CN oder NO₂ ist), D-m-X-Phe (wobei X H, OH, CH₃, Halogen, OCH₃, NH₂, CN oder NO₂ ist), D-F₅-Phe, D-Trp, D-Dip, D-2-Pal, D-Tyr(Bzl), D-His, D-Igl, D-Tyr(I), D-Bta, D-Bip, D-Npa oder D-Pal ist; A³ β-Nal, o-X-Phe (wobei X H, OH, CH₃, Halogen, OCH₃, NH₂, CN oder NO₂ ist), p-X-Phe (wobei X H, OH CH₃, Halogen, OCH₃, NH₂, CN oder NO₂ ist), m-X-Phe (wobei X H, OH CH₃, Halogen, OCH₃, NH₂, CN oder NO₂ ist), F₅₋Phe, Trp, Dip, 2-Pal, Tyr(Bzl), His, Igl, Tyr(I), Bta, Bip, Npa oder Pal ist; A⁶ Thr, Ser, Tle, Thr(Bzl), Abu, Ala, Ile, Leu, Gly, Nle, β-Ala, Gaba oder Val ist und A⁸ das D- oder L-Isomer von Thr, Dip, F₅-Phe, p-X-Phe (wobei X H, OH CH₃, Halogen, OCH₃, NH₂, CN oder NO₂ ist), o-X-Phe (wobei X H, OH CH₃, Halogen, OCH₃, NH₂, CN oder NO₂ ist), m-X-Phe (wobei X H, OH CH₃, Halogen, OCH₃, NH₂, CN oder NO₂ ist), Igl, Tyr(Bzl) oder β-Nal ist.

9. Verbindung nach Anspruch 8, bei der A¹ D-β-Nal, D-Npa, D-Igl, D-Phe, D-p-F-Phe, D-Trp, D-p-Cl-Phe oder D-p-CN-Phe ist; A³ Tyr, Tyr (I) oder Pal ist; A⁶ Val, Tle, Nle, Ile oder Leu ist; A⁸ p-F-Phe, β-Nal, Tyr, Dip, p-Cl-Phe, Igl, oder p-CN-Phe ist; R₁ H, CH₃CO, 4-(2-Hydroxyethyl)-1-piperazinylacetyl oder 4-(2-Hydroxyethyl)-1-piperizinethansulfonyl ist; R₂ H ist und R₃ NH₂ ist.

10. Verbindung nach Anspruch 7 mit der Formel:
H₂-D-Phe-D-Pen-Tyr-D-Trp-Lys-Val-Cys-Thr-NH₂;
H₂-D-β-Nal-D-Cys-Tyr-D-Trp-Lys-Val-Cys-Thr-NH₂;
H₂-D-β-Nal-D-Cys-Tyr-D-Trp-Lys-Val-Cys-β-Nal-NH₂;
H₂-D-β-Nal-D-Cys-Tyr-D-Trp-Lys-Thr-Cys-β-Nal-NH₂;
H₂-D-Phe-D-Cys-Pal-D-Trp-Lys-Thr-Cys-Thr-NH₂;
H₂-D-Phe-D-Cys-Tyr-D-Trp-Lys-Abu-Cys-Thr-NH₂;
H₂-D-β-Nal-D-Cys-Tyr-D-Trp-Lys-Abu-Cys-Thr-NH₂;
H₂-D-β-Nal-D-Cys-Tyr-D-Trp-Lys-Val-Cys-D-β-Nal-NH₂;
H₂-D-p-F-Phe-D-Cys-Pal-D-Trp-Lys-Val-Cys-D-p-F-Phe-NH₂;
H₂-D-Bip-D-Cys-Tyr-D-Trp-Lys-Val-Cys-β-Nal-NH₂;
H₂-D-Dip-D-Cys-Pal-D-Trp-Lys-Val-Cys-β-Nal-NH₂;
H₂-D-p-F-Phe-D-Cys-Pal-D-Trp-Lys-Tle-Cys-β-Nal-NH₂;
H₂-D-p-Cl-Phe-D-Cys-Pal-D-Trp-Lys-Tle-Cys-p-Cl-Phe-NH₂;
p-NO₂-D-Phe-D-Cys-Pal-D-Trp-Lys-Thr(Bzl)-Cys-Tyr(Bzl)-NH₂;
p-NO₂-D-Phe-D-Cys-Tyr(Bzl)-D-Trp-Lys-Val-Cys-Tyr(Bzl)-NH₂;
(H) (4- (2-Hydroxyethyl) -1-piperazinylacetyl) -p-NO²-D-Phe-D-Cys-Pal-D-Trp-Lys-Thr(Bzl)-Cys-Tyr(Bzl)-NH₂ oder
(H)(4-(2-Hydroxyethyl)-1-piperazinylacetyl)-p-NO₂-D-Phe-D-Cys-Tyr(Bzl)-D-Trp-Lys-Val-Cys-Tyr(Bzl)-NH₂ oder ein pharmazeutisch annehmbares Salz davon.

11. Verbindung nach Anspruch 2, bei der A¹ fehlt, R₁ ein substituiertes oder unsubstituiertes E₁CO ist und R₂ H ist.

12. Verbindung nach Anspruch 11, bei der R₁ substituiertes oder unsubstituiertes E₁CO ist (wobei E₁ Phenyl, β-Naphthylmethyl, β-Pyridinylmethyl oder 3-Indolylmethyl ist); A³ β-Nal, o-X-Phe (wobei X H, OH CH₃, Halogen, OCH₃, NH₂, CN oder NO₂ ist), p-X-Phe (wobei X H, OH CH₃, Halogen, OCH₃, NH₂, CN oder NO₂ ist), m-X-Phe (wobei X H, OH CH₃, Halogen, OCH₃, NH₂, CN oder NO₂ ist), F₅-Phe, Trp, Dip, 2-Pal, Tyr(Bzl), His, Igl, Tyr(I), Bta, Bip, Npa oder Pal ist; A⁶ Thr, Ser, Tle, Thr(Bzl), Abu, Ala, Ile, Leu, Gly, Nle, β-Ala, Gaba oder Val ist und A⁸ das D- oder L-Isomer von Thr, Dip, F₅-Phe, p-X-Phe (wobei X H, OH, CH₃, Halogen, OCH₃, NH₂, CN oder NO₂ ist), o-X-Phe (wobei X H, OH, CH₃, Halogen, OCH₃, NH₂, CN oder NO₂ ist), m-X-Phe (wobei X H, OH, CH₃, Halogen, OCH₃, NH₂, CN oder NO₂ ist), Igl, Tyr(Bzl) oder β-Nal ist.

13. Verbindung nach Anspruch 12, wobei R₁ E₁CO ist (wobei E₁ 4-Hydroxyphenyl, β-Naphthylmethyl oder Phenyl ist); A³ Tyr, Tyr(I) oder Pal ist; A⁶ Val, Tle, Nle, Ile oder Leu ist; A⁸ p-F-Phe, β-Nal, Tyr, Dip, p-Cl-Phe, Igl oder p-CN-Phe ist; R₃ NH₂ ist; oder die folgende Formel hat
(H) (3-Phenylpropionyl)-D-Cys-Tyr-D-Trp-Lys-Val-Cys-β-Nal-NH₂;
(H) (3-Phenylpropionyl)-D-Cys-Pal-D-Trp-Lys-Val-Cys-β-Nal-NH₂;
(H)(3-Phenylpropionyl)-D-Cys-Tyr-D-Trp-Lys-Thr-Cys-β-Nal-NH₂;
(H) (3-Phenylpropionyl)-D-Cys-Pal-D-Trp-Lys-Thr-Cys-β-Nal-NH₂;
(H)(3-Phenylpropionyl)-D-Cys-Tyr-D-Trp-Lys-Val-Cys-Thr-NH₂;
(H)(3-Phenylpropionyl)-D-Cys-Pal-D-Trp-Lys-Val-Cys-Thr-NH₂;
(H)(3-Phenylpropionyl)-D-Cys-Tyr-D-Trp-Lys-Thr-Cys-Thr-NH₂;
(H) (3-Phenylpropionyl)-D-Cys-Pal-D-Trp-Lys-Thr-Cys-Thr-NH₂;
(H) (3-[2-Naphthyl]propionyl)-D-Cys-Tyr-D-Trp-Lys-Val-Cys-β-Nal-NH₂;
(H)(3-[2-Naphthyl]propionyl)-D-Cys-Pal-D-Trp-Lys-Val-Cys-β-Nal-NH₂;
(H)(3-[2-Naphthyl]propionyl)-D-Cys-Tyr-D-Trp-Lys-Thr-Cys-β-Nal-NH₂;
(H)(3-[2-Naphthyl]propionyl)-D-Cys-Pal-D-Trp-Lys-Thr-Cys-β-Nal-NH₂ ;
(H) (3-[2-Naphthyl]propionyl)-D-Cys-Tyr-D-Trp-Lys-Val-Cys-Thr-NH₂;
(H) (3-[2-Naphthyl]propionyl)-D-Cys-Pal-D-Trp-Lys-Val-Cys-Thr-NH₂;
(H) (3-[2-Naphthyl]propionyl)-D-Cys-Tyr-D-Trp-Lys-Thr-Cys-Thr-NH₂;
(H) (3-[2-Naphthyl]propionyl)-D-Cys-Pal-D-Trp-Lys-Thr-Cys-Thr-NH₂;
(H) (3-[p-Hydroxyphenyl])-D-Cys-Tyr-D-Trp-Lys-Val-Cys-β-Nal-NH₂;
(H) (3-Naphthyl]propionyl)-D-Cys-Tyr-D-Trp-Lys-Abu-Cys-β-Nal- NH₂;
(H) (3-Naphthyl]propionyl)-D-Cys-Tyr-D-Trp-Lys-Abu-Cys-Thr-NH₂;
(H) (3-Phenylylpropionyl)-D-Cys-Tyr-D-Trp-Lys-Abu-Cys-β-Nal-NH₂ oder
(H)(3-Phenylylpropionyl)-D-Cys-Tyr-D-Trp-Lys-Abu-Cys-Thr-NH₂ oder
ein pharmazeutisch annehmbares Salz davon.

14. Verbindung nach Anspruch 2, bei der R₃ zusammen mit der daran gebundenen Carbonylgruppe von A⁸ unter Bildung von H, niederem Alkyl oder Hydroxy-niederem Alkyl reduziert worden ist.

15. Verbindung nach Anspruch 14, wobei A¹ das D- oder L-Isomer von β-Nal, o-X-Phe (wobei X H, OH, CH₃, Halogen, OCH₃, NH₂, CN oder NO₂ ist), -p-X-Phe (wobei X H, OH, CH₃, Halogen, OCH₃, NH₂, CN oder NO₂ ist), m-X-Phe (wobei X H, OH, CH₃, Halogen, OCH₃, NH₂, CN oder NO₂ ist), F₅-Phe, Trp, Dip, 2-Pal, Tyr(Bzl), His, Igl, Tyr(I), Bta, Bip, Npa oder Pal ist; A³ β-Nal, o-X-Phe (wobei X H, OH CH₃, Halogen, OCH₃, NH₂, CN oder NO₂ ist), p-X-Phe (wobei X H, OH CH₃, Halogen, OCH₃, NH₂, CN oder NO₂ ist), m-X-Phe (wobei X H, OH CH₃, Halogen, OCH₃, NH₂, CN oder NO₂ ist), F₅₋Phe, Trp, Dip, 2-Pal, Tyr(Bzl), His, Igl, Tyr(I), Bta, Bip, Npa oder Pal ist; A⁶ Thr, Ser, Tle, Thr(Bzl), Abu, Ala, Ile, Leu, Gly, Nle, β-Ala, Gaba oder Val ist; und A⁸ das D- oder L-Isomer von Thr, Dip, F₅-Phe, p-X-Phe (wobei X H, OH, CH₃, Halogen, OCH₃, NH₂, CN oder NO₂ ist), o-X-Phe (wobei X H, OH, CH₃, Halogen, OCH₃, NH₂, CN oder NO₂ ist), m-X-Phe (wobei X H, OH, CH₃, Halogen, OCH₃, NH₂, CN oder NO₂ ist), Igl, Tyr(Bzl) oder β-Nal ist.

16. Verbindung nach Anspruch 15, bei der A¹ das D- oder L-Isomer von β-Nal, Phe, p-F-Phe, Trp, p-Cl-Phe oder p-CN-Phe ist; A³ Tyr, Tyr(I) oder Pal ist; A⁶ Val, Tle, Nle, Ile oder Leu ist; A⁸ p-F-Phe, β-Nal, Tyr, Dip, p-Cl-Phe, Igl oder p-CN-Phe ist; R₁ H, CH₃CO, 4-(2-Hydroxyethyl)-1-piperazinylacetyl oder 4-(2-Hydroxyethyl)-1-piperizinethansulfonyl ist; R₂ H ist und R₃ zusammen mit der daran gebundenen Carboxygruppe von A⁸ unter Bildung von H oder CH₃OH reduziert worden ist.

17. Verbindung nach Anspruch 9, 13 oder 16, bei der A³ Pal ist.

18. Verbindung nach Anspruch 15 mit der Formel:
H₂-β-Nal-D-Cys-Tyr-D-Trp-Lys-Val-Cys-2R,3R-(2-Hydroxymethyl)-3-hydroxy)propylamid;
(H) (CH₃CO)-β-Nal-D-Cys-Tyr-D-Trp-Lys-Val-Cys-2R, 3R-(2-Hydroxymethyl)-3-hydroxy)propylamid;
(H) (4-(2-Hydroxyethyl)-1-piperazinylacetyl)-β-Nal-D-) Cys-Tyr-D-Trp-Lys-Val-Cys-2R,3R-(2-hydroxymethyl)-3-hydroxy)propylamid;
(H) (4-(2-Hydroxyethyl)-1-piperizinethansulfonyl)-β-Nal-D-Cys-Tyr-D-Trp-Lys-Val-Cys-2R,3R-(2-hydroxymethyl)-3-hydroxy)propylamid;
H₂-β-Nal-D-Cys-Pal-D-Trp-Lys-Val-Cys-2R,3R-(2-Hydroxymethyl)-3-hydroxy)propylamid;
(H)(CH₃CO)-β-Nal-D-Cys-Pal-D-Trp-Lys-Val-Cys-2R,3R-(2-Hydroxymethyl)-3-hydroxy)propylamid;
(H)(4-(2-Hydroxyethyl)-1-piperazinylacetyl)-β-Nal-D-Cys-Pal-D-Trp-Lys-Val-Cys-2R,3R-(2-hydroxymethyl)-3-hydroxy)-propylamid;
(H) (4-(2-Hydroxyethyl)-1-piperizinethansulfonyl)-β-Nal-D-Cys-Pal-D-Trp-Lys-Val-Cys-2R,3R-(2-hydroxymethyl)-3-hydroxy)propylamid;
H₂-β-Nal-D-Cys-Tyr-D-Trp-Lys-Thr-Cys-2R,3R-(2-hydroxymethyl)-3-hydroxy)propylamid;
(H) (CH₃CO)-β-Nal-D-Cys-Tyr-D-Trp-Lys-Thr-Cys-2R, 3R-(2-hydroxymethyl)-3-hydroxy)propylamid;
(H) (4-(2-Hydroxyethyl)-1-piperazinylacetyl)-β-Nal-D-Cys-Tyr-D-Trp-Lys-Thr-Cys-2R,3R-(2-hydroxymethyl)-3-hydroxy)-propylamid;
(H)(4-(2-Hydroxyethyl)-1-piperizinethansulfonyl)-β-Nal-D-Cys-Tyr-D-Trp-Lys-Thr-Cys-2R,3R-(2-hydroxymethyl)-3-hydroxy)propylamid;
H₂-β-Nal-D-Cys-Pal-D-Trp-Lys-Thr-Cys-2R,3R-(2-hydroxymethyl)-3-hydroxy)propylamid;
(H) (CH₃CO)-β-Nal-D-Cys-Pal-D-Trp-Lys-Thr-Cys-2R,3R-(2-hydroxymethyl)-3-hydroxy)propylamid;
(H)(4-(2-Hydroxyethyl)-1-piperazinylacetyl)-β-Nal-D-Cys-Pal-D-Trp-Lys-Thr-Cys-2R,3R-(2-hydroxymethyl)-3-hydroxy)propylamid;
(H)(4-(2-Hydroxyethyl)-1-piperizinethansulfonyl)-β-Nal-D-Cys-Pal-D-Trp-Lys-Thr-Cys-2R,3R-(2-hydroxymethyl)-3-hydroxy)propylamid;
H₂-Phe-D-Gys-Tyr-D-Trp-Lys-Val-Cys-2R,3R-(2-Hydroxymethyl)-3-hydroxy)propylamid;
(H)(CH₃CO)Phe-D-Cys-Tyr-D-Trp-Lys-Val-Cys-2R,3R-(2-Hydroxymethyl)-3-hydroxy)propylamid;
(H)(4-f2-Hydroxyethyl)-1-piperazinylacetyl)Phe-D-Cys-Tyr-D-Trp-Lys-Val-Cys-2R,3R-(2-hydroxymethyl)-3-hydroxy)-propylamid;
(H) (4-(2-Hydroxyethyl)-1-piperizinethansulfonyl)-Phe-D-Cys-Tyr-D-Trp-Lys-Val-Cys-2R,3R-(2-hydroxymethyl)-3-hydroxy)propylamid;
H₂-Phe-D-Cys-Pal-D-Trp-Lys-Val-Cys-2R,3R-(2-hydroxymethyl)-3-hydroxy)propylamid;
H(CH₃CO)Phe-D-Cys-Pal-D-Trp-Lys-Val-Cys-2R,3R-(2-Hydroxymethyl)-3-hydroxy)propylamid;
(H)(4-(2-Hydroxyethyl)-1-piperazinylacetyl)Phe-D-Cys-Pal-D-Trp-Lys-Val-Cys-2R,3R-(2-hydroxymethyl)-3-hydroxy)-propylamid;
(H)(4-(2-Hydroxyethyl)-1-piperizinethansulfonyl)Phe-D-Cys-Pal-D-Trp-Lys-Val-Cys-2R,3R-(2-hydroxymethyl)-3-hydroxy)propylamid;
H₂-Phe-D-Cys-Tyr-D-Trp-Lys-Thr-Cys-2R,3R-(2-hydroxymethyl)-3-hydroxy)propylamid;
(H) (CH₃CO) Phe-D-Cys-Tyr-D-Trp-Lys-Thr-Cys-2R,3R-(2-hydroxymethyl)-3-hydroxy)propylamid;
(H) (4-(2-Hydroxyethyl)-1-piperazinylacetyl)Phe-D-Cys-Tyr-D-Trp-Lys-Thr-Cys-2R,3R-(2-hydroxymethyl)-3-hydroxy)-propylamid;
(H) (4-(2-Hydroxyethyl)-1-piperizinethansulfonyl)Phe-D-Cys-Tyr-D-Trp-Lys-Thr-Cys-2R,3R-(2-hydroxymethyl)-3-hydroxy)propylamid;
H₂-Phe-D-Cys-Pal-D-Trp-Lys-Thr-Cys-2R,3R-(2-Hydroxymethyl)-3-hydroxy)propylamid;
(H) (CH₃CO)Phe-D-Cys-Pal-D-Trp-Lys-Thr-Cys-2R,3R-(2-Hydroxymethyl)-3-hydroxy)propylamid;
(H)(4-(2-Hydroxyethyl)-1-piperazinylacetyl)Phe-D-Cys-Pal-D-Trp-Lys-Thr-Cys-2R,3R-(2-hydroxymethyl)-3-hydroxy)-propylamid;
(H)(4-(2-Hydroxyethyl)-1-piperizinethansulfonyl)-Phe-D-Cys-Pal-D-Trp-Lys-Thr-Cys-2R,3R-(2-hydroxymethyl)-3-hydroxy)propylamid;
H₂-β-Nal-D-Cys-Tyr-D-Trp-Lys-Val-Cys-2R-(2-Naphthyl)-ethylamid;
(H) (CH₃CO)-β-Nal-D-Cys-Tyr-D-Trp-Lys-Val-Cys-2R-(2-Naphthyl)ethylamid;
(H) (4-(2-Hydroxyethyl)-1-piperazinylacetyl)-β-Nal-D-Cys-Tyr-D-Trp-Lys-Val-Cys-2R-(2-naphthyl)ethylamid;
(H) (4-(2-Hydroxyethyl)-1-piperizinethansulfonyl)-β-Nal-D-Cys-Tyr-D-Trp-Lys-Val-Cys-2R-(2-naphthyl)ethylamid;
H₂-β-Nal-D-Cys-Pal-D-Trp-Lys-Val-Cys-2R-(2-Naphthyl)ethylamid;
(H) (CH₃CO)-β-Nal-D-Cys-Pal-D-Trp-Lys-Val-Cys-2R-(2-Naphthyl)ethylamid;
(H)(4-(2-Hydroxyethyl)-1-piperazinylacetyl)-β-Nal-D-Cys-Pal-D-Trp-Lys-Val-Cys-2R-(2-naphthyl)ethylamid;
(H)(4-(2-Hydroxyethyl)-1-piperizinethansulfonyl)-β-Nal-D-Cys-Pal-D-Trp-Lys-Val-Cys-2R-(2-naphthyl)ethylamid;
H₂-β-Nal-D-Cys-Tyr-D-Trp-Lys-Thr-Cys-2R-(2-Naphthyl)ethylamid;
(H)(CH₃CO)-β-Nal-D-Cys-Tyr-D-Trp-Lys-Thr-Cys-2R-(2-Naphthyl)ethylamid;
(H) (4-(2-Hydroxyethyl)-1-piperazinylacetyl)-β-Nal-D-Cys-Tyr-D-Trp-Lys-Thr-Cys-2R-(2-naphthyl)ethylamid;
(H) (4-(2-Hydroxyethyl)-1-piperizinethansulfonyl)-β-Nal-D-Cys-Tyr-D-Trp-Lys-Thr-Cys-2R-(2-naphthyl)ethylamid;
H₂-β-Nal-D-Cys-Pal-D-Trp-Lys-Thr-Cys-2R-(2-Naphthyl)ethylamid;
(H)(CH₃CO)-β-Nal-D-Cys-Pal-D-Trp-Lys-Thr-Cys-2R-(2-Naphthyl)ethylamid;
(H) (4-(2-Hydroxyethyl)-1-piperazinylacetyl)-β-Nal-D-Cys-Pal-D-Trp-Lys-Thr-Cys-2R-(2-naphthyl)ethylamid;
(H) (4-{2-Hydroxyethyl)-1-piperizinethansulfonyl)-β-Nal-D-Cys-Pal-D-Trp-Lys-Thr-Cys-2R-(2-naphthyl)ethylamid;
H₂-Phe-D-Cys-Tyr-D-Trp-Lys-Val-Cys-2R-(2-Naphthyl)ethylamid;
(H) (CH₃CO) Phe-D-Cys-Tyr-D-Trp-Lys-Val-Cys-2R-(2-Naphthyl)ethylamid;
(H) (4-(2-Hydroxyethyl)-1-piperazinylacetyl)Phe-D-Cys-Tyr-D-Trp-Lys-Val-Cys-2R-(2-naphthyl)ethylamid;
(H)(4-(2-Hydroxyethyl)-1-piperizinethansulfonyl)Phe-D-Cys-Tyr-D-Trp-Lys-Val-Cys-2R-(2-naphthyl)ethylamid;
H₂-Phe-D-Cys-Pal-D-Trp-Lys-Val-Cys-2R-(2-Naphthyl)-ethylamid;
(H) (CH₃CO)Phe-Cys-Pal-D-Trp-Lys-Val-Cys-2R-(2-Naphthyl)-ethylamid;
(H)(4-(2-Hydroxyethyl)-1-piperazinylacetyl)Phe-D-Cys-Pal-D-Trp-Lys-Val-Cys-2R-(2-naphthyl)ethylamid;
(H)(4-(2-Hydroxyethyl)-1-piperizinethansulfonyl)-Phe-D-Cys-Pal-D-Trp-Lys-Val-Cys-2R-(2-naphthyl)ethylamid;
H₂-Phe-D-Cys-Tyr-D-Trp-Lys-Thr-Cys-2R-(2-Naphthyl)-ethylamid;
(H)(CH₃CO)Phe-D-Cys-Tyr-D-Trp-Lys-Thr-Cys-2R-(2-Naphthyl)ethylamid;
(H)(4-(2-Hydroxyethyl)-1-piperazinylacetyl)Phe-D-Cys-Tyr-D-Trp-Lys-Thr-Cys-2R-(2-naphthyl)ethylamid;
(H)(4-(2-Hydroxyethyl)-1-piperizinethansulfonyl)-Phe-D-Cys-Tyr-D-Trp-Lys-Thr-Cys-2R-(2-naphthyl)ethylamid;
H₂-Phe-D-Cys-Pal-D-Trp-Lys-Thr-Cys-2R-(2-Naphthyl)-ethylamid;
(H) (CH₃CO)Phe-Cys-Pal-D-Trp-Lys-Thr-Cys-2R-(2-Naphthyl)-ethylamid;
(H) (4-(2-Hydroxyethyl)-1-piperazinylacetyl)Phe-D-Cys-Pal-D-Trp-Lys-Thr-Cys-2R-(2-naphthyl)ethylamid;
(H) (4-(2-Hydroxyethyl)-1-piperizinethansulfonyl)Phe-D-Cys-Pal-D-Trp-Lys-Thr-Cys-2R-(2-naphthyl)ethylamid;
H₂-β-Nal-D-Cys-Tyr-D-Trp-Lys-Abu-Cys-2R-(2-Naphthyl)ethylamid;
H₂-Phe-D-Cys-Tyr-D-Trp-Lys-Abu-Cys-2R-(2-Naphthyl)ethylamid;
H₂-β-Nal-D-Cys-Tyr-D-Trp-Lys-Abu-Cys-2R,3R-(2-hydroxymethyl)-3-hydroxy)propylamid oder H₂-Phe-D-Cys-Tyr-D-Trp-Lys-Abu-Cys-2R,3R-(2-Hydroxymethyl)-3-hydroxy)propylamid oder ein pharmazeutisch annehmbares Salz davon.

19. Verbindung nach Anspruch 1, bei der A² eine aromatische D-Aminosäure oder eine aliphatische D-Aminosäure ist, A⁷ eine aromatische Aminosäure oder eine aliphatische Aminosäure ist und A⁴ D-Trp ist.

20. Verbindung nach Anspruch 19, bei der A¹ eine L-Aminosäure ist und A² eine aromatische D-Aminosäure ist.

21. Verbindung nach Anspruch 20, bei der A₁, A₃ und A₇ unabhängig β-Nal, o-X-Phe (wobei X H, OH, CH₃, Halogen, OCH₃, NH₂, CN oder NO₂ ist), p-X-Phe (wobei X H, OH, CH₃, Halogen, OCH₃, NH₂, CN oder NO₂ ist), m-X-Phe (wobei X H, OH, CH₃, Halogen, OCH₃, NH₂, CN oder NO₂ ist), F₅-Phe, Trp, Dip, 2-Pal, Tyr(Bzl), His, Igl, Tyr(I), Bta, Bip, Npa oder Pal sind; A² D-β-Nal, D-o-X-Phe (wobei X H, OH CH₃, Halogen, OCH₃, NH₂, CN oder NO₂ ist), D-p-X-Phe (wobei X H, OH CH₃, Halogen, OCH₃, NH₂, CN oder NO₂ ist), D-m-X-Phe (wobei X H, OH CH₃, Halogen, OCH₃, NH₂, CN oder NO₂ ist), D-F₅-Phe, D-Trp, D-Dip, D-2-Pal, D-Tyr(Bzl), D-His, D-Igl, D-Tyr(I), D-Bta, D-Bip, D-Npa oder D-Pal ist; A⁶ Thr, Ser, Tle, Thr(Bzl), Abu, Ala, Ile, Leu, Gly, Nle, p-Ala, Gaba oder Val ist; und A⁸ das D- oder L-Isomer von Thr, Dip, F₅-Phe, p-X-Phe (wobei X H, OH, CH₃, Halogen, OCH₃, NH₂, CN oder NO₂ ist), o-X-Phe (wobei X H, OH, CH₃, Halogen, OCH₃, NH₂, CN oder NO₂ ist), m-X-Phe (wobei X H, OH, CH₃, Halogen, OCH₃, NH₂, CN oder NO₂ ist), Igl, Tyr (Bzl) oder β-Nal ist.

22. Verbindung nach Anspruch 21, bei der A¹ β-Nal oder Phe ist, A² D-Cpa oder D-Phe ist; A³ Phe oder Tyr ist; A⁶ Abu, Thr oder Val ist; A⁷ Phe ist und A⁸ Thr ist; R₁ H, CH₃CO, 4-(2-Hydroxyethyl)-1-piperazinylacetyl oder 4-(2-Hydroxyethyl)-1-piperizinethansulfonyl ist; R₂ H ist und R₃ NH₂ ist; oder die folgende Formel hat:
H₂-Phe-D-Phe-Tyr-D-Trp-Lys-Thr-Phe-Thr-NH₂;
H₂-Phe-D-Phe-Tyr-D-Trp-Lys-Val-Phe-Thr-NH₂;
H₂-Phe-D-Cpa-Tyr-D-Trp-Lys-Val-Phe-Thr-NH₂;
H₂-β-Nal-D-Cpa-Tyr-D-Trp-Lys-Val-Phe-Thr-NH₂;
(H) (CH₃CO) -β Nal-D-Cpa-Tyr-D-Trp-Lys-Val-Phe-Thr-NH₂;
(H) (4-(2-Hydroxyethyl)-1-piperazinylacetyl)-β-Nal-D-Cpa-Tyr-D-Trp-Lys-Val-Phe-Thr-NH₂;
(H) (4-(2-Hydroxyethyl)-1-piperizinethansulfonyl)-β-Nal-D-Cpa-Tyr-D-Trp-Lys-Val-Phe-Thr-NH₂;
H₂-β-Nal-D-Cpa-Pal-D-Trp-Lys-Val-Phe-Thr-NH₂;
(H) (CH₃CO)-β-Nal-D-Cpa-Pal-D-Trp-Lys-Val-Phe-Thr-NH₂;
(H)(4-(2-Hydroxyethyl)-1-piperazinylacetyl)-β-Nal-D-Cpa-Pal-D-Trp-Lys-Val-Phe-Thr-NH₂;
(H)(4-(2-Hydroxyethyl)-1-piperazinylacetyl)-β-Nal-D-Cpa-Pal-D-Trp-Lys-Val-Phe-Thr-NH₂;
H₂-β-Nal-D-Cpa-Tyr-D-Trp-Lys-Thr-Phe-Thr-NH₂;
(H)(CH₃CO)-β-Nal-D-Cpa-Tyr-D-Trp-Lys-Thr-Phe-Thr-NH₂;
(H)(4-(2-Hydroxyethyl)-1-piperazinylacetyl)-β-Nal-D-Cpa-Tyr-D-Trp-Lys-Thr-Phe-Thr-NH₂;
(H) (4-(2-Hydroxyethyl)-1-piperizinethansulfonyl)-β-Nal-D-Cpa-Tyr-D-Trp-Lys-Thr-Phe-Thr-NH₂;
H₂-β-Nal-D-Cpa-Pal-D-Trp-Lys-Thr-Phe-Thr-NH₂;
(H) (CH₃CO)-β-Nal-D-Cpa-Pal-D-Trp-Lys-Thr-Phe-Thr-NH₂;
(H) (4-(2-Hydroxyethyl)-1-piperazinylacetyl)-β-Nal-D-Cpa-Pal-D-Trp-Lys-Thr-Phe-Thr-NH₂;
(H)(4-(2-Hydroxyethyl)-1-piperizinethansulfonyl)-β-Nal-D-Cpa-Pal-D-Trp-Lys-Thr-Phe-Thr-NH₂;
H₂-β-Nal-D-Cpa-Tyr-D-Trp-Lys-Val-Phe-β-Nal-NH₂;
(H) (CH₃CO)-β-Nal-D-Cpa-Tyr-D-Trp-Lys-Val-Phe-β-Nal-NH₂;
(H)(4-(2-Hydroxyethyl)-1-piperazinylacetyl)-β-Nal-D-Cpa-Tyr-D-Trp-Lys-Val-Phe-β-Nal-NH₂ oder
(H) (4-(2-Hydroxyethyl)-1-piperizinethansulfonyl)-β-Nal-D-Cpa-Tyr-D-Trp-Lys-Val-Phe-β-Nal-NH₂;
H₂-β-Nal-D-Cpa-Tyr-D-Trp-Lys-Val-Phe-β-Nal-NH₂- oder
H₂-β-Nal-D-Cpa-Tyr-D-Trp-Lys-Val-Phe-Thr-NH₂ oder ein pharmazeutisch annehmbares Salz davon.

23. Verbindung nach Anspruch 19, bei der A¹ eine D-Aminosäure ist und A² eine aromatische D-Aminosäure ist.

24. Verbindung nach Anspruch 23, bei der A¹ und A² unabhängig D-β-Nal, D-o-X-Phe (wobei X H, OH, CH₃, Halogen, OCH₃, NH₂, CN oder NO₂ ist) , D-p-X-Phe (wobei X H, OH CH₃, Halogen, OCH₃, NH₂, CN oder NO₂ ist), D-m-X-Phe (wobei X H, OH, CH₃, Halogen, OCH₃, NH₂, CN oder NO₂ ist), D-F₅-Phe, D-Trp, D-Dip, D-2-Pal, D-Tyr(Bzl), D-His, D-Igl, D-Tyr(I), D-Bta, D-Bip, D-Npa oder D-Pal sind; A³ und A⁷ unabhängig β-Nal, o-X-Phe (wobei X H, OH, CH₃, Halogen, OCH₃, NH₂, CN oder NO₂ ist), p-X-Phe (wobei X H, OH CH₃, Halogen, OCH₃, NH₂, CN oder NO₂ ist), m-X-Phe (wobei X H, OH, CH₃, Halogen, OCH₃, NH₂, CN oder NO₂ ist), F₅ - Phe , Trp, Dip, 2-Pal, His, Igl, Tyr(I), Bta, Bip, Npa oder Pal sind; A⁶ Thr, Ser, Tle, Thr(Bzl), Abu, Ala, Ile, Leu, Gly, Nle, β-Ala, Gaba oder Val ist und A⁸ das D- oder L-Isomer von Thr, Dip, F₅-Phe, p-XPhe (wobei X H, OH, CH₃, Halogen, OCH₃, NH₂, CN oder NO₂ ist), o-X-Phe (wobei X H, OH, CH₃, Halogen, OCH₃, NH₂, CN oder NO₂ ist), m-X-Phe (wobei X H, OH, CH₃, Halogen, OCH₃, NH₂, CN oder NO₂ ist), Igl, Tyr(Bzl) oder β-Nal ist.

25. Verbindung nach Anspruch 24, bei der A¹ D-β-Nal oder D-Phe ist, A² D-Cpa oder D-Phe ist; A³ Phe oder Tyr ist; A⁶ Thr oder Val ist; A⁷ Phe ist und A⁸ Thr ist; R₁ H, CH₃CO, 4-(2-Hydroxyethyl)-1-piperazinylacetyl oder 4-(2-Hydroxyethyl)-1-piperizinethansulfonyl ist; R₂ H ist und R₃ NH₂ ist oder die folgende Formel hat:
H₂-D-β-Nal-D-Cpa-Phe-D-Trp-Lys-Val-Phe-Thr-NH₂;
H₂-D-β-Nal-D-Phe-Tyr-D-Trp-Lys-Thr-Phe-Thr-NH₂;
H₂-D-Phe-D-Phe-Tyr-D-Trp-Lys-Val-Phe-Thr-NH₂;
H₂-D-β-Nal-D-Cpa-Tyr-D-Trp-Lys-Val-Phe-Thr-NH₂ oder
H₂-D-β-Nal-D-Cpa-Tyr-D-Trp-Lys-Val-Phe-β-Nal-NH₂
oder ein pharmazeutisch annehmbares Salz davon.

## Revendications

1. Composé de formule : 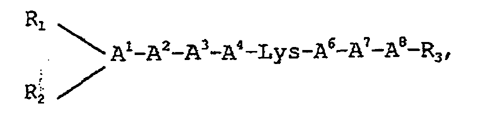 dans laquelle :
- A¹ est un isomère D ou L d'un acide aminé aromatique, ou est supprimé ;
- A² est un isomère D choisi dans le groupe constitué par Cys, Pen, un acide aminé aromatique ou un acide aminé aliphatique ;
- A³ est un acide aminé aromatique ;
- A⁴ représente Trp ou D-Trp ;
- A⁶ représente Thr, Thr(Bzl), Gly, Ser, un Eaa ou un acide aminé aliphatique ;
- A⁷ représente Cys, Pen ou un acide aminé aromatique ou aliphatique ;
- A⁸ est un isomère D ou L choisi dans le groupe constitué par Thr, Ser, un acide aminé aromatique ou un acide aminé aliphatique ;
- R₁ et R₂ représentent chacun indépendamment H ou alkyle inférieur, aryle, aryl alkyle inférieur, hétérocycle, hétérocycle alkyle inférieur, E₁SO₂ ou E₁CO (où E₁ représente aryle, aryl alkyle inférieur, hétérocycle ou hétérocycle alkyle inférieur) substitué ou non substitué, ledit substituant étant halo, alkyle inférieur, hydroxy, halo alkyle inférieur ou hydroxy alkyle inférieur ; et
- R₃ représente OH, NH₂, alcoxy en C₁₋₁₂ ou NH-Y-CH₂-Z, où Y est une fraction hydrocarbonée en C₁₋₁₂ et Z représente H, OH, CO₂H ou CONH₂, ou R₃, conjointement avec le groupe carbonyle de A⁸ attaché à celui-ci, sont réduits pour former H, alkyle inférieur ou hydroxy alkyle inférieur ; à la condition que si A² représente D-Cys ou D-Pen, et A⁷ représente Cys ou Pen, alors une liaison disulfure lie les chaînes latérales de A² et A⁷, et si A¹ représente D-Phe ou p-NO₂-Phe ; A² représente D-Cys ; A³ représente Phe ou Tyr ; A⁶ représente Thr ou Val; et A⁷ représente Cys ; alors A⁸ représente β-Nal.

2. Composé selon la revendication 1, dans lequel A² représente D-Cys, A⁷ représente Cys et A⁴ représente D-Trp.

3. Composé selon la revendication 2, dans lequel A¹ représente un L-amino-acide aromatique.

4. Composé selon la revendication 3, dans lequel A¹ et A³, indépendamment, représentent β-Nal, o-X-Phe (où X représente H, OH, CH₃, halo, OCH₃, NH₂, CN ou NO₂) , p-X-Phe (où X représente H, OH, CH₃, halo, OCH₃, NH₂, CN ou NO₂), m-X-Phe (où X représente H, OH, CH₃, halo, OCH₃, NH₂, CN ou NO₂), F₅-Phe, Trp, Dip, 2-Pal, Tyr(Bzl), His, Igl, Tyr(I), Bta, Bip, Npa ou Pal ; A⁶ représente Thr, Ser, Tle, Thr(Bzl), Abu, Ala, Ile, Leu, Gly, Nle, β-Ala, Gaba ou Val ; et A⁸ représente l'isomère D ou L de Thr, Dip, F₅₋Phe, p-X-Phe (où X représente H, OH, CH₃, halo, OCH₃, NH₂, CN ou NO₂), o-X-Phe (où X représente H, OH, CH₃, halo, OCH₃, NH₂, CN ou NO₂), m-X-Phe (où X représente H, OH, CH₃, halo, OCH₃, NH₂, CN ou NO₂), Igl, Tyr (Bzl) ou βNal.

5. Composé selon la revendication 4, dans lequel A¹ représente β-Nal, Npa, Igl, Phe, p-F-Phe, Trp, p-Cl-Phe ou p-CN-Phe ; A³ représente Tyr, Tyr(I) ou Pal; A⁶ représente Val, Tle, Nle, Ile ou Leu ; A⁸ représente p-F-Phe, β-Nal, Tyr, Dip, p-Cl-Phe, Igl ou p-CN-Phe ; R₁ représente H, CH₃CO, 4-(2-hydroxyéthyl)-1-pipérazinylacétyle, ou 4-(2-hydroxyéthyl)-1-pipérizineéthanesulfonyle ; R₂ représente H ; et R₃ représente NH₂.

6. Composé selon la revendication 4, de formule :
H₂-β-Nal-D-Cys-Tyr-D-Trp-Lys-Val-Cys-β-Nal-NH₂ ;
(H) (CH₃CO)-β-Nal-D-Cys-Tyr-D-Trp-Lys-Val-Cys-β-Nal-NH₂(V);
(H)-(4-(2-hydroxyéthyl)-1-pipérazinylacétyl)-β-Nal-D-Cys-Tyr-D-Trp-Lys-Val-Cys-β-Nal-NH₂ ;
(H)-(4-(2-hydroxyéthyl)-1-pipérizineéthanesulfonyl)-β-Nal-D-Cys-Tyr-D-Trp-Lys-Val-Cys-β-Nal-NH₂ ;
H₂-β-Nal-D-Cys-Pal-D-Trp-Lys-Val-Cys-β-Nal-NH₂ ;
(H) (CH₃CO)-β-Nal-D-Cys-Pal-D-Trp-Lys-Val-Cys-β-Nal-NH₂ ;
(H)-(4-(2-hydroxyéthyl)-1-pipérazinylacétyl)-β-Nal-D-Cys-Pal-D-Trp-Lys-Val-Cys-β-Nal-NH₂ ;
(H)-(4-(2-hydroxyéthyl)-1-pipérizineéthanesulfonyl)-β-Nal-D-Cys-Pal-D-Trp-Lys-Val-Cys-β-Nal-NH₂ ;
H₂-β-Nal-D-Cys-Tyr-D-Trp-Lys-Val-Cys-Thr-NH₂ ;
(H) (CH₃CO)-β-Nal-D-Cys-Tyr-D-Trp-Lys-Val-Cys-Thr-NH₂ ;
(H) (4-(2-hydroxyéthyl)-1-pipérazinylacétyl)-β-Nal-D-Cys-Tyr-D-Trp-Lys-Val-Cys-Thr-NH₂ ;
(H) (4-(2-hydroxyéthyl)-l-pipérizineéthanesulfonyl)-p-Nal-D-Cys-Tyr-D-Trp-Lys-Val-Cys-Thr-NH₂ ;
H₂-β-Nal-D-Cys-Pal-D-Trp-Lys-Val-Cys-Thr-NH₂ ;
(H) (CH₃CO)-β-Nal-D-Cys-Pal-D-Trp-Lys-Val-Cys-Thr-NH₂ ;
(H) (4-(2-hydroxyéthyl)-1-pipérazinylacétyl)-β-Nal-D-Cys-Pal-D-Trp-Lys-Val-Cys-Thr-NH₂ ;
(H) (4-(2-hydroxyéthyl)-1-pipérizineéthanesulfonyl)-β-Nal-D-Cys-Pal-D-Trp-Lys-Val-Cys-Thr-NH₂ ;
H₂-Phe-D-Cys-Tyr-D-Trp-Lys-Val-Cys-β-Nal-NH₂;
(H) (CH₃CO)Phe-D-Cys-Tyr-D-Trp-Lys-Val-Cys-β-Nal-NH₂;
(H) (4-(2-hydroxyéthyl)-1-pipérazinylacétyl)-Phe-D-Cys-Tyr-D-Trp-Lys-Val-Cys-β-Nal-NH₂;
(H) (4-(2-hydroxyéthyl)-1-pipérizineéthanesulfonyl)-Phe-D-Cys-Tyr-D-Trp-Lys-Val-Cys-β-Nal-NH₂;
H₂-Phe-D-Cys-Pal-D-Trp-Lys-Val-Cys-β-Nal-NH₂ ;
(H) (CH₃CO)Phe-D-Cys-Pal-D-Trp-Lys-Val-Cys-β-Nal-NH₂ ;
(H) (4-(2-hydroxyéthyl)-l-pipérazinylacétyl)-Phe-D-Cys-Pal-D-Trp-Lys-Val-Cys-β-Nal-NH2 ;
(H) (4-(2-hydroxyéthyl)-1-pipérizineéthanesulfonyl)-Phe-D-Cys-Pal-D-Trp-Lys-Val-Cys-fi-Nal-NH₂
H₂-Phe-D-Cys-Pal-D-Trp-Lys-Val-Cys-Thr-NH₂ ;
(H) (CH₃CO)-Phe-D-Cys-Pal-D-Trp-Lys-Val-Cys-Thr-NH₂ ;
(H) (4-(2-hydroxyéthyl)-1-pipérazinylacétyl)-Phe-D-Cys-Pal-D-Trp-Lys-Val-Cys-Thr-NH₂;
(H) (4-(2-hydroxyéthyl)-1-pipérizineéthanesulfonyl)-Phe-D-Cys-Pal-D-Trp-Lys-Val-Cys-Thr-NH₂ ;
H2-β-Nal-D-Cys-Tyr-D-Trp-Lys-Thr-Cys-β-Nal-NH₂ ;
(H) (CH₃CO)-β-Nal-D-Cys-Pal-D-Trp-Lys-Thr-Cys-β-Nal-NH₂ ;
(H) (4-(2-hydroxyéthyl)-1-pipérazinylacétyl)-β-Nal-D-Cys-Tyr-D-Trp-Lys-Thr-Cys-β-Nal-NH₂ ;
(H) (4-(2-hydroxyéthyl)-1-pipérizineéthanesulfonyl)-β-Nal-D-Cys-Tyr-D-Trp-Lys-Thr-Cys-β-Nal-NH₂ ;
H₂-β-Nal-D-Cys-Pal-D-Trp-Lys-Thr-Cys-β-Nal-NH₂ ;
(H) (CH₃CO)-β-Nal-D-Cys-Pal-D-Trp-Lys-Thr-Cys-β-Nal-NH₂ ;
(H) (4-(2-hydroxyéthyl)-1-pipérazinylacétyl)-β-Nal-D-Cys-Pal-D-Trp-Lys-Thr-Cys-β-Nal-NH₂ ;
(H) (4-(2-hydroxyéthyl)-1-pipérizineéthanesulfonyl)-β-Nal-D-Cys-Pal-D-Trp-Lys-Thr-Cys-β-Nal-NH₂ ;
H₂-β-Nal-D-Cys-Tyr-D-Trp-Lys-Thr-Cys-Thr-NH₂ ;
H(CH₃CO)-β-Nal-D-Cys-Tyr-D-Trp-Lys-Thr-Cys-Thr-NH₂;
(H) (4-(2-hydroxyéthyl)-1-pipérazinylacétyl)-p-Nal-D-Cys-Tyr-D-Trp-Lys-Thr-Cys-Thr-NH₂ ;
(H) (4-(2-hydroxyéthyl)-1-pipérizineéthanesulfonyl)-β-Nal-D-Cys-Tyr-D-Trp-Lys-Thr-Cys-Thr-NH₂ ;
H₂-β-Nal-D-Cys-Pal-D-Trp-Lys-Thr-Cys-Thr-NH₂ ;
(H) (CH₃CO)-β-Nal-D-Cys-Pal-D-Trp-Lys-Thr-Cys-Thr-NH₂;
(H) (4-(2-hydroxyéthyl)-1-pipérazinylacétyl)-β-Nal-D-Cys-Pal-D-Trp-Lys-Thr-Cys-Thr-NH₂ ;
(H) (4-(2-hydroxyéthyl)-1-pipérizineéthanesulfonyl)-β-Nal-D-Cys-Pal-D-Trp-Lys-Thr-Cys-Thr-NH₂ ;
H2-Phe-D-Cys-Tyr-D-Trp-Lys-Thr-Cys-!3-Nal-NH2 ;
(H) (CH₃CO)Phe-D-Cys-Tyr-D-Trp-Lys-Thr-Cys-β-Nal-NH₂ ;
(H) (4-(2-hydroxyéthyl)-1-pipérazinylacétyl)Phe-D-Cys-Tyr-D-Trp-Lys-Thr-Cys-β-Nal-NH₂;
(H) (4-(2-hydroxyéthyl)-1-pipérizineéthanesulfonyl)-Phe-D-Cys-Tyr-D-Trp-Lys-Thr-Cys-β-Nal-NH₂ ;
H₂-Phe-D-Cys-Pal-D-Trp-Lys-Thr-Cys-β-Nal-NH₂ ;
(H) (CH₃CO)Phe-D-Cys-Pal-D-Trp-Lys-Thr-Cys-β-Nal-NH₂ ;
(H) (4-(2-hydroxyéthyl)-1-pipérazinylacétyl)Phe-D-Cys-Pal-D-Trp-Lys-Thr-Cys-β-Nal-NH₂ ;
(H) (4-(2-hydroxyéthyl)-1-pipérizineéthanesulfonyl)Phe-D-Cys-Pal-D-Trp-Lys-Thr-Cys-β-Nal-NH₂ ;
H₂-Phe-D-Cys-Tyr-D-Trp-Lys-Thr-Cys-Thr-NH₂;
(H) (CH₃CO)Phe-D-Cys-Tyr-D-Trp-Lys-Thr-Cys-Thr-NH₂ ;
(H) (4-(2-hydroxyéthyl)-1-pipérazinylacétyl)Phe-D-Cys-Tyr-D-Trp-Lys-Thr-Cys-Thr-NH₂ ;
(H) (4-(2-hydroxyéthyl)-1-pipérizineéthanesulfonyl)Phe-D-Cys-Tyr-D-Trp-Lys-Thr-Cys-Thr-NH₂;
H₂-Phe-D-Cys-Pal-D-Trp-Lys-Thr-Cys-Thr-NH₂;
(H) (CH₃CO)-Phe-D-Cys-Pal-D-Trp-Lys-Thr-Cys-Thr-NH₂ ;
(H) (4-(2-hydroxyéthyl)-1-pipérazinylacétyl)Phe-D-Cys-Pal-D-Trp-Lys-Thr-Cys-Thr-NH₂;
(H) (4-(2-hydroxyéthyl)-1-pipérizineéthanesulfonyl)-Phe-D-Cys-Pal-D-Trp-Lys-Thr-Cys-Thr-NH₂;
H₂-β-Nal-D-Cys-Tyr-D-Trp-Lys-Abu-Cys-β-Nal-NH₂ ;
H₂-Phe-D-Cys-Tyr-D-Trp-Lys-Abu-Cys-β-Nal-NH₂ ;
H₂-β-Nal-D-Cys-Pal-D-Trp-Lys-Abu-Cys-β-Nal-NH₂ ;
H₂-Phe-D-Cys-Pal-D-Trp-Lys-Abu-Cys-β-Nal-NH₂;
H₂-β-Nal-D-Cys-Tyr-D-Trp-Lys-Abu-Cys-Thr-NH₂;
H₂-Phe-D-Pen-Tyr-D-Trp-Lys-Val-Pen-β-Nal-NH₂; or
H₂,-Phe-D-Pen-Pal-D-Trp-Lys-Thr-Pen-Thr-NH₂;
H₂-Dip-D-Cys-Pal-D-Trp-Lys-Val-Cys-Dip-NH₂;
H₂-F₅-Phe-D-Cys-His-D-Trp-Lys-Val-Cys-F₅-Phe-NH₂;
H₂-Dip-D-Cys-Pal-D-Trp-Lys-Val-Cys-β-Nal-NH₂;
H₂-m-F-Phe-D-Cys-Pal-D-Trp-Lys-Val-Cys-m-F-Phe-NH₂;
H₂-o-F-Phe-D-Cys-Pal-D-Trp-Lys-Val-Cys-o-F-Phe-NH₂;
H₂-p-F-Phe-D-Cys-Pal-D-Trp-Lys-Val-Cys-F₅-Phe-NH₂;
H₂-F₅-Phe-D-Cys-PaL-D-Trp-Lys-Val-Cys-F₅-Phe-NH₂;
H₂-F₅-Phe-D-Cys-2-Pal-D-Trp-Lys-Val-Cys-F₅-Phe-NH₂;
H₂-β-Nal-D-Cys-His-D-Trp-Lys-Val-Cys-D-Dip-NH₂;
H₂-Dip-D-Cys-His-D-Trp-Lys-Val-Cys-β-Nal-NH₂;
H₂-Dip-D-Cys-His-D-Trp-Lys-Val-Cys-Dip-NH₂;
H₂-β-Nal-D-Cys-His-D-Trp-Lys-Val-Cys-β-Nal-NH₂;
H₂-Trp-D-Cys-Tyr-D-Trp-Lys-Val-Cys-D-β-Nal-NH₂;
H₂-β-Nal-D-Cys-Tyr-D-Trp-Lys-Val-Cys-D-β-Nal-NH₂;
H₂-β-Nal-D-Cys-Pal-D-Trp-Lys-Val-Cys-D-p-F-Phe-NH₂:
H₂-β-Nal-D-Cys-Pal-D-Trp-Lys-Tle-Cys-β-Nal-NH₂;
H₂-p-F-Phe-D-Cys-Pal-D-Trp-Lys-Val-Cys-β-Nal-NH₂;
H₂-β-Nal-D-Cys-Pal-D-Trp-Lys-Nle-Cys-β-Nal-NH₂;
H₂-β-Nal-D-Cys-Pal-D-Trp-Lys-Ile-Cys-β-Nal-NH₂;
H₂-β-Nal-D-Cys-Pal-D-Trp-Lys-Gly-Cys-β-Nal-NH₂;
H₂-β-Nal-D-Cys-Pal-D-Trp-Lys-Ala-Cys-β-Nal-NH₂;
H₂-β-Nal-D-Cys-Pal-D-Trp-Lys-Leu-Cys-β-Nal-NH₂;
H₂-Bip-D-Cys-Tyr-D-Trg-Lys-Ile-Cys-Bip-NH₂;
H₂-p-F-Phe-D-Cys-His-D-Trp Lys-Val-Cys-p-F-Phe-NH₂;
H₂-Npa-D-Cys-Pal-D-Trp-Lys-Val-Cys-Tyr-NH₂;
H₂-m-F-Phe-D-Cys-His-D-Trp-Lys-Val-Cys-m-F-Phe-NH₂;
H₂-o-F-Phe-D-Cys-His-D-Trp-Lys-Val-Cys-o-F-Phe-NH₂;
H₂-β-Nal-D-Cys-Pal-D-Trp-Lys-Val-Cys-Dip-NH₂;
H₂-Cpa-D-Cys-Pal-D-Trp-Lys-Val-Cys-Cpa-NH₂;
H₂-Igl-D-Cys-Pal-D-Trp-Lys-Val-Cys-Igl-NH₂;
H₂-β-Nal-D-Cys-Pal-D-Trp-Lys-Val-Cys-D-Dip-NH₂;
H₂-β-Nal-P-Cys-3-I-Tyr-D-Trp-Lys-Val-Cys-β-Nal-NH₂;
H₂-p-CN-Phe-D-Cys-Pal-D-Trp-Lys-Val-Cys-p-CN-Phe-NH₂;
H₂-β-Nal-D-Cys-Tyr-D-Trp-Lys-Val-Cys-D-Dip-NH₂;
H₂-β-Nal-D-Cys-Bta-B-Trp-Lys-Val-Cys-β-Nal-NH₂;
H₂-p-F-Phe-D-Cys-pal-D-Trp-Lys-Tle-Cys-β-Nal-NH₂;
H₂-Bpa-D-Cys-Pal-D-Trp-Lys-Val-Cys-Bpa-NH₂;
H₂-Iph-D-Cys-Pal-D-Trp-Lys-Val-Cys-Iph-NH₂;
H₂-Trp-D-Cys-Pal-D-Trp-Lys-Tle-Cys-β-Nal-NH₂;
H₂-p-Cl-Phe-D-Cys-Pal-D-Trp-Lys-Val-Cys-β-Nal-NH₂;
H₂-p-Cl-Phe-D-Cys-Pal-D-Trp-Lys-Tle-Cys-β-Nal-NH₂;
H₂-p-Cl-Phe-D-Cys-Pal-D-Trp-Lys-Tle-Cys-p-Cl-Phe-NH₂;
H₂-p-Cl-Phe-D-Cys-Pal-D-Trp-Lys-Cha-Cys-p-Cl-Phe-NN₂;
H₂-p-Cl-Phe-D-Cys-Tyr (I) -D-Trp-Lys-Val-Cys-p-Cl-Phe-NH₂: H₂-p-Cl-Phe-D-Cys-Tyr (I)-D-Trp-Lys-Val-Cys-β-Nal-NH₂: H₂-p-Cl-Phe-D-Cys-Tyr (I)-D-Trp-Lys-Tle-Cys-β-Nal-NH₂;
H₂-p-F-Phe-D-Cys-Tyr(I)-D-Trp-Lys-Val-Cys-β-Nal-NH₂;
H₂-p-F-Phe-D-Cys-Tyr (I) -D-Trp-Lys-Tle-Cys-β-Nal-NH₂;
H₂-β-Nal-D-Cys-Tyr-D-Trp-Lys-Abu-Cys-β-Nal-NH₂;
(H) (CH₃CO)-β-Nal-D-Cys-Tyr-D-Trp-Lys-Abu-Cys-β-Nal-NH₂; H₂-p-NO₂-Phe-D-Cys-Tyr-D-Trp-Lys-Abu-Cys-β-Nal-NH₂;
(H) (CH₃CO)-β-Nal-D-Cys-Tyr-D-Trp-Lys-Abu-Cys-P-Nal-NH₂; H₂-p-NO₂-Phe-D-Cys-Tyr (Bzl) -D-Trp-Lys-Thr (Bzl) -Cys-Nal-NH₂;
(H) (4-(2-hydroxyéthyl)-1-pipérazinylacétyl)-p-NO₂-Phe-D-Cys-Tyr(Bzl)-D-Trp-Lys-Thr(Bzl)-Cys-β-Nal-NH₂;
(H) (4-(2-hydroxyéthyl)-1-pipérazinylacétyl)-p-NO₂-Phe-D-Cys-Tyr-D-Trp-Lys-Thr-Cys-Tyr-NH₂;
H₂-p-NO₂-Phe-D-Cys-Tyr-D-Trp-Lys-Val-Cys-β-Nal-NH₂ ;
(H) (4-(2-hydroxyéthyl)-1-pipérazinylacétyl)-p-N02-Phe-D-Cys-Tyr-D-Trp-Lys-Val-Cys-β-Nal-NH₂;
(H) (4-(2-hydroxyéthyl)-1-pipérazinylacétyl)-β-Nal-Phe-D-Cys-Tyr-D-Trp-Lys-Val-Cys-β-Nal-NH₂;
H₂-β-Nal-D-Cys-Tyr(Bzl)-D-Trp-Lys-Thr(Bzl)-Cys-β-Nal-NH₂ ; ou
(H) (4-(2-hydroxyéthyl)-1-pipérazinylacétyl)-β-Nal-D-Cys-Tyr(Bzl)-D-Trp-Lys-Thr(Bzl)-Cys-Tyr(Bzl)-NH₂ ;
ou un sel pharmaceutiquement acceptable de ceux-ci.

7. Composé selon la revendication 2, dans lequel A¹ est un D-amino-acide aromatique.

8. Composé selon la revendication 7, dans lequel A¹ représente D-β-Nal, D-o-X-Phe (où X représente H, OH, CH₃, halo, OCH₃, NH₂, CN ou NO₂), D-p-X-Phe (où X représente H, OH, CH₃, halo, OCH₃, NH₂, CN ou NO₂), Dm-X-Phe (où X représente H, OH, CH₃, halo, OCH₃, NH₂, CN ou NO₂), D-F₅-Phe, D-Trp, D-Dip, D-2-Pal, D-Tyr(Bzl), D-His, D-Igl, DTyr(I), D-Bta, D-Bip, D-Npa ou D-Pal ; A³ représente β-Nal, o-X-Phe (où X représente H, OH, CH₃, halo, OCH₃, NH₂, CN ou NO₂), p-X-Phe (où X représente H, OH, CH₃, halo, OCH₃, NH₂, CN ou NO₂), m-X-Phe (où X représente H, OH, CH₃, halo, OCH₃, NH₂, CN ou NO₂), F₅-Phe, Trp, Dip, 2-Pal, Tyr(Bzl), His, Igl, Tyr (I), Bta, Bip, Npa ou Pal ; A⁶ représente Thr, Ser, Tle, Thr(Bzl), Abu, Ala, Ile, Leu, Gly, Nle, β-Ala, Gaba ou Val ; et A⁸ représente l'isomère D ou L de Thr, Dip, F₅-Phe, p-X-Phe (où X représente H, OH, CH₃, halo, OCH₃, NH₂, CN ou NO₂), o-X-Phe (où X représente H, OH, CH₃, halo, OCH₃, NH₂, CN ou NO₂), m-X-Phe (où X représente H, OH, CH₃, halo, OCH₃, NH₂, CN ou NO₂), Igl, Tyr (Bzl) ou β-Nal.

9. Composé selon la revendication 8, dans lequel A¹ représente D-β-Nal, D-Npa, D-Igl, D-Phe, D-p-F-Phe, D-Trp, D-p-Cl-Phe ou D-p-CN-Phe ; A³ représente Tyr, Tyr(I) ou Pal ; A⁶ représente Val, Tle, Nle, Ile ou Leu ; A⁸ représente p-F-Phe, β-Nal, Tyr, Dip, p-Cl-Phe, Igl ou p-CNPhe ; R₁ représente H, CH₃CO, 4-(2-hydroxyéthyl)-1-pipérazinylacétyle, ou 4-(2-hydroxyéthyl)-1-pipérizineéthanesulfonyl ; R₂ représente H ; et R₃ représente NH₂.

10. Composé selon la revendication 7, de formule :
H₂-D-Phe-D-Pen-Tyr-D-Trp-Lys-Val-Cys-Thr-NH₂;
H₂=D-β-Nal-D-Cys-Tyr-D-Trp-Lys-Val-Cys-Thr-NH₂;
H₂-D-β-Nal-D-Cys-Tyr-D-Trp-Lys-Val-Cys-β-Nal-NH₂;
H₂-D-β-Nal-D-Cys-Tyr-D-Trp-Lys-Thr-Cys-β-Nal-NH₂:
H₂-D-Phe-D-Cys-PaZ-D-Trp-Lys-Thr-Cys-Thr-NH₂:
H₂-D-Phe-D-Cys-Tyr-D-Trp-Lys-Abu-Cys-Thr-NH₂;
H₂-D-β-Nal-D-Cys-Tyr-D-Trp-Lys-Abu-Cys-Thr-NH₂;
H₂-D-β-Nal-D-Cys-Tyr-D-Trp-Lys-Val-Cys-D-β-Nal-NH₂;
H₂-D-p-F-Phe-D-Cys-Pal-D-Trp-Lys-Val-Cys-D-p-F-Phe-NH₂;
H₂-D-Bip-D-Cys-Tyr-D-Trp-Lys-Val-Cys-β-Nal-NH₂;
H₂-D-Dip-D-Cys-Pal-D-Trp-Lys-Val-Cys-β-Nal-NH₂ ;
H₂-D-p-F-Phe-D-Cys-Pal-D-Trp-Lys-Tle-Cys-β-Nal-NH₂;
H₂-D-p-Cl-Phe-D-Cys-Pal-D-Trp-Lys-Tle-Cys-p-Cl-Phe-NH₂:
p-NO₂-D-Phe-D-Cys-Pal-D-Trp-Lys-Thr(Bzl)-Cys-Tyr(BZl)-NH₂;
p-NO₂-D-Phe-D-Cys-Tyr(Bzl)-D-Trp-Lys-Val-Cys-Tyr(Bzl)-NH₂:
(H)(4-(2-hydroxyéthyl)-1-pipérazinylacétyl)-p-N0₂-D-Phe-D-Cys-Pal-D-Trp-Lys-Thr(Bzl)-Cys-Tyr(Bzl)-NH₂ ; ou
(H) (4-(2-hydroxyéthyl)-1-pipérazinylacétyl)-p-NO₂-D-Phe-D-Cys-Tyr(Bzl)-D-Trp-Lys-Val-Cys-Tyr(Bzl)-NH₂ ; ou un sel pharmaceutiquement acceptable de ceux-ci.

11. Composé selon la revendication 2, dans lequel A¹ est supprimé, R₁ représente E₁CO substitué ou non substitué, et R² représente H.

12. Composé selon la revendication 11, dans lequel R₁ représente E₁CO substitué ou non substitué (où E₁ représente phényle, β-naphtylméthyle, β-pyridinylméthyle ou 3-indolylméthyle) ; A³ représente β-Nal, O-X-Phe (où X représente H, OH, CH₃, halo, OCH₃, NH₂, CN ou NO₂), p-X-Phe (où X représente H, OH, CH₃, halo, OCH₃, NH₂, CN ou NO₂), m-X-Phe (où X représente H, OH, CH₃, halo, OCH₃, NH₂, CN ou NO₂), F₅-Phe, Trp, Dip, 2-Pal, Tyr(Bzl), His, Igl, Tyr (I), Bta, Bip, Npa ou Pal ; A⁶ représente Thr, Ser, Tle, Thr(Bzl), Abu, Ala, Ile, Leu, Gly, Nle, β-Ala, Gaba ou Val ; et A⁸ représente l'isomère D ou L de Thr, Dip, F₅₋Phe, p-X-Phe (où X représente H, OH, CH₃, halo, OCH₃, NH₂, CN ou NO₂), o-X-Phe (où X représente H, OH, CH₃, halo, OCH₃, NH₂, CN ou NO₂), m-X-Phe (où X représente H, OH, CH₃, halo, OCH₃, NH₂, CN ou NO₂), Igl, Tyr(Bzl) ou β-Nal.

13. Composé selon la revendication 12, dans lequel R₁ représente E₁CO (où E₁ représente 4-hydroxyphényle, β-naphtylméthyle ou phényle) ; A³ représente Tyr, Tyr(I) ou Pal ; A⁶ représente Val, Tle, Nle, Ile ou Leu ; A⁸ représente p-F-Phe, β-Nal, Tyr, Dip, p-C1-Phe, Igl ou p-CN-Phe ; R₃ représente NH₂ ; ou de la formule
(H) (3-phénylpropionyl)-D-Cys-Tyr-D-Trp-Lys-Val-Cys-β-Nal-NH₂ ;
(H) (3-phénylpropionyl)-D-Cys-Pal-D-Trp-Lys-Val-Cys-β-Nal-NH₂ ;
(H) (3-phénylpropionyl)-D-Cys-Tyr-D-Trp-Lys-Thr-Cys-β-Nal-NH₂ ;
(H) (3-phénylpropionyl)-D-Cys-Pal-D-Trp-Lys-Thr-Cys-β-Nal-NH₂ ;
(H) (3-phénylpropionyl)-D-Cys-Tyr-D-Trp-Lys-Val-Cys-Thr-NH₂ ;
(H) (3-phénylpropionyl)-D-Cys-Pal-D-Trp-Lys-Val-Cys-Thr-NH₂ ;
(H) (3-phénylpropionyl)-D-Cys-Tyr-D-Trp-Lys-Thr-Cys-Thr-NH₂ ;
(H) (3-phénylpropionyl)-D-Cys-Pal-D-Trp-Lys-Thr-Cys-Thr-NH₂ ;
(H) (3-[2-naphtyl]propionyl)-D-Cys-Tyr-D-Trp-Lys-Val-Cys-β-Nal-NH₂ ;
(H) (3-[2-naphtyl]propionyl)-D-Cys-Pal-D-Trp-Lys-Val-Cys-β-Nal-NH₂ ;
(H) (3-[2-naphtyl]propionyl)-D-Cys-Tyr-D-Trp-Lys-Thr-Cys-β-Nal-NH₂ ;
(H) (3-[2-naphtyl]propionyl)-D-Cys-Pal-D-Trp-Lys-Thr-Cys-β-Nal-NH₂ ;
(H) (3-[2-naphtyl]propionyl)-D-Cys-Tyr-D-Trp-Lys-Val-Cys-Thr-NH₂ ;
(H) (3-[2-naphtyl]propionyl)-D-Cys-Pal-D-Trp-Lys-Val-Cys-Thr-NH₂ ;
(H) (3-[2-naphtyl]propionyl)-D-Cys-Tyr-D-Trp-Lys-Thr-Cys-Thr-NH₂ ;
(H) (3-[2-naphtyl]propionyl)-D-Cys-Pal-D-Trp-Lys-Thr-Cys-Thr-NH₂;
(H) (3-[p-hydroxyphényl])-D-Cys-Tyr-D-Trp-Lys-Val-Cys-β-Nal-NH₂ ;
(H) (3-naphtyl]propionyl)-D-Cys-Tyr-D-Trp-Lys-Abu-Cys-β-Nal-NH₂ ;
(H) (3-naphtyl]propionyl)-D-Cys-Tyr-D-Trp-Lys-Abu-Cys-Thr-NH₂;
(H) (3-phénylylpropionyl)-D-Cys-Tyr-D-Trp-Lys-Abu-Cys-β-Nal-NH₂ ; ou
(H) (3-phénylylpropionyl)-D-Cys-Tyr-D-Trp-Lys-Abu-Cys-Thr-NH₂ ; ou un sel pharmaceutiquement acceptable de ceux-ci.

14. Composé selon la revendication 2, dans lequel R₃, conjointement avec le groupe carbonyl de A⁸ qui est attaché à celui-ci, sont réduits pour former H, alkyl inférieur ou hydroxy alkyle inférieur.

15. Composé selon la revendication 14, dans lequel A¹ représente l'isomère D ou L de β-Nal, o-X-Phe (où X représente H, OH, CH₃, halo, OCH₃, NH₂, CN ou NO₂), -p-X-Phe (où X représente H, OH, CH₃, halo, OCH₃, NH₂, CN ou NO₂), m-X-Phe (où X représente H, OH, CH₃, halo, OCH₃, NH₂, CN ou NO₂), F₅-Phe, Trp, Dip, 2-Pal, Tyr(Bzl), His, Igl, Tyr (I), Bta, Bip, Npa ou Pal ; A³ représente β-Nal, o-X-Phe (où X représente H, OH, CH₃, halo, OCH₃, NH₂, CN ou NO₂), p-X-Phe (où X représente H, OH, CH₃, halo, OCH₃, NH₂, CN ou NO₂), m-X-Phe (où X représente H, OH, CH₃, halo, OCH₃, NH₂, CN ou NO₂), F₅-Phe, Trp, Dip, 2-Pal, Tyr(Bzl), His, Igl, Tyr(I), Bta, Bip, Npa ou Pal ; A⁶ représente Thr, Ser, Tle, Thr(Bzl), Abu, Ala, Ile, Leu, Gly, Nle, β-Ala, Gaba ou Val ; et A⁸ représente l'isomère D ou L de Thr, Dip, F₅-Phe, p-X-Phe (où X représente H, OH, CH₃, halo, OCH₃, NH₂, CN ou NO₂), o-X-Phe (où X représente H, OH, CH₃, halo, OCH₃, NH₂, CN ou NO₂), m-X-Phe (où X représente H, OH, CH₃, halo, OCH₃, NH₂, CN ou NO₂), Igl, Tyr (Bzl) ou β-Nal.

16. Composé selon la revendication 15, dans lequel A¹ représente l'isomère D ou L de β-Nal, Phe, p-F-Phe, Trp, p-Cl-Phe ou p-CN-Phe ; A³ représente Tyr, Tyr(I) ou Pal ; A⁶ représente Val, Tle, Nle, Ile ou Leu ; A⁸ représente p-F-Phe, β-Nal, Tyr, Dip, p-Cl-Phe, Igl ou p-CN-Phe ; R₁ représente H, CH₃CO, 4-(2-hydroxyéthyl)-1-pipérazinylacétyle ou 4-(2-hydroxyéthyl)-1-pipérizine-éthanesulfonyle ; R₂ représente H, et R₃, conjointement avec le groupe carboxy de A⁸ attaché à celui-ci sont réduits pour former H ou CH₃OH.

17. Composé selon l'une des revendications 9, 13 ou 16, dans lequel A³ représente Pal.

18. Composé selon la revendication 15, de formule :
H₂-β-Nal-D-Cys-Tyr-D-Trp-Lys-Val-Cys-2R,3R-(2-hydroxyméthyl)-3-hydroxy)propylamide ;
(H) (CH₃CO)-β-Nal-D-Cys-Tyr-D-Trp-Lys-Val-Cys-2R,3R-(2-hydroxyméthyl)-3-hydroxy)propylamide ;
(H)(4-(2-hydroxyéthyl)-1-pipérazinylacétyl)-β-Nal-D-Cys-Tyr-D-Trp-Lys-Val-Cys-2R,3R-(2-hydroxyméthyl)-3-hydroxy)propylamide ;
(H)(4-(2-hydroxyéthyl)-1-pipérizineéthanesulfonyl)-β-Nal-D-Cys-Tyr-D-Trp-Lys-Val-Cys-2R,3R-(2-hydroxyméthyl)-3-hydroxy)propylamide ;
H₂-β-Nal-D-Cys-Pal-D-Trp-Lys-Val-Cys-2R,3R-(2-hydroxyméthyl)-3-hydroxy)propylamide ;
(H)(CH₃CO)-β-Nal-D-Cys-Pal-D-Trp-Lys-Val-Cys-2R,3R-(2-hydroxyméthyl)-3-hydroxy)propylamide ;
(H)(4-(2-hydroxyéthyl)-1-pipérazinylacétyl)-β-Nal-D-Cys-Pal-D-Trp-Lys-Val-Cys-2R,3R-(2-hydroxyméthyl)-3-hydroxy)propylamide ;
(H)(4-(2-hydroxyéthyl)-1-pipérizineéthanesulfonyl) -β-Nal-D-Cys-Pal-D-Trp-Lys-Val-Cys-2R,3R-(2-hydroxyméthyl)-3-hydroxy)propylamide ;
H₂-β-Nal-D-Cys-Tyr-D-Trp-Lys-Thr-Cys-2R,3R-(2-hydroxyméthyl)-3-hydroxy)propylamide ;
(H) (CH₃CO)-β-Nal-D-Cys-Tyr-D-Trp-Lys-Thr-Cys-2R,3R-(2-hydroxyméthyl)-3-hydroxy)propylamide ;
(H) (4-(2-hydroxyéthyl)-1-pipérazinylacétyl)-β-Nal-D-Cys-Tyr-D-Trp-Lys-Thr-Cys-2R,3R-(2-hydroxyméthyl)-3-hydroxy)propylamide ;
(H)(4-(2-hydroxyéthyl)-1-pipérizineéthanesulfonyl)-β-Nal-D-Cys-Tyr-D-Trp-Lys-Thr-Cys-2R,3R-(2-hydroxyméthyl)-3-hydroxy)propylamide ;
H₂-β-Nal-D-Cys-Pal-D-Trp-Lys-Thr-Cys-2R, 3R-(2-hydroxyméthyl)-3-hydroxy)propylamide ;
(H) (CH₃CO)-β-Nal-D-Cys-Pal-D-Trp-Lys-Thr-Cys-2R,3R-(2-hydroxyméthyl)-3-hydroxy)propylamide ;
(H) (4-(2-hydroxyéthyl)-1-pipérazinylacétyl)-β-Nal-D-Cys-Pal-D-Trp-Lys-Thr-Cys-2R,3R-(2-hydroxyméthyl)-3-hydroxy)propylamide ;
(H) (4-(2-hydroxyéthyl)-1-pipérizineéthanesulfonyl)-β-Nal-D-Cys-Pal-D-Trp-Lys-Thr-Cys-2R,3R-(2-hydroxyméthyl)-3-hydroxy)propylamide ;
H₂-Phe-D-Cys-Tyr-D-Trp-Lys-Val-Cys-2R,3R-(2-hydroxyméthyl)-3-hydroxy)propylamide ;
(H) (CH₃CO)-Phe-D-Cys-Tyr-D-Trp-Lys-Val-Cys-2R,3R-(2-hydroxyméthyl)-3-hydroxy)propylamide ;
(H)(4-(2-hydroxyéthyl)-1-pipérazinylacétyl)Phe-D-Cys-Tyr-D-Trp-Lys-Val-Cys-2R,3R-(2-hydroxyméthyl)-3-hydroxy)propylamide ;
(H)(4-(2-hydroxyéthyl)-1-pipérizineéthanesulfonyl)Phe-D-Cÿs-Tyr-D-Trp-Lys-Val-Cys-2R,3R-(2-hydroxyméthyl)-3-hydroxy)propylamide ;
H₂-Phe-D-Cys-Pal-D-Trp-Lys-Val-Cys-2R, 3R-(2-hydroxyméthyl)-3-hydroxy)propylamide ;
H(CH₃CO)Phe-D-Cys-Pal-D-Trp-Lys-Val-Cys-2R,3R-(2-hydroxyméthyl)-3-hydroxy)propylamide ;
(H)(4-(2-hydroxyéthyl)-1-pipérazinylacétyl)Phe-D-Cys-Pal-D-Trp-Lys-Val-Cys-2R,3R-(2-hydroxyméthyl)-3-hydroxy) propylamide ;
(H)(4-(2-hydroxyéthyl)-1-pipérizineéthanesulfonyl)Phe-D-Cys-Pal-D-Trp-Lys-Val-Cys-2R,3R-(2-hydroxyméthyl)-3-hydroxy)propylamide ;
H₂-Phe-D-Cys-Tyr-D-Trp-Lys-Thr-Cys-2R,3R-(2-hydroxyméthyl)-3-hydroxy)propylamide ;
(H) (CH₃CO)Phe-D-Cys-Tyr-D-Trp-Lys-Thr-Cys-2R,3R-(2-hydroxyméthyl)-3-hydroxy)propylamide ;
(H)(4-(2-hydroxyéthyl)-1-pipérazinylacétyl)Phe-D-Cys-Tyr-D-Trp-Lys-Thr-Cys-2R,3R-(2-hydroxyméthyl)-3-hydroxy)propylamide ;
(H)(4-(2-hydroxyéthyl)-1-pipérizineéthanesulfonyl)Phe-D-Cys-Tyr-D-Trp-Lys-Thr-Cys-2R,3R-(2-hydroxyméthyl)-3-hydroxy)propylamide ;
H₂-Phe-D-Cys-Pal-D-Trp-Lys-Thr-Cys-2R,3R-(2-hydroxyméthyl)-3-hydroxy)propylamide ;
(H) (CH₃CO)Phe-D-Cys-Pal-D-Trp-Lys-Thr-Cys-2R,3R-(2-hydroxyméthyl)-3-hydroxy)propylamide ;
(H)(4-(2-hydroxyéthyl)-1-pipérazinylacétyl)Phe-D-Cys-Pal-D-Trp-Lys-Thr-Cys-2R,3R-(2-hydroxyméthyl)-3-hydroxy)propylamide ;
(H)(4-(2-hydroxyéthyl)-1-pipérizineéthanesulfonyl)Phe-D-Cys-Pal-D-Trp-Lys-Thr-Cys-2R,3R-(2-hydroxyméthyl)-3-hydroxy)propylamide ;
H₂-β-Nal-D-Cys-Tyr-D-Trp-Lys-Val-Cys-2R-(2-naphtyl)éthylamide ;
(H) (CH₃CO)-β-Nal-D-Cys-Tyr-D-Trp-Lys-Val-Cys-2R-(2-naphtyl)éthylamide ;
(H)(4-(2-hydroxyéthyl)-l-pipérazinylacétyl)-p-Nal-D-Cys-Tyr-D-Trp-Lys-Val-Cys-2R-(2-naphtyl)éthylamide ;
(H)(4-(2-hydroxyéthyl)-l-pipérizineéthanesulfonyl)-P-Nal-D-Cys-Tyr-D-Trp-Lys-Val-Cys-2R-(2-naphtyl)éthylamide ;
H₂-β-Nal-D-Cys-Pal-D-Trp-Lys-Val-Cys-2R-(2-naphtyl)éthylamide ;
(H) (CH₃CO)-β-Nal-D-Cys-Pal-D-Trp-Lys-Val-Cys-2R-(2-naphtyl)éthylamide ;
(H) (4-(2-hydroxyéthyl)-1-pipérazinylacétyl)-β-Nal-D-Cys-Pal-D-Trp-Lys-Val-Cys-2R-(2-naphtyl)éthylamide ;
(H) (4-(2-hydroxyéthyl)-1-pipérizineéthanesulfonyl)-β-Nal-D-Cys-Pal-D-Trp-Lys-Val-Cys-2R-(2-naphtyl)éthylamide ;
H₂-β-Nal-D-Cys-Tyr-D-Trp-Lys-Thr-Cys-2R-(2-naphtyl)éthylamide ;
(H)(CH₃CO)-β-Nal-D-Cys-Tyr-D-Trp-Lys-Thr-Cys-2R-(2-naphtyl)éthylamide ;
(H)(4-(2-hydroxyéthyl)-1-pipérazinylacétyl)-β-Nal-D-Cys-Tyr-D-Trp-Lys-Thr-Cys-2R-(2-naphtyl)éthylamide ;
(H)(4-(2-hydroxyéthyl)-1-pipérizineéthanesulfonyl)-β-Nal-D-Cys-Tyr-D-Trp-Lys-Thr-Cys-2R-(2-naphtyl)éthylamide ;
H₂-β-Nal-D-Cys-Pal-D-Trp-Lys-Thr-Cys-2R-(2-naphtyl)éthylamide ;
(H) (CH₃CO)-β-Nal-D-Cys-Pal-D-Trp-Lys-Thr-Cys-2R-(2-naphtyl)éthylamide ;
(H) (4-(2-hydroxyéthyl)-1-pipérazinylacétyl)-β-Nal-D-Cys-Pal-D-Trp-Lys-Thr-Cys-2R-(2-naphtyl)éthylamide ;
(H) (4-(2-hydroxyéthyl)-1-pipérizineéthanesulfonyl)-β-Nal-D-Cys-Pal-D-Trp-Lys-Thr-Cys-2R-(2-naphtyl) éthylamide ;
H₂-Phe-D-Cys-Tyr-D-Trp-Lys-Val-Cys-2R-(2-naphtyl)éthylamide ;
(H)(CH₃CO)-Phe-D-Cys-Tyr-D-Trp-Lys-Val-Cys-2R-(2-naphtyl)éthylamide ;
(H) (4-(2-hydroxyéthyl)-1-pipérazinylacétyl)Phe-D-Cys-Tyr-D-Trp-Lys-Val-Cys-2R-(2-naphtyl)éthylamide ;
(H)(4-(2-hydroxyéthyl)-1-pipérizineéthanesulfonyl)Phe-D-Cys-Tyr-D-Trp-Lys-Val-Cys-2R-(2-naphtyl)éthylamide ;
H₂-Phe-D-Cys-Pal-D-Trp-Lys-Val-Cys-2R-(2-naphtyl)éthylamide ;
(H)(CH₃CO)Phe-D-Cys-Pal-D-Trp-Lys-Val-Cys-2R-(2-naphtyl)éthylamide ;
(H) (4-(2-hydroxyéthyl)-1-pipérazinylacétyl)Phe-D-Cys-Pal-D-Trp-Lys-Val-Cys-2R-(2-naphtyl)éthylamide ;
(H) (4-(2-hydroxyéthyl)-1-pipérizineéthanesulfonyl)-Phe-D-Cys-Pal-D-Trp-Lys-Val-Cys-2R-(2-naphtyl)éthylamide ;
H₂-Phe-D-Cys-Tyr-D-Trp-Lys-Thr-Cys-2R-(2-naphtyl)éthylamide ;
(H)(CH₃CO)Phe-D-Cys-Tyr-D-Trp-Lys-Thr-Cys-2R-(2-naphtyl)éthylamide ;
(H)(4-(2-hydroxyéthyl)-1-pipérazinylacétyl)Phe-D-Cys-Tyr-D-Trp-Lys-Thr-Cys-2R-(2-naphtyl)éthylamide ;
(H)(4-(2-hydroxyéthyl)-1-pipérizineéthanesulfonyl)Phe-D-Cys-Tyr-D-Trp-Lys-Thr-Cys-2R-(2-naphtyl)éthylamide ;
H₂-Phe-D-Cys-Pal-D-Trp-Lys-Thr-Cys-2R-(2-naphtyl)éthylamide ;
(H) (CH₃CO)Phe-Cys-Pal-D-Trp-Lys-Thr-Cys-2R-(2-naphtyl)éthylamide ;
(H)(4-(2-hydroxyéthyl)-1-pipérazinylacétyl)Phe-D-Cys-Pal-D-Trp-Lys-Thr-Cys-2R-(2-naphtyl)éthylamide ;
(H)(4-(2-hydroxyéthyl)-1-pipérizineéthanesulfonyl)Phe-D-Cys-Pal-D-Trp-Lys-Thr-Cys-2R-(2-naphtyl)éthylamide ;
H₂-β-Nal-D-Cys-Tyr-D-Trp-Lys-Abu-Cys-2R-(2-naphtyl)éthylamide ;
H₂-Phe-D-Cys-Tyr-D-Trp-Lys-Abu-Cys-2R-(2-naphtyl)éthylamide ;
H₂-β-Nal-D-Cys-Tyr-D-Trp-Lys-Abu-Cys-2R,3R-(2-hydroxyméthyl)-3-hydroxy)propylamide ; ou
H₂-Phe-D-Cys-Tyr-D-Trp-Lys-Abu-Cys-2R,3R-(2-hydroxyméthyl)-3-hydroxy)propylamide ; ou un sel pharmaceutiquement acceptable de ceux-ci.

19. Composé selon la revendication 1, dans lequel A₂ est un D-amino-acide aromatique ou un D-amino-acide aliphatique, A₇ est un acide aminé aromatique ou un acide aminé aliphatique et A₄ est D-trp.

20. Composé selon la revendication 19, dans lequel A₁ est un L-amino-acide et A₂ est un D-amino-acide aromatique.

21. Composé selon la revendication 20, dans lequel A₁, A₃ et A₇ représentent indépendamment β-Nal, o-X-Phe (où X représente H, OH, CH₃, halo, OCH₃, NH₂, CN ou NO₂), p-X-Phe (où X représente H, OH, CH₃, halo, OCH₃, NH₂, CN ou NO₂) , m-X-Phe (où X représente H, OH, CH₃, halo, OCH₃, NH₂, CN ou NO₂) , F₅-Phe, Trp, Dip, 2-Pal, Tyr (Bzl) , His, Igl, Tyr (I) , Bta, Bip, Npa ou Pal ; A² représente D-β-Nal, D-o-X-Phe (où X représente H, OH, CH₃, halo, OCH₃, NH₂, CN ou NO₂), D-p-X-Phe (où X représente H, OH, CH₃, halo, OCH₃, NH₂, CN ou NO₂), D-m-X-Phe (où X représente H, OH, CH₃, halo, OCH₃, NH₂, CN ou NO₂), D-F₅-Phe, D-Trp, D-Dip, D-2-Pal, D-Tyr(Bzl), D-His, D-Igl, D-Tyr(I), D-Bta, D-Bip, D-Npa ou D-Pal ; A⁶ représente Thr, Ser, Tle, Thr(Bzl), Abu, Ala, Ile, Leu, Gly, Nle, β-Ala, Gaba ou Val; et A⁸ représente l'isomère D ou L de Thr, Dip, F₅-Phe, p-X-Phe (où X représente H, OH, CH₃, halo, OCH₃, NH₂, CN ou NO₂), o-X-Phe (où X représente H, OH, CH₃, halo, OCH₃, NH₂, CN ou NO₂) , m-X-Phe (où X représente H, OH, CH₃, halo, OCH₃, NH₂, CN ou NO₂), Igl, Tyr (Bzl) ou β-Nal.

22. Composé selon la revendication 21, dans lequel A¹ représente β-Nal ou Phe ; A² représente D-Cpa ou D-Phe ; A³ représente Phe ou Tyr; A⁶ représente Abu, Thr ou Val ; A⁷ représente Phe ; et A⁸ représente Thr ; R₁ représente H, CH₃CO, 4-(2-hydroxyéthyl)-1-pipérazinylacétyle ou 4-(2-hydroxyéthyl)-1-pipérizineéthanesulfonyle ; R₂ représente H ; et R₃ représente NH₂ ; ou
de formule :
H₂-Phe-D-Phe-Tyr-D-Trp-Lys-Thr-Phe-Thr-NH₂;
H₂-Phe-D-Phe-Tyr-D-Trp-Lys-Val-Phe-Thr-NH₂;
H₂-Phe-D-Cpa-Tyr-D-Trp-Lys-Val-Phe-Thr-NH₂;
H₂-β-Nal-D-Cpa-Tyr-D-Trp-Lys-Val-Phe-Thr-NH₂;
(H) (CH₃CO) -β-Nal-D-Cpa-Tyr-D-Trp-Lys-Val-Phe-Thr-NH₂ ;
(H)(4-(2-hydroxyéthyl)-1-pipérazinylacétyl)-β-Nal-D-Cpa-Tyr-D-Trp-Lys-Val-Phe-Thr-NH₂ ;
(H)(4-(2-hydroxyéthyl)-1-pipérizineéthanesulfonyl)-β-Nal-D-Cpa-Tyr-D-Trp-Lys-Val-Phe-Thr-NH₂ ;
H₂-β-Nal-D-Cpa-Pal-D-Trp-Lys-Val-Phe-Thr-NH₂;
(H) (CH₃CO)-β-Nal-D-Cpa-Pal-D-Trp-Lys-Val-Phe-Thr-NH₂;
(H) (4-(2-hydroxyéthyl)-1-pipérazinylacétyl)-β-Nal-D-Cpa-Pal-D-Trp-Lys-Val-Phe-Thr-NH₂ ;
(H)(4-(2-hydroxyéthyl)-l-pipérizineéthanesulfonyl)-p-Nal-D-Cpa-Pal-D-Trp-Lys-Val-Phe-Thr-NH₂ ;
H₂-β-Nal-D-Cpa-Tyr-D-Trp-Lys-Thr-Phe-Thr-NH₂ ;
(H)(CH₃CO)-β-Nal-D-Cpa-Tyr-D-Trp-Lys-Thr-Phe-Thr-NH₂ ;
(H) (4-(2-hydroxyéthyl)-1-pipérazinylacétyl)-β-Nal-D-Cpa-Tyr-D-Trp-Lys-Thr-Phe-Thr-NH₂ ;
(H)(4-(2-hydroxyéthyl)-1-pipérizineéthanesulfonyl)-β-Nal-D-Cpa-Tyr-D-Trp-Lys-Thr-Phe-Thr-NH₂ ;
H₂-β-Nal-D-Cpa-Pal-D-Trp-Lys-Thr-Phe-Thr-NH₂ ;
(H) (CH₃CO) -β-Nal-D-Cpa-Pal-D-Trp-Lys-Thr-Phe-Thr-NH₂ ;
(H) (4-(2-hydroxyéthyl)-1-pipérazinylacétyl)-β-Nal-D-Cpa-Pal-D-Trp-Lys-Thr-Phe-Thr-NH₂ ;
(H) (4-(2-hydroxyéthyl)-1-pipérizineéthanesulfonyl)-β-Nal-D-Cpa-Pal-D-Trp-Lys-Thr-Phe-Thr-NH₂ ;
H₂-β-Nal-D-Cpa-Tyr-D-Trp-Lys-Val-Phe-β-Nal-NH₂ ;
(H) (CH₃CO)-β-Nal-D-Cpa-Tyr-D-Trp-Lys-Val-Phe-β-Nal-NH₂;
(H) (4-(2-hydroxyéthyl)-1-pipérazinylacétyl)-β-Nal-D-Cpa-Tyr-D-Trp-Lys-Val-Phe-β-Nal-NH₂ ; ou
(H) (4-(2-hydroxyéthyl)-1-pipérizineéthanesulfonyl)-β-Nal-D-Cpa-Tyr-D-Trp-Lys-Val-Phe-β-Nal-NH₂ ;
H₂-β-Nal-D-Cpa-Tyr-D-Trp-Lys-Val-Phe-β-Nal-NH₂- ; ou
H₂-β-Nal-D-Cpa-Tyr-D-Trp-Lys-Val-Phe-Thr-NH₂ ; ou un sel pharmaceutiquement acceptable de ceux-ci.

23. Composé selon la revendication 19, dans lequel A¹ représente un D-amino-acide et A² représente un D-amino-acide aromatique.

24. Composé selon la revendication 23, dans lequel A¹ et A² représentent, indépendamment, D-β-Nal, D-o-X-Phe (où X représente H, OH, CH₃, halo, OCH₃, NH₂, CN ou NO₂), D-p-X-Phe (où X représente H, OH, CH₃, halo, OCH₃, NH₂, CN ou NO₂), D-m-X-Phe (où X représente H, OH, CH₃, halo, OCH₃, NH₂, CN, ou NO₂), D-F₅-Phe, D-Trp, D-Dip, D-2-Pal, D-Tyr(Bzl), D-His, D-Igl, D-Tyr(I), D-Bta, D-Bip, D-Npa ou D-Pal ; A³ et A⁷ représentent, indépendamment, β-Nal, o-X-Phe (où X représente H, OH, CH₃, halo, OCH₃, NH₂, CN ou NO₂), p-X-Phe (où X représente H, OH, CH₃, halo, OCH₃, NH₂, CN ou NO₂), m-X-Phe (où X représente H, OH, CH₃, halo, OCH₃, NH₂, CN ou NO₂), F₅-Phe, Trp, Dip, 2-Pal, His, Igl, Tyr(I), Bta, Bip, Npa, Tyr(Bzl) ou Pal ; A⁶ représente Thr, Ser, Tle, Thr(Bzl), Abu, Ala, Ile, Leu, Gly, Nle, β-Ala, Gaba ou Val ; et A⁸ représente l'isomère D ou L de Thr, Dip, F₅₋Phe, p-X-Phe (où X représente H, OH, CH₃, halo, OCH₃, NH₂, CN ou NO₂), O-X-Phe (où X représente H, OH, CH₃, halo, OCH₃, NH₂, CN ou NO₂), m-X-Phe (où X représente H, OH, CH₃, halo, OCH₃, NH₂, CN ou NO₂), Igl, Tyr (Bzl) ou β-Nal.

25. Composé selon la revendication 24, dans lequel A¹ représente D-β-Nal ou D-Phe ; A² représente D-Cpa ou D-Phe ; A³ représente Phe ou Tyr ; A⁶ représente Thr ou Val ; A⁷ représente Phe et A⁸ représente Thr ; R₁ représente H, CH₃CO, 4-(2-hydroxyéthyl)-1-pipérazinylacétyle, ou 4-(2-hydroxyéthyl)-1-pipérizineéthanesulfonyle ; R₂ représente H ; et R₃ représente NH₂ ; ou
de formule
H₂-D-β-Nal-D-Gpa-Phe-D-Trp-Lys-Val-Phe-Thr-NH₂;
H₂-D-β-Nal-D-Phe-Tyr-D-Trp-Lys-Thr-Phe-Thr-NH₂;
H₂-D-Phe-D-Phe-Tyr-D-Trp-Lys-Val-Phe-Thr-NH₂;
H₂-D-β-Nal-D-cpa-Tyr-D-Trp- Lys- Val- Phe-Thr-NH₂; ou
H₂-D-β-Nal-D-Cpa-Tyr-D-Trp-Lys-Val-Phe-β-Nal-NH₂;
ou un sel pharmaceutiquement acceptable de ceux-ci.
